# EUROPEAN PATENT APPLICATION

(11) **EP 4 737 478 A1**
(43) Date of publication of application: **06.05.2026**
(21) Application number: 24830820.7
(22) Date of filing: 26.06.2024
(51) Int. Cl.: C07K 14/705, C07K 19/00, G01N 33/542

(54) **MODIFIED G PROTEIN COUPLED RECEPTOR AND USE THEREOF**

(30) Priority: 27.06.2023 CN 202310765506
(71) Applicant: Alphelix Biotech Co., Ltd., Foshan, Guangdong 528000 (CN)
(72) Inventor: LI, Minghui, Foshan, Guangdong 528000 (CN)
(74) Representative: Ostertag & Partner Patentanwälte mbB
(86) International application number: PCT/CN2024/101657
(87) International publication number: WO 2025/002184

(57) **Abstract**

The present application belongs to the technical field of protein engineering, and particularly relates to a modified G protein coupled receptor, a complex comprising the modified G protein coupled receptor, and use thereof. The present application provides a modified G protein coupled receptor which successively comprises, from an N-terminus to a C-terminus, the N terminus, a first transmembrane domain, a first intracellular loop, a second transmembrane domain, a first extracellular loop, a third transmembrane domain, a second intracellular loop, a fourth transmembrane domain, a second extracellular loop, a fifth transmembrane domain, a third intracellular loop, a sixth transmembrane domain, a third extracellular loop, a seventh transmembrane domain and the C terminus, wherein at least a part of the first intracellular loop, the second intracellular loop and/or the third intracellular loop is replaced with an optional first linker, a first part of a fluorescent protein and an optional second linker, wherein the first linker and the second linker each independently comprise one or more amino acids.

## Description

### CROSS-REFERENCE TO RELATED APPLICATION

This application claims the priority of application Chinese patent CN2023107655060 filed on June 27, 2023 and entitled "modification method of G protein receptor and use", the disclosure of which is incorporated by reference herein in its entirety.

### TECHNICAL FIELD

The present application belongs to the technical field of protein engineering, and particularly relates to a modified G protein coupled receptor, a complex comprising the modified G protein coupled receptor, and use thereof.

### BACKGROUND

A majority of drug molecules exert effects by binding to corresponding target proteins in vivo. They activate or inhibit the function of a target protein, and change the physiological process of a human body, thereby achieving the purpose of disease treatment. A G protein coupled receptor (GPCR) is a type of target proteins having an important drug development and research value in the human body. About 40% of the drugs currently existing in the market are based on the G protein coupled receptor as a target. Many drugs using these proteins as targets are still being developed. A large number of GPCRs exist in the human body, more than 800 of which have been found. These GPCRs share certain commonalities in their spatial structures: they all comprise 7 transmembrane helices; the N-terminus and 3 interhelical loop structures are located at the outer side of the cell, and the C-terminus and the other 3 interhelical loop structures are located at the inner side of the cell. These proteins are located on a cell membrane, and as receptors of signal molecules, transmit signals to the interior of the cell. A foreign signal molecule binds to a part of GPCR located at the outer side of the cell, causing the conformational change of the transmembrane helices of the receptor so that the intracellular part of the receptor binds to the G protein and triggers the physiological effect in the cell. GPCR is involved in a wide variety of physiological processes and plays a crucial role in maintaining normal life activities in the human body. The drug molecules can exert agonistic or inhibitory effects on GPCR by binding to GPCR, thereby regulating the physiological processes inside the cell. Therefore, in theory, GPCR can be widely used in the discovery and screening of the drugs.

However, many GPCRs cannot stably exist in the ligand-free state, thereby restricting their uses in the discovery, screening and other aspects of the drugs. DNA encoding compound library screening rapidly developed in recent years and identifying compounds binding to specific target proteins from compound mixtures utilizing a mass spectrometric technique propose extremely high requirements on screened target proteins, including: good stability and similar qualities of binding to natural wild type protein ligands, especially unoccupied ligand binding sites. For the discovery of therapeutic antibody drugs, regardless of a hybridoma technology, a single B cell technology or various display technologies, compared with cells that overexpress target proteins, the purified target proteins with the aforementioned characteristics have significant advantages, greatly accelerate the research and development process, and avoid the interference of other background proteins. In another aspect, accurately determining the affinity between target GPCR and a compound or an antibody by utilizing these stable purified proteins in combination with biological and physical technologies such as surface plasmon resonance (SPR) is extremely conducive to optimization and improvement of the drugs.

In another aspect, analyzing the structures of complexes formed by drug molecules or potential drug molecules and their target GPCR proteins is of great guiding significance for studying their mechanism of action and further research and development of drugs. At present, the cryogenic electron microscopy has been widely applied in structural analysis of GPCR, thereby yielding a large number of GPCR structures. However, the most of these structures are composite structures formed by GPCRs binding to agonists and coupled with G proteins, and such complexes can meet the requirements of the cryogenic electron microscopy on the sizes of target protein molecules. When GPCRs are in a non-agonistic state due to binding to antagonists and inverse agonists or negative modulators, they together with G proteins do not form stable complexes. The molecular weight of a single GPCR protein is in a range of 30-60 kD in most cases, and therefore its structure is difficultly analyzed via cryogenic electron microscopy.

Hence, there is an urgent need in the art to improve the stability of the G protein coupled receptor in the ligand-free state and address the issue of analyzing the structure of GPCR in the inactive state via cryogenic electron microscopy.

### SUMMARY

In order to solve the above problems, the present application provides a modified G protein coupled receptor, a complex comprising the modified G protein coupled receptor, and use thereof.

In a first aspect, the present application provides a modified G protein coupled receptor, the modified G protein coupled receptor successively comprising, from an N terminus to a C terminus, the N terminus, a first transmembrane domain, a first intracellular loop, a second transmembrane domain, a first extracellular loop, a third transmembrane domain, a second intracellular loop, a fourth transmembrane domain, a second extracellular loop, a fifth transmembrane domain, a third intracellular loop, a sixth transmembrane domain, a third extracellular loop, a seventh transmembrane domain and the C terminus, wherein at least a part of the first intracellular loop, the second intracellular loop and/or the third intracellular loop is replaced with an optional first linker, a first part of a fluorescent protein and an optional second linker, wherein the first linker and the second linker each independently comprise one or more amino acids.

In a second aspect, the present application provides a complex, the complex comprising the modified G protein coupled receptor according to the first aspect of the present application and a second part of a fluorescent protein, wherein the first part of the fluorescent protein binds to the second part of the fluorescent protein.

In a third aspect, the present application provides use of the modified G protein coupled receptor according to the first aspect of the present application or the complex according to the second aspect of the present application in ligand affinity determination and drug discovery or screening.

In a fourth aspect, the present application provides use of the complex according to the second aspect of the present application in analysis of a 3D structure via cryogenic electron microscopy.

The modified G protein coupled receptor and the corresponding complex of the present application have good stability in the ligand-free state, its ligand binding site is unoccupied, it has ligand binding activity similar to that of a wild type G protein coupled receptor, and therefore can be used for discovery and screening of the drugs. In addition, the complex of the present application can be used for analysis of a 3D structure via cryogenic electron microscopy, thereby providing further guidance for development and optimization of the drugs.

### BRIEF DESCRIPTION OF THE DRAWINGS

FIG. 1 is a schematic diagram showing the protein engineering modification of GPCR;
FIG. 2 is an identification graph of a GFP-clamp protein after being purified; wherein, A in FIG. 2 is a molecular sieve chromatography result graph, and B in FIG. 2 is a sodium dodecyl sulfate (SDS) gel electrophoresis identification result graph;
FIG. 3 is a screening result graph showing that at least a part of a third intracellular loop of an A2A adenosine receptor is replaced with an optional first linker, a first part (β-strands 10-11 of GFP, referred to as GFP10-11 for short) of a fluorescent protein and an optional second linker, wherein a replacement position is shown;
FIG. 4 is a screening result graph showing that at least a part of a third intracellular loop of an A2A adenosine receptor is replaced with an optional first linker, GFP10-11 and an optional second linker, wherein amino acid sequence information of the optional first linker and the optional second linker is shown;
FIG. 5 is a map of a pFastBac4R insect cell expression vector after pFastBac Dual-based vector modification;

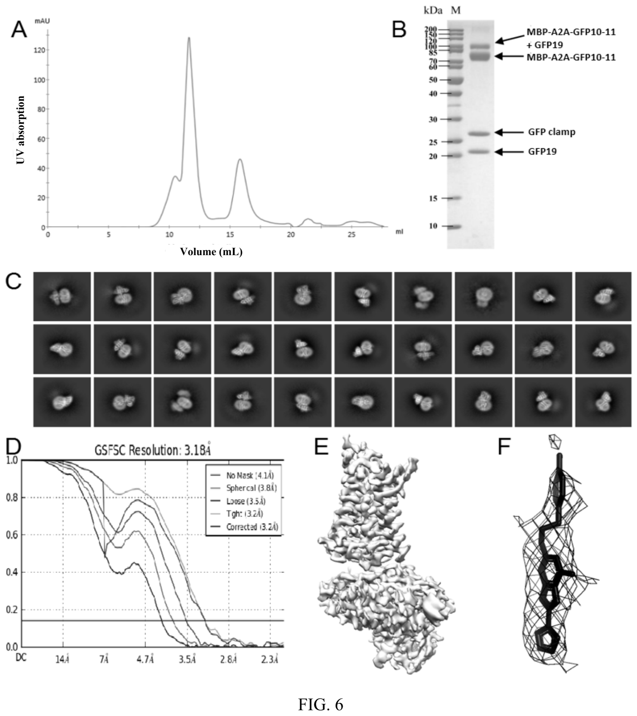

FIG. 6 is a result graph showing purification and identification of an A2A adenosine receptor modified protein complex, as well as structural analysis of its complex with a small-molecule inhibitor ZM241385; wherein, A in FIG. 6 is a molecular sieve chromatography result graph, B in FIG. 6 is an SDS gel electrophoresis identification result graph, C in FIG. 6 is a 2D classification result diagram of cryogenic electron microscopy data particles; D in FIG. 6 shows a 3D reconstruction FSC curve of cryogenic electron microscopy data; E in FIG. 6 shows the overall density of a 3D-reconstructed protein complex via cryogenic electron microscopy; and F in FIG. 6 shows the density of a 3D-reconstructed ZM241385 molecule via cryogenic electron microscopy.
FIG. 7 is a map of a mammalian cell expression vector pBacMam4R;
FIG. 8 is a result graph showing the purification and identification of a CNR1 cannabinoid receptor modified protein complex and structural analysis of its complex with a small-molecule inhibitor taranabant complex; wherein, A in FIG. 8 is a molecular sieve chromatography result graph, and B in FIG. 8 is an SDS gel electrophoresis identification result graph;
FIG. 9 is a molecular sieve chromatography and SDS gel electrophoresis identification result graph of a purified clamp protein with 186-Cys mutation (GFP clamp-Cys); wherein, A in FIG. 9 is a molecular sieve chromatography result graph, and B in FIG. 9 is an SDS gel electrophoresis identification result graph.

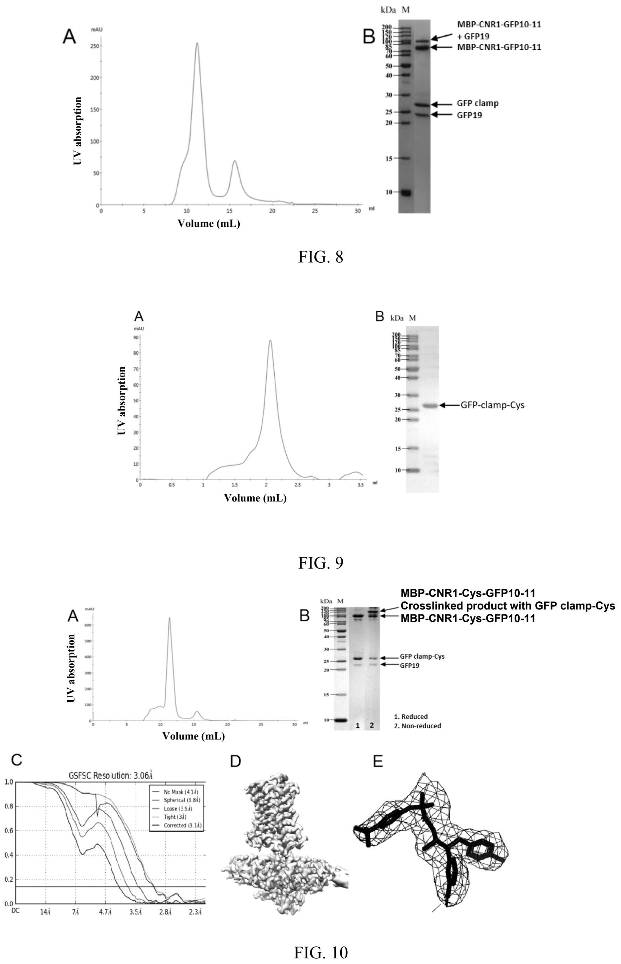

FIG. 10 is a result graph showing the purification and identification of a CNR1 cannabinoid receptor modified protein complex crosslinked via a disulfide bond and structural analysis of its complex with an inhibitor taranabant; wherein, A in FIG. 10 is a molecular sieve chromatography result graph, B in FIG. 10 is an SDS gel electrophoresis identification result graph, C in FIG. 10 is a 3D reconstruction FSC curve of cryogenic electron microscopy data, D in FIG. 10 shows the overall density of a 3D-reconstructed protein complex via cryogenic electron microscopy, and E in FIG. 10 shows the density of the taranabant molecule;

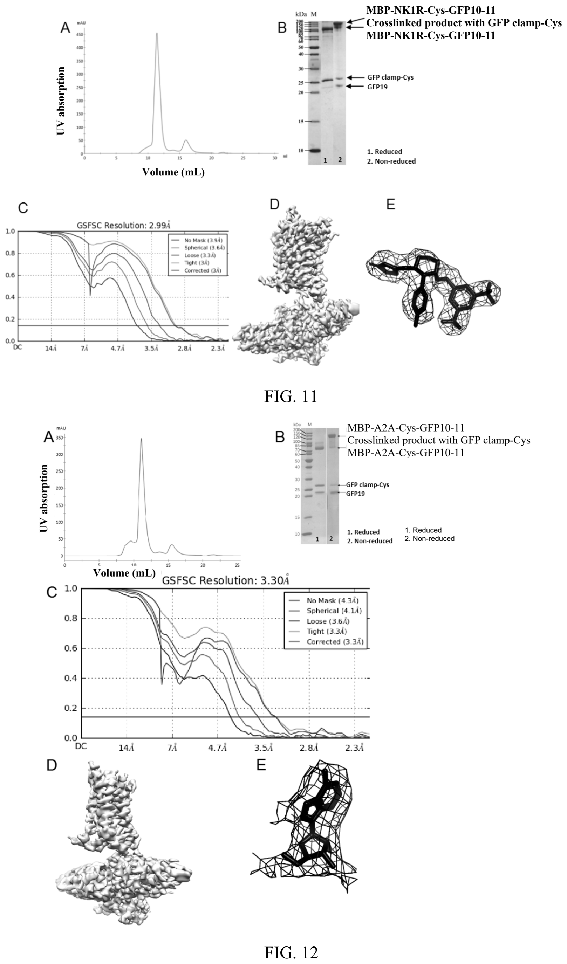

FIG. 11 is a result graph showing the purification and identification of a NK1R neurokinin receptor modified protein complex crosslinked via a disulfide bond and structural analysis of its complex with an inhibitor aprepitant; wherein, A in FIG. 11 is a molecular sieve chromatography result graph, B in FIG. 11 is an SDS gel electrophoresis identification result graph, C in FIG. 11 is a 3D reconstruction FSC curve of cryogenic electron microscopy data, D in FIG. 11 shows the overall density of a 3D-reconstructed protein complex via cryogenic electron microscopy, and E in FIG. 11 shows the density of the aprepitant molecule;
FIG. 12 is a result graph showing the purification and identification of an A2A adenosine receptor modified protein complex crosslinked via a disulfide bond and structural analysis of its complex with an inhibitor agonist adenosine molecule; wherein, A in FIG. 12 is a molecular sieve chromatography result graph, B in FIG. 12 is an SDS gel electrophoresis identification result graph, C in FIG. 12 is a 3D reconstruction FSC curve of cryogenic electron microscopy data, D in FIG. 12 shows the overall density of a 3D-reconstructed protein complex via cryogenic electron microscopy, and E in FIG. 12 shows the density of the adenosine molecule;
FIG. 13 is a purification and identification and structural analysis result graph of a CCR8 chemokine receptor modified protein complex crosslinked via a disulfide bond; wherein, A in FIG. 13 is a molecular sieve chromatography result graph, B in FIG. 13 is an SDS gel electrophoresis identification result graph, C in FIG. 13 is a 3D reconstruction FSC curve of cryogenic electron microscopy data, D in FIG. 13 shows the overall density of a 3D-reconstructed protein complex via cryogenic electron microscopy;
FIG. 14 is a purification and identification and structural analysis graph of a GPRC5D receptor modified protein complex crosslinked via a disulfide bond; wherein, A in FIG. 14 is a molecular sieve chromatography result graph, B in FIG. 14 is an SDS gel electrophoresis identification result graph, C in FIG. 14 is a 3D reconstruction FSC curve of cryogenic electron microscopy data, D in FIG. 14 shows the overall density of a 3D-reconstructed protein complex via cryogenic electron microscopy;
FIG. 15 is a molecular sieve chromatography and SDS gel electrophoresis identification result graph of a purified GPR52-Cys-GFP10-11 protein; wherein, A in FIG. 15 is a molecular sieve chromatography result graph, and B in FIG. 15 is an SDS gel electrophoresis identification result graph;
FIG. 16 is a molecular sieve chromatography and SDS gel electrophoresis identification result graph of a purified MC4R-Cys-GFP10-11 protein; wherein, A in FIG. 16 is a molecular sieve chromatography result graph, and B in FIG. 16 is an SDS gel electrophoresis identification result graph.
FIG. 17 is a molecular sieve chromatography and SDS gel electrophoresis identification result graph of a purified GNRHR-Cys-GFP 10-11 protein; wherein, A in FIG. 17 is a molecular sieve chromatography result graph, and B in FIG. 17 is an SDS gel electrophoresis identification result graph;
FIG. 18 is a molecular sieve chromatography and SDS gel electrophoresis identification result graph of a purified PTGDR2-Cys-GFP10-11 protein; wherein, A in FIG. 18 is a molecular sieve chromatography result graph, and B in FIG. 18 is an SDS gel electrophoresis identification result graph;
FIG. 19 is a molecular sieve chromatography and SDS gel electrophoresis identification result graph of a purified HTR2C-Cys-GFP10-11 protein; wherein, A in FIG. 19 is a molecular sieve chromatography result graph, and B in FIG. 19 is an SDS gel electrophoresis identification result graph;
FIG. 20 is a molecular sieve chromatography and SDS gel electrophoresis identification result graph of a purified ADRA2B-Cys-GFP10-11 protein; wherein, A in FIG. 20 is a molecular sieve chromatography result graph, and B in FIG. 20 is an SDS gel electrophoresis identification result graph;
FIG. 21 is a molecular sieve chromatography and SDS gel electrophoresis identification result graph of a purified ADRB1-Cys-GFP10-11 protein; wherein, A in FIG. 21 is a molecular sieve chromatography result graph, and B in FIG. 21 is an SDS gel electrophoresis identification result graph;
FIG. 22 is a molecular sieve chromatography and SDS gel electrophoresis identification result graph of a purified ADRB2-Cys-GFP10-11 protein; wherein, A in FIG. 22 is a molecular sieve chromatography result graph, and B in FIG. 22 is an SDS gel electrophoresis identification result graph;
FIG. 23 is a molecular sieve chromatography and SDS gel electrophoresis identification result graph of a purified CSAR1-Cys-GFP 10-11 protein; wherein, A in FIG. 23 is a molecular sieve chromatography result graph, and B in FIG. 23 is an SDS gel electrophoresis identification result graph;
FIG. 24 is a molecular sieve chromatography and SDS gel electrophoresis identification result graph of a purified CCR2-Cys-GFP10-11 protein; wherein, A in FIG. 24 is a molecular sieve chromatography result graph, and B in FIG. 24 is an SDS gel electrophoresis identification result graph;
FIG. 25 is a molecular sieve chromatography and SDS gel electrophoresis identification result graph of a purified CCR5-Cys-GFP10-11 protein; wherein, A in FIG. 25 is a molecular sieve chromatography result graph, and B in FIG. 25 is an SDS gel electrophoresis identification result graph;
FIG. 26 is a molecular sieve chromatography and SDS gel electrophoresis identification result graph of a purified CCR6-Cys-GFP10-11 protein; wherein, A in FIG. 26 is a molecular sieve chromatography result graph, and B in FIG. 26 is an SDS gel electrophoresis identification result graph;
FIG. 27 is a molecular sieve chromatography and SDS gel electrophoresis identification result graph of a purified CCR7-Cys-GFP10-11 protein; wherein, A in FIG. 27 is a molecular sieve chromatography result graph, and B in FIG. 27 is an SDS gel electrophoresis identification result graph;
FIG. 28 is a molecular sieve chromatography and SDS gel electrophoresis identification result graph of a purified CHRM2-Cys-GFP10-11 protein; wherein, A in FIG. 28 is a molecular sieve chromatography result graph, and B in FIG. 28 is an SDS gel electrophoresis identification result graph;
FIG. 29 is a molecular sieve chromatography and SDS gel electrophoresis identification result graph of a purified CLTR2-Cys-GFP10-11 protein; wherein, A in FIG. 29 is a molecular sieve chromatography result graph, and B in FIG. 29 is an SDS gel electrophoresis identification result graph;
FIG. 30 is a molecular sieve chromatography and SDS gel electrophoresis identification result graph of a purified CXCR2-Cys-GFP10-11 protein; wherein, A in FIG. 30 is a molecular sieve chromatography result graph, and B in FIG. 30 is an SDS gel electrophoresis identification result graph;
FIG. 31 is a molecular sieve chromatography and SDS gel electrophoresis identification result graph of a purified CXCR4-Cys-GFP10-11 protein; wherein, A in FIG. 31 is a molecular sieve chromatography result graph, and B in FIG. 31 is an SDS gel electrophoresis identification result graph;
FIG. 32 is a molecular sieve chromatography and SDS gel electrophoresis identification result graph of a purified DRD2-Cys-GFP10-11 protein; wherein, A in FIG. 32 is a molecular sieve chromatography result graph, and B in FIG. 32 is an SDS gel electrophoresis identification result graph;
FIG. 33 is a molecular sieve chromatography and SDS gel electrophoresis identification result graph of a purified DRD3-Cys-GFP10-11 protein; wherein, A in FIG. 33 is a molecular sieve chromatography result graph, and B in FIG. 33 is an SDS gel electrophoresis identification result graph;
FIG. 34 is a molecular sieve chromatography and SDS gel electrophoresis identification result graph of a purified GPBAR-Cys-GFP10-11 protein; wherein, A in FIG. 34 is a molecular sieve chromatography result graph, and B in FIG. 34 is an SDS gel electrophoresis identification result graph;
FIG. 35 is a molecular sieve chromatography and SDS gel electrophoresis identification result graph of a purified HRH1-Cys-GFP10-11 protein; wherein, A in FIG. 35 is a molecular sieve chromatography result graph, and B in FIG. 35 is an SDS gel electrophoresis identification result graph;
FIG. 36 is a molecular sieve chromatography and SDS gel electrophoresis identification result graph of a purified HTR1A-Cys-GFP10-11 protein; wherein, A in FIG. 36 is a molecular sieve chromatography result graph, and B in FIG. 36 is an SDS gel electrophoresis identification result graph;
FIG. 37 is a molecular sieve chromatography and SDS gel electrophoresis identification result graph of a purified HTR1B-Cys-GFP10-11 protein; wherein, A in FIG. 37 is a molecular sieve chromatography result graph, and B in FIG. 37 is an SDS gel electrophoresis identification result graph;
FIG. 38 is a molecular sieve chromatography and SDS gel electrophoresis identification result graph of a purified HTR2B-Cys-GFP10-11 protein; wherein, A in FIG. 38 is a molecular sieve chromatography result graph, and B in FIG. 38 is an SDS gel electrophoresis identification result graph;
FIG. 39 is a molecular sieve chromatography and SDS gel electrophoresis identification result graph of a purified LPAR1-Cys-GFP10-11 protein; wherein, A in FIG. 39 is a molecular sieve chromatography result graph, and B in FIG. 39 is an SDS gel electrophoresis identification result graph;
FIG. 40 is a molecular sieve chromatography and SDS gel electrophoresis identification result graph of a purified MTNR1B-Cys-GFP10-11 protein; wherein, A in FIG. 40 is a molecular sieve chromatography result graph, and B in FIG. 40 is an SDS gel electrophoresis identification result graph;
FIG. 41 is a molecular sieve chromatography and SDS gel electrophoresis identification result graph of a purified NPY1R-Cys-GFP10-11 protein; wherein, A in FIG. 41 is a molecular sieve chromatography result graph, and B in FIG. 41 is an SDS gel electrophoresis identification result graph;
FIG. 42 is a molecular sieve chromatography and SDS gel electrophoresis identification result graph of a purified OPRD-Cys-GFP10-11 protein; wherein, A in FIG. 42 is a molecular sieve chromatography result graph, and B in FIG. 42 is an SDS gel electrophoresis identification result graph;
FIG. 43 is a molecular sieve chromatography and SDS gel electrophoresis identification result graph of a purified OX2R-Cys-GFP10-11 protein; wherein, A in FIG. 43 is a molecular sieve chromatography result graph, and B in FIG. 43 is an SDS gel electrophoresis identification result graph;
FIG. 44 is a molecular sieve chromatography and SDS gel electrophoresis identification result graph of a purified PTGDR-Cys-GFP10-11 protein; wherein, A in FIG. 44 is a molecular sieve chromatography result graph, and B in FIG. 44 is an SDS gel electrophoresis identification result graph;
FIG. 45 is a molecular sieve chromatography and SDS gel electrophoresis identification result graph of a purified GPR146-Cys-GFP10-11 protein; wherein, A in FIG. 45 is a molecular sieve chromatography result graph, and B in FIG. 45 is an SDS gel electrophoresis identification result graph;
FIG. 46 is a molecular sieve chromatography and SDS gel electrophoresis identification result graph of a purified MCHR1-Cys-GFP10-11 protein; wherein, A in FIG. 46 is a molecular sieve chromatography result graph, and B in FIG. 46 is an SDS gel electrophoresis identification result graph;
FIG. 47 is a molecular sieve chromatography and SDS gel electrophoresis identification result graph of a purified TAAR1-Cys-GFP10-11 protein; wherein, A in FIG. 47 is a molecular sieve chromatography result graph, and B in FIG. 47 is an SDS gel electrophoresis identification result graph;
FIG. 48 is a molecular sieve chromatography and SDS gel electrophoresis identification result graph of a purified AGTR1-Cys-GFP10-11 protein; wherein, A in FIG. 48 is a molecular sieve chromatography result graph, and B in FIG. 48 is an SDS gel electrophoresis identification result graph;
FIG. 49 is a molecular sieve chromatography and SDS gel electrophoresis identification result graph of a purified FPR1-Cys-GFP10-11 protein; wherein, A in FIG. 49 is a molecular sieve chromatography result graph, and B in FIG. 49 is an SDS gel electrophoresis identification result graph;
FIG. 50 is a molecular sieve chromatography and SDS gel electrophoresis identification result graph of a purified GALR1-Cys-GFP10-11 protein; wherein, A in FIG. 50 is a molecular sieve chromatography result graph, and B in FIG. 50 is an SDS gel electrophoresis identification result graph;
FIG. 51 is a molecular sieve chromatography and SDS gel electrophoresis identification result graph of a purified GHSR-Cys-GFP10-11 protein; wherein, A in FIG. 51 is a molecular sieve chromatography result graph, and B in FIG. 51 is an SDS gel electrophoresis identification result graph;
FIG. 52 is a molecular sieve chromatography and SDS gel electrophoresis identification result graph of a purified CCKAR-Cys-GFP10-11 protein; wherein, A in FIG. 52 is a molecular sieve chromatography result graph, and B in FIG. 52 is an SDS gel electrophoresis identification result graph;
FIG. 53 is a molecular sieve chromatography and SDS gel electrophoresis identification result graph of a purified MTLR-Cys-GFP10-11 protein; wherein, A in FIG. 53 is a molecular sieve chromatography result graph, and B in FIG. 53 is an SDS gel electrophoresis identification result graph;
FIG. 54 is a molecular sieve chromatography and SDS gel electrophoresis identification result graph of a purified EDNRA-Cys-GFP10-11 protein; wherein, A in FIG. 54 is a molecular sieve chromatography result graph, and B in FIG. 54 is an SDS gel electrophoresis identification result graph;
FIG. 55 is a molecular sieve chromatography and SDS gel electrophoresis identification result graph of a purified PRLHR-Cys-GFP10-11 protein; wherein, A in FIG. 55 is a molecular sieve chromatography result graph, and B in FIG. 55 is an SDS gel electrophoresis identification result graph;
FIG. 56 is a molecular sieve chromatography and SDS gel electrophoresis identification result graph of a purified NPFF1-Cys-GFP10-11 protein; wherein, A in FIG. 56 is a molecular sieve chromatography result graph, and B in FIG. 56 is an SDS gel electrophoresis identification result graph;
FIG. 57 is a molecular sieve chromatography and SDS gel electrophoresis identification result graph of a purified CXCR1-Cys-GFP10-11 protein; wherein, A in FIG. 57 is a molecular sieve chromatography result graph, and B in FIG. 57 is an SDS gel electrophoresis identification result graph;
FIG. 58 is a molecular sieve chromatography and SDS gel electrophoresis identification result graph of a purified TRFR-Cys-GFP10-11 protein; wherein, A in FIG. 58 is a molecular sieve chromatography result graph, and B in FIG. 58 is an SDS gel electrophoresis identification result graph;
FIG. 59 is a molecular sieve chromatography and SDS gel electrophoresis identification result graph of a purified CML1-Cys-GFP10-11 protein; wherein, A in FIG. 59 is a molecular sieve chromatography result graph, and B in FIG. 59 is an SDS gel electrophoresis identification result graph;
FIG. 60 is a molecular sieve chromatography and SDS gel electrophoresis identification result graph of a purified QRFPR-Cys-GFP10-11 protein; wherein, A in FIG. 60 is a molecular sieve chromatography result graph, and B in FIG. 60 is an SDS gel electrophoresis identification result graph;
FIG. 61 is a molecular sieve chromatography and SDS gel electrophoresis identification result graph of a purified SSTR2-Cys-GFP10-11 protein; wherein, A in FIG. 61 is a molecular sieve chromatography result graph, and B in FIG. 61 is an SDS gel electrophoresis identification result graph;
FIG. 62 is a molecular sieve chromatography and SDS gel electrophoresis identification result graph of a purified FFAR1-Cys-GFP10-11 protein; wherein, A in FIG. 62 is a molecular sieve chromatography result graph, and B in FIG. 62 is an SDS gel electrophoresis identification result graph;
FIG. 63 is a molecular sieve chromatography and SDS gel electrophoresis identification result graph of a purified PTAFR-Cys-GFP10-11 protein; wherein, A in FIG. 63 is a molecular sieve chromatography result graph, and B in FIG. 63 is an SDS gel electrophoresis identification result graph;
FIG. 64 is a molecular sieve chromatography and SDS gel electrophoresis identification result graph of a purified P2RY1-Cys-GFP10-11 protein; wherein, A in FIG. 64 is a molecular sieve chromatography result graph, and B in FIG. 64 is an SDS gel electrophoresis identification result graph;
FIG. 65 is a molecular sieve chromatography and SDS gel electrophoresis identification result graph of a purified HCAR2-Cys-GFP10-11 protein; wherein, A in FIG. 65 is a molecular sieve chromatography result graph, and B in FIG. 65 is an SDS gel electrophoresis identification result graph;
FIG. 66 is a molecular sieve chromatography and SDS gel electrophoresis identification result graph of a purified SUCR1-Cys-GFP10-11 protein; wherein, A in FIG. 66 is a molecular sieve chromatography result graph, and B in FIG. 66 is an SDS gel electrophoresis identification result graph;
FIG. 67 is a molecular sieve chromatography and SDS gel electrophoresis identification result graph of a purified APJ-Cys-GFP10-11 protein; wherein, A in FIG. 67 is a molecular sieve chromatography result graph, and B in FIG. 67 is an SDS gel electrophoresis identification result graph;
FIG. 68 is a molecular sieve chromatography and SDS gel electrophoresis identification result graph of a purified GPR39-Cys-GFP10-11 protein; wherein, A in FIG. 68 is a molecular sieve chromatography result graph, and B in FIG. 68 is an SDS gel electrophoresis identification result graph;
FIG. 69 is a molecular sieve chromatography and SDS gel electrophoresis identification result graph of a purified GPR75-Cys-GFP10-11 protein; wherein, A in FIG. 69 is a molecular sieve chromatography result graph, and B in FIG. 69 is an SDS gel electrophoresis identification result graph;
FIG. 70 is a molecular sieve chromatography and SDS gel electrophoresis identification result graph of a purified CCR1-Cys-GFP10-11 protein; wherein, A in FIG. 70 is a molecular sieve chromatography result graph, and B in FIG. 70 is an SDS gel electrophoresis identification result graph;
FIG. 71 is a molecular sieve chromatography and SDS gel electrophoresis identification result graph of a purified CCR4-Cys-GFP10-11 protein; wherein, A in FIG. 71 is a molecular sieve chromatography result graph, and B in FIG. 71 is an SDS gel electrophoresis identification result graph;
FIG. 72 is a molecular sieve chromatography and SDS gel electrophoresis identification result graph of a purified PTGER4-Cys-GFP10-11 protein; wherein, A in FIG. 72 is a molecular sieve chromatography result graph, and B in FIG. 72 is an SDS gel electrophoresis identification result graph;
FIG. 73 is a result graph showing the purification and identification and structural analysis of a glucagon receptor GCGR modified protein complex crosslinked via a disulfide bond; wherein, A in FIG. 73 is a molecular sieve chromatography result graph, B in FIG. 73 is an SDS gel electrophoresis identification result graph, C in FIG. 73 is a 3D reconstruction FSC curve of cryogenic electron microscopy data, D in FIG. 73 shows the overall density of a 3D-reconstructed protein complex via cryogenic electron microscopy;
FIG. 74 is a purification and identification and molecular sieve chromatography graph of wild type CCR8 and 5 different CCR8 fusion proteins; wherein, A in FIG. 74 is an SDS gel electrophoresis identification result graph, and B-G in FIG. 74 are molecular sieve chromatography result graphs;
FIG. 75 is a molecular sieve chromatography and SDS gel electrophoresis identification result graph of a purified CRFR1-ICL1-GFP10-11 protein; wherein, A in FIG. 75 is a molecular sieve chromatography result graph, and B in FIG. 75 is an SDS gel electrophoresis identification result graph;
FIG. 76 is a molecular sieve chromatography and SDS gel electrophoresis identification result graph of a purified CRFR1-ICL2-GFP10-11 protein; wherein, A in FIG. 76 is a molecular sieve chromatography result graph, and B in FIG. 76 is an SDS gel electrophoresis identification result graph;
FIG. 77 is an SDS gel electrophoresis identification result graph of a GFP-clamp-avi-biotin protein and a GFP-clamp-Cys-avi-biotin protein; wherein, A in FIG. 77 is an SDS gel electrophoresis identification result graph of a GFP-clamp-avi-biotin protein, and B in FIG. 77 is an SDS gel electrophoresis identification result graph of a GFP-clamp-Cys-avi-biotin protein;
FIG. 78 is a result graph showing the purification and identification of MC4R-GFP10-11 modified protein complex and the determination of its affinity to a ligand; wherein, A in FIG. 78 is a molecular sieve chromatography result graph, and B in FIG. 78 is an SDS gel electrophoresis identification result graph, and C-F in FIG. 78 are affinity determination result graphs;
FIG. 79 is a result graph showing the purification and identification of GPR75-GFP10-11 modified protein complex and the determination of its affinity to a ligand; wherein, A in FIG. 79 is a molecular sieve chromatography result graph, and B in FIG. 79 is an SDS gel electrophoresis identification result graph, and C in FIG. 79 is an affinity determination result graph; and

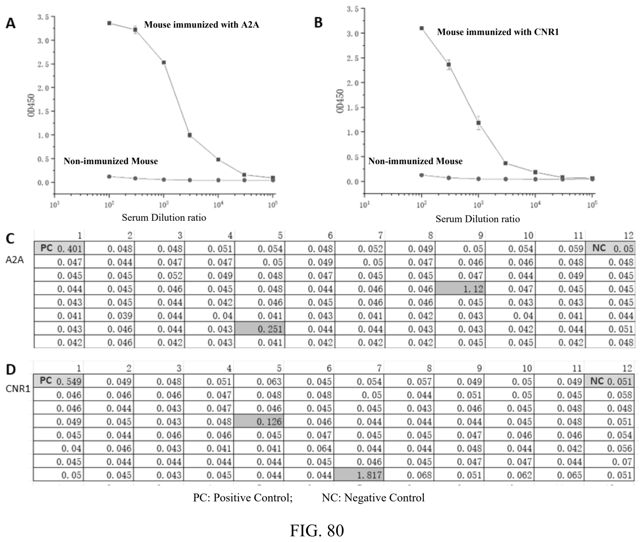

FIG. 80 is an immunodetection result graph of an A2A adenosine receptor modified protein complex and a CNR1 cannabinoid receptor modified protein complex; wherein, A in FIG. 80 is a titer detection result graph of an A2A adenosine receptor modified protein complex on A2A-immunized mouse serum; B in FIG. 80 is a titer detection result graph of a CNR1 cannabinoid receptor modified protein complex on CNR1-immunized mouse serum; C in FIG. 80 is an enzyme-linked immunosorbent assay (ELISA) detection result graph of an A2A adenosine receptor modified protein complex and anti-A2A hybridoma 96-well plate supernatant; D in FIG. 80 is an ELISA detection result graph of a CNR1 cannabinoid receptor modified protein complex and anti-CNR1 hybridoma 96-well plate supernatant.

### DETAILED DESCRIPTION OF THE EMBODIMENTS

Unless otherwise defined, all technical and scientific terms used herein have the same meaning as commonly understood by one of ordinary skill in the art to which this application pertains. For example, Concise Dictionary of Biomedicine and Molecular Biology, Juo, Pei-Show, 2nd edition, 2002, CRC Press; The Dictionary of Cell and Molecular Biology, 5th edition, 2013, Academic Press; and Oxford Dictionary Of Biochemistry And Molecular Biology, 2nd edition, 2006, Oxford University Press, which provide a general dictionary of many terms used in the present application for technical personnel.

As used herein, the expression "A and/B" or "A and/or B" includes three situations: (1) A; (2) B; and (3) A and B. The expression "A, B and/C" or "A, B and/or C" includes seven situations: (1) A; (2) B; (3) C; (4) A and B; (5) A and C; (6) B and C; and (7) A, B and C. The meanings of similar expressions can be deduced by analogy.

In a first aspect, the present application provides a modified G protein coupled receptor, the modified G protein coupled receptor successively comprising, from an N terminus to a C terminus, the N terminus, a first transmembrane domain, a first intracellular loop, a second transmembrane domain, a first extracellular loop, a third transmembrane domain, a second intracellular loop, a fourth transmembrane domain, a second extracellular loop, a fifth transmembrane domain, a third intracellular loop, a sixth transmembrane domain, a third extracellular loop, a seventh transmembrane domain and the C terminus, wherein at least a part of the first intracellular loop, the second intracellular loop and/or the third intracellular loop is replaced with an optional first linker, a first part of a fluorescent protein and an optional second linker, wherein the first linker and the second linker each independently comprise one or more amino acids.

The modified G protein coupled receptor of the present application has relatively good stability in the ligand-free state, its ligand binding site is unoccupied, it has ligand binding activity similar to that of a wild type G protein coupled receptor, and therefore can be used for discovery and screening of the drugs.

Herein, the "G protein coupled receptor" shall be understood in its broadest sense, which is collectively referred to a class of membrane protein receptors. The receptors have common features: they all consist of a polypeptide chain, including 7 transmembrane α-helices, and there are G protein binding sites (guanosine nucleotide-binding proteins) on the C-terminus of the peptide chain and on the intracellular loop (third intracellular loop) linking (counted from the N-terminus of the peptide chain) the fifth and sixth transmembrane helices.

Herein, unless otherwise indicated or the context is clearly contradictory, both a wild-type G protein coupled receptor and a modified G protein coupled receptor are encompassed when referring to the "G protein coupled receptor".

Since the G protein coupled receptors have a similar overall structure and a secondary structure combined mode, and the embodiments of the present application have selected representative members (e.g., A2A, CNR1, NK1R, CCR8, GPRC5D, GCGR, MC4R, GPR75, GPR52, GNRHR, PTGDR2, HTR2C, ADRA2B, ADRB1, ADRB2, C5AR1, CCR2, CCR5, CCR6, CCR7, CHRM2, CLTR2, CXCR2, CXCR4, DRD2, DRD3, GPBAR, HRH1, HTR1A, HTR1B, HTR2B, LPAR1, MTNR1B, NPY1R, OPRD, OX2R, PTGDR, GPR146, MCHR1, TAAR1, AGTR1, FPR1, GALR1, GHSR, CCKAR, MTLR, EDNRA, PRLHR, NPFF1, CXCR1, TRFR, CML1, QRFPR, SSTR2, FFAR1, PTAFR, P2RY1, HCAR2, SUCR1, APJ, GPR39, GPR75, PTGER4, CCR1, CCR4) from numerous subfamilies for experiments. The results show that stable proteins are obtained in all cases so that it can be reasonably inferred that the modified GPCR of the present application is applicable to and cover a wide range of GPCRs, including but not limited to 5-HT1A, 5-HT1B, 5-HT1D, -5-HT1E, 5-HT1F, 5-HT2A, 5-HT2B, 5-HT2C, 5-HT4, 5HT5A, 5-HT6, 5-HT7, M1, M2, M3, M4, M5, A1, A2A, A2B, A3, ADA1A, ADA1B, ADA1D, ADA2A, ADA2B, ADA2C, ADRB1, ADRB2, ADRB3, C3a, C5a, C5L2, AT1, AT2, APJ, GPBA, BB1, BB2, BB3, B1, B2, CB1, CB2, CCR1, CCR2, CCR3, CCR4, CCR5, CCR6, CCR7, CCR8, CCR9, CCR10, CXCR1, CXCR2, CXCR3, CXCR4, CXCR5, CXCR6, CXCR7, CX3CR1, XCR1, CCK1, CCK2, D1, D2, D3, D4, D5, ETA, ETB, GPER, FPR1, FPR2/ALX, FPR3, FFA1, FFA2, FFA3, GPR42, GAL1, GAL2, GAL3, GHSR, FSH, LH, TSH, GnRH, GnRH2, H1, H2, H3, H4, HCA1, HCA2, HCA3, KISSR, BLT1, BLT2, CysLT1, CysLT2, OXE, FPR2/ALX, LPA1, LPA2, LPA3, LPA4, LPA5, S1P1, S1P2, S1P3, S1P4, S1P5, MCH1, MCH2, MC1, MC2, MC3, MC4, MC5, MT1, MT2, MTLR, NMU1, NMU2, NPFF1, NPFF2, NPS, NPBW1, NPBW2, Y1, Y2, Y4, Y5, NTS1, NTS2, delta, kappa, mu, NOP, OX1, OX2, P2Y1, P2Y2, P2Y4, P2Y6, P2Y11, P2Y12, P2Y13, P2Y14, QRFP, PAF, PKR1, PKR2, PRRP, DP1, DP2, EP1, EP2, EP3, EP4, FP. IP1, TP, PAR1, PAR2, PAR3, PAR4, RXFP1, RXFP2, RXFP3, RXFP4, SST1, SST2, SST3, SST4, SST5, NK1, NK2, NK3, TRH1, TA1, UT, V1A, V1B, V2, OT, CCRL2, CMKLR1, GPR1, GPR3, GPR4, GPR6, GPR12, GPR15, GPR17, GPR18, GPR19, GPR20, GPR21, GPR22, GPR25, GPR26, GPR27, GPR31, GPR32, GPR33, GPR34, GPR35, GPR37, GPR37L1, GPR39, GPR42. GPR45, GPR50, GPR52, GPR55, GPR61, GPR62, GPR63, GPR65, GPR68, GPR75, GPR78, GPR79, GPR82, GPR83, GPR84, GPR85, GPR87, GPR88, GPR101, GPR119, GPR120, GPR132, GPR135, GPR139, GPR141, GPR142, GPR146, GPR148, GPR149, GPR150, GPR151, GPR152, GPR153, GPR160, GPR161, GPR162, GPR171, GPR173, GPR174, GPR176, GPR182, GPR183, LGR4, LGR5, LGR6, LPAR6, MAS1, MAS1L, MRGPRD, MRGPRE, MRGPRF, MRGPRG, MRGPRX1, MRGPRX2, MRGPRX3, MRGPRX4, OPN3, OPN5, OXGR1, P2RY8, P2RY10, SUCNR1, TAAR2, TAAR3, TAAR4, TAAR5, TAAR6, TAAR8, TAAR9, CCPB2, CCRL1, FY, CT, CALRL, CRF1, CRF2, GHRH, GIP, GLP-1, GLP-2, GCGR, SCTR, PTH1, PTH2, PAC1, VPAC1, VPAC2, BAI1, BAI2, BAI3, CD97, CELSR1, CELSR2, CELSR3, ELTD1, EMR1, EMR2, EMR3, EMR4P, GPR56, GPR64, GPR97, GPR98, GPR110, GPR111, GPR112, GPR113, GPR114, GPR115, GPR116, GPR123, GPR124, GPR125, GPR126, GPR128, GPR133, GPR143, GPR144, GPR157, LPHN1, LPHN2, LPHN3, CaS, GPRC6, GABAB1, GABAB2, mGlu1, mGlu2, mGlu3, mGlu4, mGlu5, mGlu6, mGlu7, mGlu8, GPR156, GPR158, GPR179, GPRC5A, GPRC5B, GPRC5C, GPRC5D, frizzled, FZD1, FZD2, FZD3, FZD4, FZD5, FZD6, FZD7, FZD8, FZD9, FZD10 and SMO.

In some embodiments, the first linker and the second linker can each optionally be present independently of one another. That is to say, only the first linker or only the second linker is present, neither of the first linker and the second linker is present, or both the first linker and the second linker are present.

In some embodiments, the first linker and the second linker can each be independently selected from a peptide segment comprising 1-50 amino acids, a peptide segment comprising 1-40 amino acids, a peptide segment comprising 1-30 amino acids, a peptide segment comprising 1-20 amino acids, a peptide segment comprising 1-10 amino acids, a peptide segment comprising 1-9 amino acids, a peptide segment comprising 1-8 amino acids, a peptide segment comprising 1-7 amino acids, a peptide segment comprising 1-6 amino acids or a peptide segment comprising 1-5 amino acids.

In some embodiments, the first linker and the second linker can each independently be 1 amino acid, a peptide segment consisting of 2 amino acids, a peptide segment consisting of 3 amino acids, a peptide segment consisting of 4 amino acids, a peptide segment consisting of 5 amino acids, a peptide segment consisting of 6 amino acids, a peptide segment consisting of 7 amino acids, a peptide segment consisting of 8 amino acids, a peptide segment consisting of 9 amino acids, a peptide segment consisting of 10 amino acids or a peptide segment consisting of more than 10 amino acids.

In some embodiments, the first linker and the second linker can be identical or different.

Herein, the "fluorescent protein" encompasses a wild type fluorescent protein and a modified (e.g., obtained by truncation, extension, insertion, deletion or substitution) fluorescent protein.

In some embodiments, the fluorescent protein comprises one or more of a green fluorescent protein (GFP), a red fluorescent protein, a yellow fluorescent protein, a blue fluorescent protein, a cyan fluorescent variant and an enhanced green fluorescent protein.

In some embodiments, the fluorescent protein comprises an amino acid sequence as shown in SEQ ID NO. 81 or an amino acid sequence having at least 50%, at least 60%, at least 70%, at least 80%, at least 90%, at least 95%, at least 96%, at least 97%, at least 98%, at least 99% and at least 99.5% sequence identity to the amino acid sequence as shown in SEQ ID NO. 81.

In some embodiments, the first part of the fluorescent protein comprises β-strands 10-11, β-strands 1-2, β-strands 8-11 or β-strands 1-4 of the fluorescent protein.

In some embodiments, the first part of the fluorescent protein comprises an amino acid sequence as shown in SEQ ID NO. 1 or an amino acid sequence having at least 50%, at least 60%, at least 70%, at least 80%, at least 90%, at least 95%, at least 96%, at least 97%, at least 98%, at least 99% and at least 99.5% sequence identity to the amino acid sequence as shown in SEQ ID NO. 1.

As used herein, the term "identity" refers to similarity degree between a pair of sequences (nucleotides or amino acids). The identity is determined by dividing the number of identical residues by the total number of residues, and multiplying the quotient by 100 to obtain a percentage. Gaps are not taken into account when the identity is evaluated. Therefore, two copies of a completely identical sequence have 100% identity, but a sequence having deletion, addition or substitution may have relatively low identity. It is known to those skilled in the art that there are some computer programs available for determining sequence identity, such as those programs employing algorithms like BLAST. BLAST nucleotide searches are performed using an NBLAST program, and BLAST protein searches are conducted using a BLASTP program, wherein default parameters of each program are used.

In some embodiments, the amino acid at the position 34.51 of the Ballesteros-Weinstein numbering system in the second intracellular loop is cysteine.

In some embodiments, the amino acid at the position 34.51 of the Ballesteros-Weinstein numbering system in the second intracellular loop is naturally cysteine. In some embodiments, the amino acid at the position 34.51 of the Ballesteros-Weinstein numbering system in the second intracellular loop is mutated to cysteine (e.g., represented as GPCR-Cys-GFP10-11).

In some embodiments, the modified G protein coupled receptor comprises an amino acid sequence as shown in any one of SEQ ID Nos. 3-71 or an amino acid sequence having at least 50%, at least 60%, at least 70%, at least 80%, at least 90%, at least 95%, at least 96%, at least 97%, at least 98%, at least 99%, at least 99.5% sequence identity to the amino acid sequence as shown in any one of SEQ ID Nos. 3-71.

In a second aspect, the present application provides a complex, the complex comprising the modified G protein coupled receptor according to the first aspect of the present application and a second part of a fluorescent protein, wherein the first part of the fluorescent protein binds to the second part of the fluorescent protein.

The complex of the present application has good stability in the ligand-free state, its ligand binding site is unoccupied, and it has ligand binding activity similar to that of the wild type G protein coupled receptor and therefore can be used for discovery and screening of the drugs. In addition, the complex of the present application can be used for analysis of a 3D structure via cryogenic electron microscopy, thereby providing further guidance for development and optimization of the drugs.

In some embodiments, a linker group is not contained between the first part of the fluorescent protein and the second part of the fluorescent protein. In other words, the first part of the fluorescent protein binds to the second part of the fluorescent protein via van der Waals forces.

In some embodiments, the first part of the fluorescent protein and the second part of the fluorescent protein jointly constitute a completely fluorescent protein.

In some embodiments, the modified G protein coupled receptor and the second part of the fluorescent protein are co-expressed so that the first part of the fluorescent protein binds to the second part of the fluorescent protein.

In some embodiments, co-expression is performed in a eukaryocyte expression system.

In some embodiments, the eukaryocytes are insect cells or mammalian cells. In some embodiments, the insect cells are Spodoptera frugiperda cells Sf-9. In some embodiments, the mammalian cells are HEK293F cells.

In some embodiments, the expressions of the modified G protein coupled receptor and the second part (e.g., GFP1-9 protein) of the fluorescent protein are respectively driven by different promoters, e.g., by a polyhedrin gene promoter and a P10 promoter respectively.

In some embodiments, the N-terminus of a vector for co-expression has a signal peptide sequence and/or a maltose binding protein fusion tag. In some embodiments, the downstream of the vector for co-expression has an internal ribosome entry site.

In some embodiments, a gene sequence encoding the second part (e.g., GFP1-9 protein) of the fluorescent protein is cloned to the internal ribosome entry site of the expression vector.

In some embodiments, the complete fluorescent protein can be a wild type fluorescent protein, or a modified fluorescent protein.

In some embodiments, the second part of the fluorescent protein comprises β-strands 1-9, β-strands 3-11, β-strands 1-7 or β-strands 5-11 of the fluorescent protein.

In some embodiments, the first part of the fluorescent protein comprises β-strands 10-11 of the fluorescent protein, and the second part of the fluorescent protein comprises β-strands 1-9 of the fluorescent protein. In some embodiments, the first part of the fluorescent protein comprises β-strands 1-2 of the fluorescent protein, and the second part of the fluorescent protein comprises β-strands 3-11 of the fluorescent protein. In some embodiments, the first part of the fluorescent protein comprises β-strands 8-11 of the fluorescent protein, and the second part of the fluorescent protein comprises β-strands 1-7 of the fluorescent protein. In some embodiments, the first part of the fluorescent protein comprises β-strands 1-4 of the fluorescent protein, and the second part of the fluorescent protein comprises β-strands 5-11 of the fluorescent protein.

In some embodiments, the second part of the fluorescent protein comprises an amino acid sequence as shown in SEQ ID NO. 2 or an amino acid sequence having at least 50%, at least 60%, at least 70%, at least 80%, at least 90%, at least 95%, at least 96%, at least 97%, at least 98%, at least 99%, at least 99.5% sequence identity to the amino acid sequence as shown in SEQ ID NO. 2.

In some embodiments, the first part of the fluorescent protein comprises an amino acid sequence as shown in SEQ ID NO. 1 or an amino acid sequence having 50%, 60%, 70%, 80%, 90%, 95%, 96%, 97%, 98%, 99%, 99.5% sequence identity to the amino acid sequence as shown in SEQ ID NO. 1, and the second part of the fluorescent protein comprises an amino acid sequence as shown in SEQ ID NO. 2 or an amino acid sequence having 50%, 60%, 70%, 80%, 90%, 95%, 96%, 97%, 98%, 99%, 99.5% sequence identity to the amino acid sequence as shown in SEQ ID NO. 2.

In some embodiments, the first part of the fluorescent protein comprises an amino acid sequence as shown in SEQ ID NO. 1, and the second part of the fluorescent protein comprises an amino acid sequence as shown in SEQ ID NO. 2.

In some embodiments, the complex further comprises a clamp protein which binds to the first part of the fluorescent protein and the second part of the fluorescent protein.

In some embodiments, linker groups are not contained between the clamp protein and the first part of the fluorescent protein and between the clamp protein and the second part of the fluorescent protein. In other words, the clamp protein binds to the first part of the fluorescent protein and the second part of the fluorescent protein via van der Waals forces.

In some embodiments, the clamp protein comprises a biomarker. The biomarker can be any biomarker known by those skilled in the art, for example can be any one of any molecule pairs capable of specifically binding, the molecule pairs capable of specifically binding include but are not limited to biotin/avidin (e.g., biotin/streptavidin, biotin/neutravidin), antibody/antigen, antibody/hapten, DNA/complementary DNA or RNA, enzyme/substrate, enzyme/inhibitor, enzyme/cofactor, receptor/ligand (e.g., hormone/hormone receptor, folic acid/folate receptor), lectin/carbohydrate, staphylococcal protein A/IgG, cation/anion, spy-tag/spy-catcher, strep-tag (and its mutants)/streptavidin (its mutants). In some embodiments, the clamp protein comprises the biotin marker. In some embodiments, the clamp protein comprises an avi tag. For compound drug screening and therapeutic antibody drug discovery using purified GPCR, it is highly convenient if the target GPCR has a biotin tag, as the target protein can be immobilized or modified through the extremely strong binding between the biotin and streptavidin.

In some embodiments, the complex is a ternary complex formed by the modified G protein coupled receptor and the second part of the fluorescent protein as well as the clamp protein.

In some embodiments, the molecular weight of the ternary complex is more than 85 kD.

In some embodiments, the ternary complex can be purified through affinity chromatography and molecular sieve chromatography.

The clamp protein can be any protein that can bind to the first part of the fluorescent protein or the second part of the fluorescent protein so as to immobilize or encircle the first part of the fluorescent protein or the second part of the fluorescent protein. For example, in the case that the fluorescent protein is GFP, the first part of the fluorescent protein can comprise an amino acid sequence as shown in SEQ ID NO. 1, the second part of the fluorescent protein can comprise an amino acid sequence as sown in SEQ ID NO. 2, and the clamp protein can comprise an amino acid sequence (GFP clamp) as shown in SEQ ID NO. 72.

In some embodiments, the clamp protein comprises an amino acid sequence as shown in any one of SEQ ID Nos. 72-74 and SEQ ID NO. 82 or an amino acid sequence having at least 50%, at least 60%, at least 70%, at least 80%, at least 90%, at least 95%, at least 96%, at least 97%, at least 98%, at least 99%, at least 99.5% sequence identity to the amino acid sequence as shown in any one of SEQ ID Nos. 72-74 and SEQ ID NO. 82.

In some embodiments, cysteine is located at the position 186 of the clamp protein, and the position refers to SEQ ID NO. 72.

In some embodiments, cysteine is naturally located at the position 186 of the clamp protein, and the position refers to SEQ ID NO. 72. In some embodiments, the position 186 of the clamp protein is mutated to cysteine, and the position refers to SEQ ID NO. 72, and therefore the resulting clamp protein can comprise an amino acid sequence (GFP clamp-Cys) as shown in SEQ ID NO. 73.

In some embodiments, a disulfide bond is formed between the amino acid at the position 186 of the clamp protein and the amino acid at the position 34.51 of the Ballesteros-Weinstein numbering system in the second intracellular loop. In some embodiments, other linker groups, other than the disulfide bond, are not contained between the clamp protein and the first part of the fluorescent protein and between the clamp protein and the second part of the fluorescent protein.

In a spatial structure, GPCRs have certain common commonalities in their spatial structures: they comprise 7 transmembrane helices; and the N-terminus and 3 interhelical loop structures are located outside the cell, and the C-terminus and the other 3 interhelical loop structures are located inside the cell. These proteins are located on a cell membrane, and as receptors of signal molecules, transmit signals to the interior of the cell. A foreign signal molecule binds to a part of GPCR located at the outer side of the cell, causing the conformational change of the transmembrane helix of the receptor so that the intracellular part of the receptor binds to the G protein and triggers the physiological effect inside the cell. GPCR is involved in a wide variety of physiological processes and plays a crucial role in maintaining normal life activities in the human body. The drug molecule binds to these receptor proteins to generate agonistic or inhibitory effects on them, thereby regulating the physiological process inside the cell. Analyzing the complex structures formed by these drugs or potential drugs and their target GPCR proteins has significant guiding significance for studying the mechanism of action of these compounds and further drug development.

In a third aspect, the present application provides use of the modified G protein coupled receptor according to the first aspect of the present application or the complex according to the second aspect of the present application in ligand affinity determination and drug discovery or screening.

For the use in ligand affinity determination, drug discovery or screening, the following conditions are not necessary: (1) the amino acid at the position 34.51 of the Ballesteros-Weinstein numbering system in the second intracellular loop is cysteine; (2) the amino acid at the position 186 of the clamp protein is cysteine; and (3) the disulfide bond is formed between the amino acid at the position 186 of the clamp protein and the amino acid at the position 34.51 of the Ballesteros-Weinstein numbering system in the second intracellular loop. In other words, regardless of meeting the above-mentioned conditions, both the modified G protein coupled receptor according to the first aspect of the present application and the complex according to the second aspect can be used for ligand affinity determination and drug discovery or screening.

In a fourth aspect, the present application provides use of the complex according to the second aspect of the present application in analysis of a 3D structure via cryogenic electron microscopy.

In some embodiments, the amino acid at the position 34.51 of the Ballesteros-Weinstein numbering system in the second intracellular loop of the modified G protein coupled receptor is cysteine, cysteine is located at the position 186 of the clamp protein, and the disulfide bond is formed between the amino acid at the position 186 of the clamp protein and the amino acid at the position 34.51 of the Ballesteros-Weinstein numbering system in the second intracellular loop.

In some embodiments, provided is use of the complex according to the second aspect of the present application in the analysis of the 3D structure of the modified G protein coupled receptor via cryogenic electron microscopy.

In some embodiments, provided is use of the complex according to the second aspect of the present application in the analysis of the 3D structure of the complex via cryogenic electron microscopy.

In some embodiments, provided is use of the complex according to the second aspect of the present application in the analysis of the 3D structure of a complex formed by binding a compound molecule to the complex via cryogenic electron microscopy.

In some embodiments, the compound molecule is the inhibitor or agonist of the G protein coupled receptor.

The complex of the present application has relatively good stability in the ligand-free state, its ligand binding site is unoccupied, and it has ligand binding activity similar to that of the wild type G protein coupled receptor and therefore can be used for discovery and screening of the drugs. In addition, the complex of the present application can be used for analyzing the 3D structure of the modified G protein coupled receptor, the complex formed by binding the complex or compound molecule to the complex via cryogenic electron microscopy, thereby providing further guidance for development and optimization of the drug.

In the modified G protein coupled receptor provided in the present application, a short fusion fragment is inserted into a specific position of GPCR. The short fusion fragment is utilized to bind to two other chaperone proteins so that the size of the whole complex particle is more than 85 kD, and the chaperone proteins are sufficiently close to GPCR to form a structure with a certain rigidity. Amino acids at adjacent positions on the GPCR and one of its chaperone proteins are mutated to cysteines, thereby forming the disulfide bond between them, and then reducing the flexibility of the entire complex. Thus, the 3D structure of the complex formed by the GPCR and the ligand compound can be analyzed via cryogenic electron microscopy.

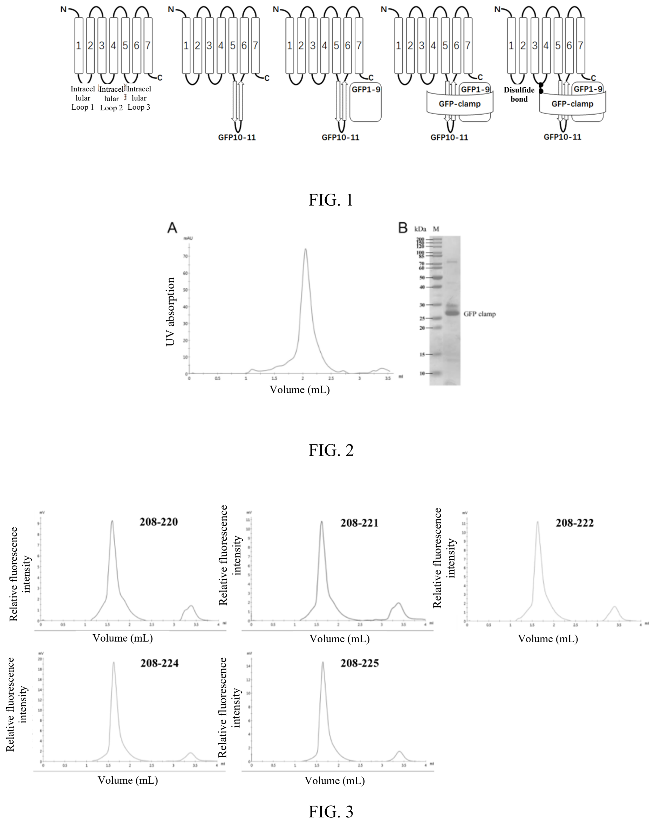

In a specific embodiment, by utilizing the property that the green fluorescent protein can be divided into two parts, i.e., GFP1-9 comprising first-ninth sheets (i.e., β-strands) and GFP10-11 comprising tenth-eleventh sheets and the two parts can be spontaneously bound, a first intracellular loop between a first transmembrane helix (TM1) and a second transmembrane helix (TM2) of GPCR, a second intracellular loop between a third transmembrane helix (TM3) of GPCR and a fourth transmembrane helix (TM4) of GPCR, or a third intracellular loop between a fifth transmembrane helix (TM5) and a sixth transmembrane helix (TM6) of GPCR is replaced with GFP10-11 via a genetic engineering method. For the A2A adenosine receptor, GFP10-11 can be inserted between L208 (Ballesteros-Weinstein numbering system 5.69a) and L225 (Ballesteros-Weinstein numbering system 6.27a), and linker peptide segments, as linkers, can be optionally added at two ends. For other GPCRs, GFP10-11 can be inserted into the position (between positions 5.69a and 6.27a of the Ballesteros-Weinstein numbering system) corresponding to that in the A2A adenosine receptor, or 1, 2, 3, 4 or 5 amino acids at the linker can be replaced with other amino acid sequences, such as a corresponding sequence in the A2A adenosine receptor. Such the GFP10-11 insertion fragment is flexibly connected with the first and second helices, the third and fourth helices or the fifth and sixth of GPCR, which does not affect GPCR to be folded into a correct structure, and does not form continuous helices together with a GPCR sequence at the insertion position to reduce the limitation of the GPCR structure so that the modified GPCR can still bind to a ligand molecule. The fusion protein (GPCR-GFP10-11, i.e., the modified GPCR of the present application) formed by this GPCR and the GFP10-11 fragment together with a GFP1-9 fragment is co-expressed in the cell, and the GFP10-11 fragment in the GPCR-GFP10-11 fusion protein binds to GFP1-9 to form a complete fluorescent GFP molecule. In the subsequent purification process, a GFP-clamp protein with a specific sequence is added to bind to a GFP portion. The complex formed by such the modified GPCR such as A2A adenosine receptor and chaperone proteins GFP1-9 and GFP-clamp exhibits relatively strong rigidity as a whole, and can be used for structural analysis via cryogenic electron microscopy so that a high-resolution complex structure formed with an inhibitor compound is obtained. In addition, to further improve the overall rigidity of the complex formed by the modified GPCR and chaperone proteins GFP1-9 and GFP-clamp, the amino acid at the position 34.51 of the Ballesteros-Weinstein numbering system in the second intracellular loop of GPCR is mutated to cysteine, and meanwhile the amino acid at the position 186 on the GFP-clamp is mutated to cysteine. In this way, the disulfide bond is formed between GPCR and GFP-clamp. The generation of the disulfide bond induces crosslinking between GPCR and GFP-clamp. In non-reductive SDS electrophoresis, one strip with increased molecular weight is presented; and in reductive SDS electrophoresis, the disulfide bond is reduced, but the own strips of two proteins are still presented, so it can be used for identifying the formation of the disulfide bond. The design of the disulfide bond in this way greatly reduces the flexibility of the overall complex, makes the overall structure have relatively strong rigidity, and is suitable for structural analysis via cryogenic electron microscopy. The diagram of the modified G protein coupled receptor of the present application is seen in FIG. 1.

**The present application has the following benefits:**
(1)The modified G protein coupled receptor obtained in the present application has a molecular weight of up to more than 85 kD; since the replacement fragment (the optional first linker, the first part of the fluorescent protein and the optional second linker) inserted onto GPCR is simultaneously connected with two sites on GPCR while binding to the second part of the fluorescent protein and two chaperone proteins of the clamp protein, the overall complex has a relatively strong rigidity in the spatial structure, which is conducive to structural analysis via cryogenic electron microscopy; and the formation of the disulfide bond makes the benefits more obvious.
(2) The fragment of the first part (e.g., GFP10-11) of the fluorescent protein is very short, so its insertion has little impact on GPCR expression and correct folding; moreover, it does not form a continuous helical structure with the helices on the GPCR, resulting in minimal constraint on GPCR conformation, and therefore it is suitable for structural analysis of various types of GPCRs with conformational differences.
(3) The stable complex formed by the modified GPCR of the present application and the second part of the fluorescent protein is well expressed in both insect cells and mammalian cells.
(4) The formed complex is not easily dissociated in the process of purification, and does not need to conduct lots of exploration experiments on the formation conditions and purification conditions of the complex.
(5) The structure of the complex formed by GPCR and the agonist can be analyzed, and the structure of the complex formed by GPCR and the inhibitor can also be analyzed.
(6) The formation of the complex with chaperone proteins (i.e., the second part of the fluorescent protein and the clamp protein) does not require the addition of the ligand. The expensive ligand compound can be added to bind to the complex when the sample is prepared for cryo-EM after sample purification, thereby significantly reducing costs.
(7) The purification of a batch of protein complexes can be used for preparing multiple complexes formed with different small-molecule compounds, thereby decreasing time and work load and reducing costs.
(8) The fluorescence characteristics of the fluorescent protein are conducive to expression and screening of a small number of samples using a fluorescence-detected size exclusion chromatography, thereby saving costs.
(9) The antibody screening process that is time-consuming and low in certainty is not needed.
(10) The universality is good, and many types of GPCRs can all be used for obtaining stable proteins.
(11) GPCR proteins without ligands and with native conformation can be obtained, which have natural ligand binding activity similar to that of a wild type GPCR, and are suitable for screening of compound drugs and discovery of functional monoclonal antibodies.

### Examples

Experimental methods in examples described below, unless specially stated, are all conventional methods. Pharmaceutical reagents used in examples described below, unless specially stated, are all purchased from conventional biochemical reagent stores.

### Example 1: Structural analysis of A2A adenosine receptor and ZM241385 complex via cryogenic electron microscopy

### (1) Expression and purification of GFP-clamp

The DNA sequence encoding a GFP-clamp protein (SEQ ID NO. 72) was cloned to an Escherichia coli expression plasmid pMALc2x such that the N-terminus of the GFP-clamp protein had a maltose-binding protein fusion tag and a tobacco etch virus (TEV) protease digestion site, and the C-terminus of the GFP-clamp protein had a histidine fusion tag. The plasmid was transformed into Rosetta^{™} 2 (DE3) competent cells. Positive single colonies were picked and inoculated into a 20 mL LB culture medium supplemented with antibiotics, bacterial cells were incubated on a small scale until they were in a supersaturated state; 20 mL of bacterial cells was transferred to a 800 mL culture medium containing ampicillin and chloramphenicol for expansion; and when the absorbance value of a bacterial suspension at 600 nm was measured as about 0.6, 0.5 mM isopropyl-β-D-thiogalactoside was added, and then the above mixed bacterial suspension was subjected to induction overnight at 20°C. The induced bacterial suspension was collected in a large volume of centrifugal bottle and then centrifuged for 30 min at 4000 rpm at 4°C, the supernatant culture medium was discarded and pellets were retained. The pellets were resuspended with a buffer solution (500 mM NaCl, 5% glycerol, 20 mM imidazole, and 50 mM Tris-HCl pH 7.8), appropriate amounts of lysozyme, nuclease and phenylmethylsulfonyl fluoride were added, and then bacteria were disrupted using a cell ultrasonic disruptor. The suspension subjected to ultrasonic treatment was centrifuged again for 30 min at 20000 g at 4°C, and then supernatant was retained. A proper volume of Ni-NTA resin was taken and bound to the supernatant, and then the above materials were evenly mixed for 1.5 h at 4°C under the condition of rotation. After sufficient binding, the above mixed solution was centrifuged for 2 min at 2000 rpm at 4°C, supernatant was discarded, and the resin binding to the protein was retained. The Ni-NTA resin was transferred to a gravity column. The above-mentioned buffer solution was used to wash away impurity proteins, with approximately 15-20 column volumes being washed. The protein was eluted with an elution buffer (500 mM NaCl, 5% glycerol, 500 mM imidazole, 50 mM Tris-HCl, pH 7.8) and the eluate was collected, and then the concentration of the protein was determined. The TEV protease was added in a mass ratio of 1:10 for digestion overnight. The digested product was diluted with a buffer (500 mM NaCl, 5% glycerol, 50 mM Tris-HCl, pH 7.8) until the concentration of imidazole was 20 mM, and then bound to a proper amount of Ni-NTA resin again. The resin was washed with a buffer (500 mM NaCl, 5% glycerol, 20 mM imidazole, 50 mM Tris-HCl, pH 7.8), and then the protein was eluted using a buffer (500 mM NaCl, 5% glycerol, 500 mM imidazole, 50 mM Tris-HCl , pH 7.8). The aggregation state and purity of the protein was analyzed by molecular sieve chromatography and SDS-PAGE glue (see FIG. 2).

### (2) Optimization of fusion protein formed by A2A adenosine receptor and GFP10-11

The amino acid sequence of a human A2A adenosine receptor was derived from GenBank with the accession number NM_000675.6. The first amino acid at the N-terminus and the C-terminus sequence following the position 318 were removed. The third intracellular loop is located between L208 and L225. The DNA sequence encoding the GFP10-11 fragment (SEQ ID NO. 1) was inserted between the two amino acids via overlap polymerase chain reaction (PCR) to replace the original third intracellular loop sequence. To optimize the linker peptide segment to maintain the stability of the fusion protein and reduce flexibility, a series of combinations of positions and linker lengths were designed. All combined proteins were identified and analyzed using fluorescence-detected size exclusion chromatography (FSEC).

First, the linking position of the A2A adenosine receptor and GFP10-11 was optimized: GFP10-11 was linked between Leucine (L) at the position 208 in the TM5 transmembrane domain of the A2A adenosine receptor and arginine (R) at the position 220 in the TM6 transmembrane domain of the A2A adenosine receptor, and there were linker peptides (i.e., linkers) containing 5 amino acids on each side, wherein glycine-serine-glycine-glycine-glycine (GSGGG) (i.e., a first linker) was located at the left side, and glycine-glycine-serine-glycine-glycine (GGSGG) (i.e., second linker) was located at the right side, and this combination was denoted as: L208_GSGGG_GFP10-11_GGSGG_R220 (a). Similarly, the modified constructs at other linking positions were respectively as follows: L208_GSGGG_GFP10-11_GGSGG_A221 (b); L208_GSGGG_GFP10-11_GGSGG_R222 (c); L208_GSGGG_GFP10-11_GGSGG_T224 (d); and L208_GSGGG_GFP10-11_GGSGG_L225 (e). FIG. 3 is a screening result graph showing that at least a part of a third intracellular loop of an A2A adenosine receptor is replaced with an optional first linker, a first part (β-strands 10-11 of GFP, referred to as GFP10-11 for short) of a fluorescent protein and an optional second linker, wherein replacement positions are shown. According to the screening results, GFP10-11 was selected to be linked between Leucine (L) at the position 208 in the TM5 transmembrane domain of the A2A adenosine receptor and Leucine (L) at the position 225 in the TM6 transmembrane domain of the A2A adenosine receptor.

Then, the linkers between the A2A adenosine receptor and GFP10-11 were optimized. The linkers with different lengths were designed at two sides of GFP10-11: L208_GSGG_GFP10-11_GGSG_L225; L208_GGG_GFP10-11_GGG_L225; L208_GG_GFP10-11_GG_L225; L208_GSGGG_GFP10-11_GGSGG_L225; L208_G_GFP10-11_G_L225; L208_GGG_GFP10-11_GG_L225; L208_GG_GFP10-11_GGG_L225; L208_GG_GFP10-11_G_L225; L208_G_GFP10-11_GG_L225; L208_G_GFP10-11_L225; L208_GFP10-11_G_L225; and L208_GFP10-11_L225.

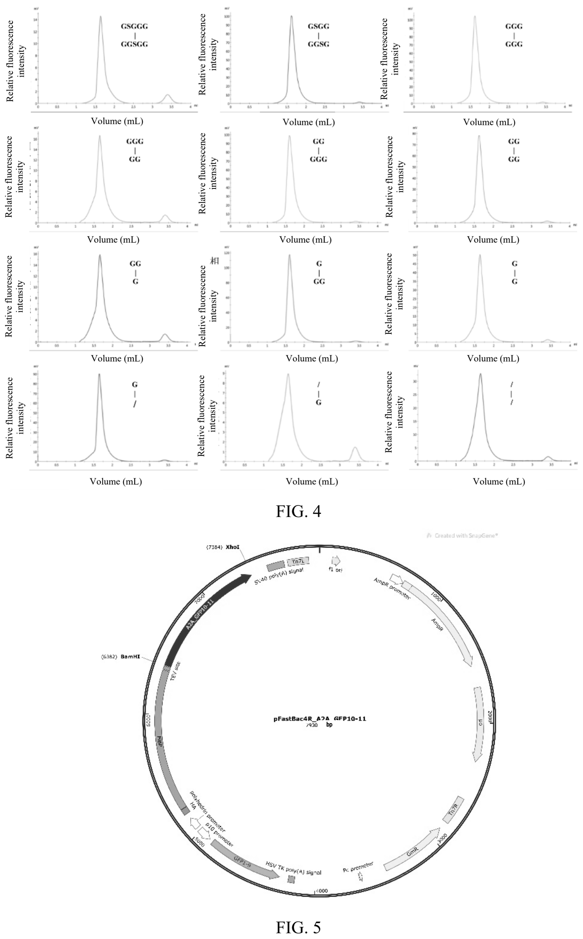

The different A2A adenosine receptor modified sequences described above were cloned to a mammalian cell expression vector pBacMam4R, and the N-terminus had a signal peptide sequence and maltose-binding protein fusion tag as well as a TEV protease digestion site. The vector also had an internal ribosome entry site. The gene sequence encoding the GFP1-9 fragment (SEQ ID NO. 2) was cloned to the internal ribosome entry site, and the A2A-GFP10-11 fusion protein and the GFP1-9 fragment were then simultaneously expressed in the cell, so that they were spontaneously bound to form a complex. The constructed vector DNA was transiently transfected to 293T cells using a lipofectamine 2000 transfection reagent. After 40 h, the cells were collected, weighed and subjected to ultrasonication, lauryl maltose neopentyl glycol (LMNG) with the concentration of 1% and cholesterol hemisuccinate (CHS) with the concentration of 0.1% were added to dissolve the cell membrane, and 50 µg of GFP-clamp protein was added to every 100 mg of cells. After incubation for 1.5 h, the cells were centrifuged at 17000 g, and supernatant was taken and analyzed using fluorescence-detected size exclusion chromatography (SEC). The fluorescence of chromatography column effluent was detected using 488 nm exciting light and 510 nm emitting light (see FIG. 4). The folding correctness and polymerization state of the protein were determined according to the positions and shapes of fluorescence peaks. Finally, the L208_G_GFP10-11_L225 sequence was selected for expression and structural analysis. The amino acid sequence of the modified A2A adenosine receptor is SEQ ID NO. 3:

PIMGSSVYITVELAIAVLAILGNVLVCWAVWLNSNLQNVTNYFVVSLAAADIA VGVLAIPFAITISTGFCAACHGCLFIACFVLVLTQSSIFSLLAIAIDRYIAIRIPLRYNGLV TGTRAKGIIAICWVLSFAIGLTPMLGWNNCGQPKEGKNHSQGCGEGQVACLFEDVVP MNYMVYFNFFACVLVPLLLMLGVYLRIFLAARRQLGHYLSTQTSLSKDLNEKRDHM VLLEFVTAAGLQKEVHAAKSLAIIVGLFALCWLPLHIINCFTFFCPDCSHAPLWLMYL AIVLSHTNSVVNPFIYAYRIREFRQTFRKIIRSHVLRQQEPFKAAG (the sequence of the GFP10-11 fragment is marked with underline, and the sequence of the short peptide at the linker is marked with a wavy line).

### (3) Structural analysis of A2A adenosine receptor and ZM241385 complex via cryogenic electron microscopy

In the modified pFastbacDual plasmid, a maltose-binding protein sequence with a hemagglutinin signal sequence at the N-terminus was added in a downstream sequence of a polyhedrin gene promoter (see FIG. 5), the nucleotide sequence encoding the A2A adenosine receptor (SEQ ID NO. 3) fused with the GFP10-11 fragment determined in (2) was cloned to the C-terminus of the maltose-binding protein, and the nucleotide sequence encoding the GFP1-9 fragment was cloned to the downstream of a P10 gene promoter. Recombinant baculoviruses were generated using a standard Bac-to-Bac baculovirus expression system operating procedure: the above-mentioned plasmid was transformed into competent DH10Bac competent cells, and incubated for 48 h in an incubator at 37°C using an LB agar culture medium containing Kanamycin, gentamicin and tetracycline as well as IPTG and Bluo-gal; by blue-white screening, white positive single colonies were picked and incubated overnight in a 5 mL LB liquid culture medium; and on the next day, the collected bacterial cells were centrifuged, and recombinant bacmid DNA was extracted from the bacterial cells. The purified recombinant bacmid DNA was transfected into *Spodoptera frugiperda* Sf-9 cells using Cellfectin transfection reagent. After 4-5 days, P0-generation recombinant baculoviruses were harvested. The Sf-9 cells were infected with the P0-generation viruses for expansion so as to obtain high-titer baculoviruses.

The high-titer baculoviruses were added to the Sf-9 cells with good growth conditions and the cell density of 2×10⁶/mL for infection. The above infected cells were subjected to shaking cultivation for 48 h in a constant temperature incubator at 27°C at 125 rpm, and then centrifuged to collect the cells. The cells were re-suspended with a buffer solution (500 mM NaCl, 5% glycerol, 50 mM Tris-HCl pH 7.8), and then disrupted using the ultrasonic cell disruptor. Lauryl maltose neopentyl glycol (LMNG) with a final concentration of 1% and cholesterol hemisuccinate (CHS) with a final concentration of 0.1% were added to the above disrupted cells. The above mixture solution was rotated for 2 h at 4°C to dissolve the cell membrane. The mixture solution was centrifuged for 30 min at 20000 g at 4°C. The supernatant was evenly mixed with an appropriate volume of amylose resin under the condition of rotation at 4°C for 1.5 h. After sufficient binding, the above mixed solution was centrifuged for 2 min at 2000 rmp at 4°C, supernatant was discarded, and the resin binding to the protein was retained. The amylose resin was transferred into the gravity column. Impurity proteins were washed away using a washing buffer (500 mM NaCl, 5% glycerol, 50 mM Tris-HCl pH 7.8, 0.01% LMNG, 0.001% CHS). A target protein was eluted with an elution buffer (500 mM NaCl, 5% glycerol, 20 mM maltose, 50 mM Tris-HCl pH7.8, 0.01% LMNG, 0.001% CHS) and collected for determining the concentration. Then, the protein was concentrated using a centrifugal filter-10K until the final volume was 500 µL. The concentrated protein was isolated using a Superdex 200 Increase 10/300 GL molecular sieve chromatography column, wherein the mobile phase was a buffer (500 mM NaCl, 5% glycerol, 50 mM Tris-HCl pH 7.8, 0.01% LMNG, 0.001% CHS), and the protein at a peak tip region of an ultraviolet absorption peak corresponding to the target protein complex was collected. An appropriate amount of excessive GFP-clamp protein was added to the collected protein to form a complex. The complex was isolated using the Superdex 200 Increase 10/300 GL molecular sieve chromatography column (see A of FIG. 6), wherein the mobile phase is a buffer (150 mM NaCl, 20 mM HEPES pH 7.4, 0.005% LMNG, 0.0005% CHS), and the protein at a peak tip region of an ultraviolet absorption peak corresponding to the target protein complex was collected and concentrated using the centrifugal filter until the concentration of the protein was 10-15 mg/mL. The components of the obtained sample were analyzed and identified through SDS-PAGE gel electrophoresis (see B of FIG. 6). The molecular sieve chromatography and SDS gel electrophoresis demonstrated that the protein possessed favorable properties and high purity, with each component contained in the protein consistent with expectations; the purified protein formed a non-aggregated UV absorption peak in molecular sieve chromatography, thereby proving good stability.

ZM241385 was dissolved into dimethylsulfoxide (DMSO) at the concentration of 100 mM, diluted by 100-fold with water until the concentration was 1 mM, and then added to the purified and concentrated protein sample in a volume ratio of 10%, so that the final concentration of ZM241385 was 100 µM. Hydrophilization was performed on a Quantifoil Cu 300 1.2/1.3 electron microscope grid using a PELCO easiGlow glow discharge device. 3 µL of protein sample was added on a hydrophilized grid using a Vitrobot cryo-sample preparation instrument. After waiting for 4 seconds, blotting was performed under the conditions of 1-6 force degrees and 3-5 seconds, and then the protein sample was quickly inserted into liquid ethane pre-cooled with liquid nitrogen for cryo-EM and subsequently transferred to liquid nitrogen for storage. The prepared frozen sample was loaded into a Titan Krios cryogenic electron microscope equipped with a Gatan K2 direct electron detector camera, and image data of protein particles in a sample well were collected at the acceleration voltage of 300 KV. Data were collected by adopting a super-resolution mode. Specific parameters were as follows: the size of pixel was 0.55Å, each image was composed of 40 frames, and the total electron dose was 60e-/Å²/s.

The collected data were used to correct the displacement induced by the electron beam using MotionCor2. The parameters of the contrast transfer function of each image were estimated in CryoSPARC software, followed by selecting protein particles and extracting the images of the protein particles. After several rounds of 2D classification and removal of error particles (see C of FIG. 6), an initial 3D density model was reconstructed ab initio. Heterogeneous refinement was performed on these different reconstructed models, and the classes with correct shapes and the highest quality were selected therefrom. The above-mentioned 2D classification and 3D heterogeneous refinement could be performed many rounds to obtain the highest-quality protein particle image and density model. Finally, a high-quality 3D density (see E of FIG. 6) was obtained through homogenous refinement and non-uniform refinement (see D of FIG. 6). The structure model of the protein complex was established using Coot software according to the 3D density obtained from cryogenic electron microscopy data, and the molecular structure model of the small-molecule compound was established according to the density of the small-molecule compound (see F of FIG. 6). Then, the structure refinement was performed using Phenix software to obtain a high-quality structure model.

### Example 2: Modification, expression and purification of human cannabinoid type I receptor (CNR1) according to the method in Example 1

The sequence of the human cannabinoid type I receptor (CNR1) was derived from GenBank number NM_001160226.3. The N-terminus and C-terminus regions of the sequence were removed, stability mutation was made to the sequence with reference to the known structure, the GFP10-11 fragment (marked with an underline) was fused into the third intracellular loop, and a linker was marked with a wavy line. The amino acid sequence of the modified CNR1 receptor-GFP10-11 fusion protein is SEQ ID NO.4:

GRGENFMDIECFMVLNPSQQLAIAVLSLTLGTFTVLENLLVLCVILHSRSLRCR
PSYHFIGSLAVADLLGSVIFVYSFIDFHVFHRKDSRNVFLFKLGGVTASFTASVGSLFL
AAIDRYISIHRPLAYKRIVTRPKAVVAFCLMWTIAIVIAVLPLLGWNCEKLQSVCSDIF
PHIDKTYLMFWIGVVSVLLLFIVYAYMYILWKARRQLGHYLSTQTSLSKDLNEKRDH
MVLLEFVTAAGLQKEIELAKTLVLILVVLIICWGPLLAIMVYDVFGKMNKLIKTVFAF
CSMLCLLNSTVNPIIYALRSKDLRHAFRSMFPS

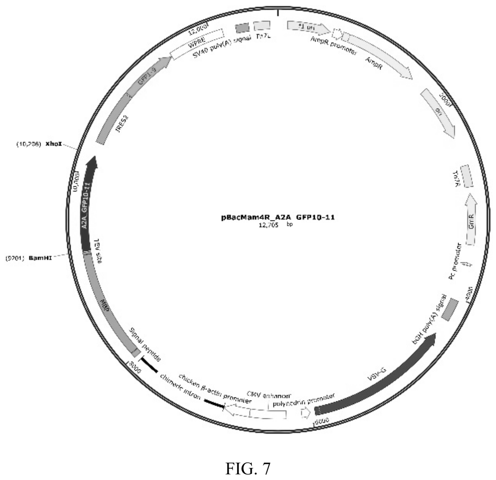

The nucleotide sequence ending the CNR1 receptor-GFP10-11 fusion protein was cloned to a mammalian cell expression vector pBacMam4R (see FIG. 7), and the N-terminus had a signal peptide sequence and a maltose binding protein fusion tag. The internal ribosome entry site (IRES) downstream of the sequence could co-express the GFP1-9 fragment. The cloned plasmid was expanded, and a large number of plasmid DNA was extracted using an Endotoxin-Free Plasmid Maxi-Prep Kit and then transfected into well-growing HEK293F cells via PEI-40K. At 24 h after transfection, 5 mM sodium butyrate was added. After 24 h, the fluorescence of the cells was observed, and the cells were centrifuged for 2 min at 2000 rpm and then collected. The above collected cells were resuspended with a buffer solution (500 mM NaCl, 5% glycerol, 50 mM Tris-HCl pH 7.8), and the cells were disrupted using a cell ultrasonic disruptor. Lauryl maltose neopentyl glycol (LMNG) with a final concentration of 1% and cholesterol hemisuccinate (CHS) with a final concentration of 0.1% were added to the above disrupted cells. The above mixture solution was rotated for 2 h at 4°C to dissolve the cell membrane. The mixture solution was centrifuged for 30 min at 20000 g at 4°C. The supernatant was evenly mixed with an appropriate volume of amylose resin under the condition of rotation at 4°C for 1.5 h. After sufficient binding, the above mixed solution was centrifuged for 2 min at 2000 rpm at 4°C, supernatant was discarded, and the resin binding to the protein was retained. The amylose resin was transferred into the gravity column. Impurity proteins were washed away using a washing buffer (500 mM NaCl, 5% glycerol, 50 mM Tris-HCl pH 7.8, 0.01% LMNG, 0.001% CHS). A target protein was eluted with an elution buffer (500 mM NaCl, 5% glycerol, 20 mM maltose, 50 mM Tris-HCl pH 7.8, 0.01% LMNG, 0.001% CHS) and collected for determining the concentration. Then, the protein was concentrated using a centrifugal filter-10K until the volume was 500 µL. The concentrated protein was isolated using a Superdex 200 Increase 10/300 GL molecularsieve chromatography column, wherein the mobile phase was a buffer (500 mM NaCl, 5% glycerol, 50 mM Tris-HCl pH 7.8, 0.01% LMNG, 0.001% CHS), and the protein at a peak tip region of an ultraviolet absorption peak corresponding to the target protein complex was collected. An appropriate amount of excessive GFP-clamp protein was added to the collected protein to form a complex. The complex was isolated using the Superdex 200 Increase 10/300 GL molecular sieve chromatography column, wherein the mobile phase is a buffer (150 mM NaCl, 20 mM HEPES pH 7.4, 0.005% LMNG, 0.0005% CHS), and the protein at a peak tip region of an ultraviolet absorption peak corresponding to the target protein complex was collected, and concentrated using the centrifugal filter until the concentration of the protein was 10-15 mg/mL. The components of the obtained sample were analyzed and identified through SDS-PAGE gel electrophoresis.

The molecular sieve chromatography and SDS gel electrophoresis demonstrated that the protein possessed favorable properties (see A and B of FIG. 8) and high purity, with each component contained consistent with expectations; the purified protein formed a non-aggregated UV absorption peak in molecular sieve chromatography, thereby proving good stability.

### Example 3: GFP-clamp cysteine mutation, and expression and purification of GFP-clamp-Cys mutant protein

The gluramic acid at the position 186 of GFP-clamp was mutated to cysteine (marked with a border); and other parts were kept unchanged, including a maltose-binding protein and a TEV protease digestion site at the N-terminus, and a histidine tag at the C-terminus. The amino acid sequence of GFP-clamp-Cys is SEQ ID NO. 73:

DLGKKLLEAARAGQDDEVRILMANGADVNAADDVGVTPLHLAAQRGHLEIV
EVLLKYGADVNAADLWGQTPLHLAATAGHLEIVEVLLKNGADVNARDNIGHTPLHL
AAWAGHLEIVEVLLKYGADVNAQDKFGKTPFDLAIDNGNEDIAEVLQKAAGGGSGG

GDVNAYDEVGWTPLHKAAWGHLCKVEDLLKNGADVNAADIDGYTPLHLAAFSGH
LEIVEVLLKYGADVNADDQAGFTPLHLAAIFGHLEIVEVLLKNGADVNAQDKFGKTP
FDLAIDNGNEDIAEVLQKAA

The plasmid having the nucleotide sequence encoding the above-mentioned GFP-clamp-Cys was transformed into Rosetta^{™} 2 (DE3) competent cells. Positive single colonies were picked and inoculated into a 20 mL LB culture medium supplemented with antibiotics, bacterial cells were incubated on a small scale until they were in a supersaturated state; 20 mL of bacterial cells was transferred to a 800 mL culture medium containing ampicillin and chloramphenicol for expansion; and when the absorbance value of a bacterial suspension at 600 nm was measured as about 0.6, 0.5 mM isopropyl-β-D-thiogalactoside was added, and then the above mixed bacterial suspension was subjected to induction overnight at 20°C. The induced bacterial suspension was collected in a large volume of centrifugal bottle and then centrifuged for 30 min at 4000 rpm at 4°C, the supernatant culture medium was discarded, and pellets were retained. The pellets were resuspended with a buffer solution (500 mM NaCl, 5% glycerol, 20 mM imidazole, 2 mM tri(2-carbonylethyl)phosphine hydrochloride (TCEP), 50 mM Tris-HCl pH 7.8), appropriate amounts of lysozyme, nuclease and phenylmethylsulfonyl fluoride were added, and then bacteria were disrupted using a cell ultrasonic disruptor. The above suspension subjected to ultrasonic treatment was centrifuged again for 30 min at 20000 g at 4°C, and then supernatant was retained. A proper volume of Ni-NTA resin was taken and bound to the supernatant, and then the above materials were evenly mixed for 1.5 h at 4°C under the condition of rotation. After sufficient binding, the above mixed solution was centrifuged for 2 min at 2000 rpm at 4°C, supernatant was discarded, and the resin binding to the protein was retained. The Ni-NTA resin was transferred to a gravity column. The above-mentioned buffer was used to wash away impurity proteins, with approximately 15-20 column volumes being washed. The protein was eluted with an elution buffer (500 mM NaCl, 5% glycerol, 500 mM imidazole, 50 mM Tris-HCl, 2 mM TCEP, pH 7.8) and the eluate was collected, and then the concentration of the protein was determined. TEV protease was added in a mass ratio of 1:10 for digestion overnight. The digested product was diluted with a buffer (500 mM NaCl, 5% glycerol, 50 mM Tris-HCl, 2 mM TCEP, pH 7.8) until the concentration of imidazole was 20 mM, and then bound to a proper amount of Ni-NTA resin again. The resin was washed with a buffer (500 mM NaCl, 5% glycerol, 20 mM imidazole, 2 mM TCEP, 50 mM Tris-HCl, pH 7.8), and then the protein was eluted using a buffer (500 mM NaCl, 5% glycerol, 500 mM imidazole, 50 mM Tris-HCl, 2 mM TECP, pH 7.8).The protein buffer was exchanged into another buffer (500 mM NaCl, 5% glycerol, 50 mM Tris-HCl, pH 7.8) using a desalting column. The molecular sieve chromatography and SDS gel electrophoresis identification results of the purified GFP-clamp-Cys protein are seen in FIG. 9.

### Example 4: Structural analysis of cannabinoid type I receptor (CNR1) cysteine mutant and inhibitor taranabant complex

On the basis of the CNR1 and GFP10-11 fusion protein sequence in Example 2, the leucine (Ballesteros-Weinstein numbering system 34.51) at the position 222 in the second intracellular loop was mutated to cysteine (marked with a border). The amino acid sequence of the modified CNR1-Cys_GFP10-11 is SEQ ID NO. 5:

GRGENFMDIECFMVLNPSQQLAIAVLSLTLGTFTVLENLLVLCVILHSRSLRCR
PSYHFIGSLAVADLLGSVIFVYSFIDFHVFHRKDSRNVFLFKLGGVTASFTASVGSLFL
AAIDRYISIHRPCAYKRIVTRPKAVVAFCLMWTIAIVIAVLPLLGWNCEKLQSVCSDIF
PHIDKTYLMFWIGVVSVLLLFIVYAYMYILWKARRQLGHYLSTQTSLSKDLNEKRDH
MVLLEFVTAAGLQKEIELAKTLVLILVVLIICWGPLLAIMVYDVFGKMNKLIKTVFAF
CSMLCLLNSTVNPIIYALRSKDLRHAFRSMFPS

The nucleotide sequence encoding the above SEQ ID NO. 5 was cloned to a mammalian cell expression vector pBacMam4R, and the N-terminus had a signal peptide sequence and a maltose binding protein fusion tag. The internal ribosome entry site (IRES) downstream of the sequence could co-express the GFP1-9 fragment. The cloned plasmid was expanded, and a large number of plasmid DNA was extracted using an Endotoxin-Free Plasmid Maxi-Prep Kit and then transfected into well-growing HEK293F cells via PEI-40K.At 24 h after transfection, 5 mM sodium butyrate was added. After 24 h, the fluorescence of the cells was observed, and the cells were centrifuged for 2 min at 2000 rpm and then collected. The cells were resuspended with a buffer solution (500 mM NaCl, 5% glycerol, 50 mM Tris-HCl pH 7.8), the purified GFP-clamp-Cys protein was added, and the cells were disrupted using a cell ultrasonic disruptor. Lauryl maltose neopentyl glycol (LMNG) with a final concentration of 1% and cholesterol hemisuccinate (CHS) with a final concentration of 0.1% were added to the above disrupted cells. The above mixture solution was rotated for 2 h at 4°C to dissolve the cell membrane. The mixture solution was centrifuged for 30 min at 20000 g at 4°C. The supernatant was evenly mixed with an appropriate volume of Ni-NTA resin under the condition of rotation at 4°C for 1.5 h. After sufficient binding, the above mixed solution was centrifuged for 2 min at 2000 rpm at 4°C, supernatant was discarded, and the resin binding to the protein was retained. The resin was transferred into the gravity column. Impurity proteins were washed away using a washing buffer (500 mM NaCl, 5% glycerol, 20 mM imidazole, 50 mM Tris-HCl pH 7.8, 0.01% LMNG, 0.001% CHS). A target protein was eluted with an elution buffer (500 mM NaCl, 5% glycerol, 500 mM imidazole, 50 mM Tris-HCl, pH 7.8, 0.01% LMNG, 0.001% CHS).The protein eluted from the Ni-NTA resin was bound to an appropriate volume of amylose resin and evenly mixed for 1.5 h at 4°C under the condition of rotation, the above mixture was centrifuged for 2 min at 2000 rpm at 4°C, supernatant was discarded, the amylose resin was transferred to the gravity column, the impurity protein was washed away with a washing buffer (500 mM NaCl, 5% glycerol, 50 mM Tris-HCl, pH 7.8, 0.01% LMNG, 0.001% CHS), and then the target protein was eluted with an eluting buffer (500 mM NaCl, 5% glycerol, 20 mM maltose, 50 mM Tris-HCl, pH 7.8, 0.01% LMNG, 0.001% CHS).After 1 mg of GFP-clamp-Cys was supplemented, the protein was concentrated using a centrifugal filter-10K until the final volume was 500 µL, the concentrated protein was further purified using Superdex 200 Increase 10/300 GL molecular sieve chromatography column (see A of FIG. 10), wherein the mobile phase was a buffer (150 mM NaCl, 20 mM HEPES pH 7.4, 0.005% LMNG, 0.0005% CHS), and the protein at a peak tip region of an ultraviolet absorption peak corresponding to the target protein complex was collected. The sample was respectively mixed with a reductive SDS loading buffer and a non-reductive SDS loading buffer, followed by treatment for 30 min at 37°C, the strips of the components of the obtained sample were analyzed by SDS gel electrophoresis (see B of FIG. 10, wherein sample 1 was a sample treated under the reductive condition, sample 2 was a sample treated under the non-reductive condition; and for the SDS gel electrophoresis of different GPCR samples in examples below, the sample 1 was a sample treated under the reductive condition, the sample 2 was a sample treated under the non-reductive condition). The molecular sieve chromatography and SDS gel electrophoresis demonstrated that the protein possessed favorable properties and high purity, with each component contained consistent with expectations; the purified protein formed a non-aggregated UV absorption peak in molecular sieve chromatography, thereby proving good stability.

The antagonist taranabant of the CNR1 receptor was dissolved into DMSO to prepare a stock solution with the concentration of 200 mM. The stock solution was diluted by 8-fold using DMSO, and then diluted by 100-fold using water to obtain a taranabant solution with the concentration of 250 µM. The taranabant solution was added to the CNR1 protein sample purified via molecular sieve chromatography in a volume of 10%, and then the protein was concentrated using a centrifugal filter-10K until the concentration of the protein was 10-15 mg/mL. Hydrophilization was performed on a Quantifoil Cu 300 1.2/1.3 electron microscope grid using a PELCO easiGlow glow discharge device. 4 µL of protein sample was added on a hydrophilized grid using a Vitrobot cryo-sample preparation instrument. After waiting for 4 seconds, blotting was performed under the conditions of 1-6 force degrees and 3-5 seconds, then the protein sample was quickly inserted into liquid ethane pre-cooled with liquid nitrogen for cryo-EM, and then the frozen protein sample was transferred to liquid nitrogen for storage. The prepared frozen sample was loaded into a Titan Krios cryogenic electron microscope equipped with a Gatan K2 direct electron detector camera, and image data of protein particles in the sample were collected at the acceleration voltage of 300 KV. Data were collected by adopting a super-resolution mode. Specific parameters were as follows: the size of pixel was 0.55 Å, each image was composed of 40 frames, and the total electron dose was 60 e⁻/Å²/s.

The collected data were used to correct the displacement induced by the electron beam using MotionCor2. The parameters of the contrast transfer function of each image were estimated in CryoSPARC software, followed by selecting protein particles and extracting the local images of the protein particles. After several rounds of 2D classification and removal of error particles, an initial 3D density model was reconstructed ab initio. Heterogeneous refinement was performed on these different reconstructed models, and the classes with correct shapes and highest quality were selected therefrom. The above-mentioned 2D classification and 3D heterogeneous refinement could be performed many rounds to obtain the highest-quality protein particle image and density model. Finally, the high-quality 3D density (see D of FIG. 10) was obtained through homogenous refinement and non-uniform refinement (see C of FIG. 10). The structure model of the protein complex was established using Coot software according to the 3D density obtained from cryogenic electron microscopy data, and the molecular structure model of the small-molecule compound was established according to the density of the small-molecule compound (see E of FIG. 10). Then, the structure refinement was performed using Phenix software to obtain a high-quality structure model.

### Example 5: Structural analysis of neurokinin receptor NK1R and inhibitor aprepitant complex

The amino acid sequence of NK1R was derived from derived from GenBank number NM_001058.4. Stability mutation was made to the region of the sequence where the C-terminus was removed with reference to the known structure, a GFP10-11 fragment (marked with an underline) was fused into the third intracellular loop, and a linker was marked with a wavy line. The leucine (Ballesteros-Weinstein numbering system 34.51) at the position 138 in the second intracellular loop was mutated to cysteine (marked with a border). The amino acid sequence of the modified NK1R-Cys_GFP10-11 fusion protein is SEQ ID NO.6:

MDNVLPVDSDLSPNISTNTSEPNQFVQPAWQIVLWAAAYTVIVVTSVVGNVV
VMWIILAHKRMRTVTNYFLVNAAFAEASMAAFNTVVNFTYAVHNEWYYGLFYCKF
HNFFPIAAIFASIYSMTAVAFDRYMAIIHPCQPRLSLTATKVVICVIWVLALLLAFPQG
YYSTTETMPSRVVCKIEWPEHPNKIYEKVYHICVTVLIYFLPLLVIGYLYTVVGITLRR
QLGHYLSTQTSLSKDLNEKRDHMVLLEFVTAAGLQKERKVVKMMIVVVCTFAICWL
PFHIFLLLPYINPDLYLKKFIQQVYLAIMWLAMSSTMYNPIIYCCLNDRFRLGFKHAFR
CCPFISAGDYEGLE

The purification and identification method of the modified NK1R-Cys_GFP10-11 fusion protein refers to Example 4. The molecular sieve chromatography result graph of the modified NK1R-Cys_GFP10-11 fusion protein is seen in A of FIG. 11, and the SDS gel electrophoresis result graph of the modified NK1R-Cys_GFP10-11 fusion protein is seen in B of FIG. 11. The molecular sieve chromatography and SDS gel electrophoresis demonstrated that the protein possessed favorable properties and high purity, with each component contained consistent with expectations; the purified protein formed a non-aggregated UV absorption peak in molecular sieve chromatography, thereby proving good stability.

The antagonist aprepitant of the NK1R receptor was dissolved into DMSO to prepare a stock solution with the concentration of 187 mM. The stock solution was diluted by 16-fold using DMSO and then diluted by 100-fold using water to obtain a taranabant solution with the concentration of 116 µM. The taranabant solution was added to the NK1R protein sample purified via molecular sieve chromatography in a volume of 10%, and then the protein was concentrated using a centrifugal filter-10K until the concentration of the protein was 10-15 mg/mL. The specific method for structural analysis refers to Example 4. The high-quality 3D density (see D of FIG. 11) was obtained through homogenous refinement and non-uniform refinement (see C of FIG. 11). The structure model of the protein complex was established using Coot software according to the 3D density obtained from cryogenic electron microscopy data, and the molecular structure model of the aprepitant was established according to the density of the aprepitant molecule (see E of FIG. 11). Then, the structure refinement was performed using Phenix software to obtain a high-quality structure model.

### Example 6: Structural analysis of A2A adenosine receptor and agonist adenosine complex

The leucine (Ballesteros-Weinstein numbering system 34.51) at the position 110 in the second intracellular loop in the A2A adenosine receptor sequence fused with the GFP10-11 fragment determined in Example 1 was mutated to cysteine (marked with a border). The amino acid sequence of the modified A2A-Cys_GFP10-11 fusion protein is SEQ ID NO.7:

PIMGSSVYITVELAIAVLAILGNVLVCWAVWLNSNLQNVTNYFVVSLAAADIA
VGVLAIPFAITISTGFCAACHGCLFIACFVLVLTQSSIFSLLAIAIDRYIAIRIPCRYNGLV
TGTRAKGIIAICWVLSFAIGLTPMLGWNNCGQPKEGKNHSQGCGEGQVACLFEDVVP
MNYMVYFNFFACVLVPLLLMLGVYLRIFLAARRQLGHYLSTQTSLSKDLNEKRDHM
VLLEFVTAAGLQKEVHAAKSLAIIVGLFALCWLPLHIINCFTFFCPDCSHAPLWLMYL
AIVLSHTNSVVNPFIYAYRIREFRQTFRKIIRSHVLRQQEPFKAAG

The purification and identification method of the modified A2A-Cys_GFP10-11 fusion protein refers to Example 4. The molecular sieve chromatography result graph of the modified A2A-Cys_GFP10-11 fusion protein is seen in A of FIG. 12, and the SDS gel electrophoresis result graph of the modified A2A-Cys_GFP10-11 fusion protein is seen in B of FIG. 12. The molecular sieve chromatography and SDS gel electrophoresis demonstrated that the protein possessed favorable properties and high purity, with each component contained consistent with expectations; the purified protein formed a non-aggregated UV absorption peak in molecular sieve chromatography, thereby proving good stability.

The A2A adenosine receptor complex purified via molecular sieve chromatography was concentrated using a centrifugal filter (a molecular cut off was 10 kDa) until the concentration of the complex was 10-15 mg/ml, and then the adenosine was dissolved into pure water to prepare a stock solution with the concentration of 10 mM. The stock solution was added to the concentrated protein solution in a volume of 10%, so that the final concentration of the adenosine was 1 mM. The specific method for structural analysis refers to Example 4. The high-quality 3D density (see D of FIG. 12) was obtained through homogenous refinement and non-uniform refinement (see C of FIG. 12). The structure model of the protein complex was established using Coot software according to the 3D density obtained from cryogenic electron microscopy data, and the molecular structure model of the adenosine was established according to the density of the adenosine molecule (see E of FIG. 12).Then, the structure refinement was performed using Phenix software to obtain a high-quality structure model.

### Example 7: Structural analysis of chemokine receptor CCR8

The sequence of the chemokine receptor CCR8 was derived from an amino acid sequence with the GenBank accession number NM_005201.4. The first amino acid in the sequence was removed and the GFP10-11 fragment (marked with an underline) was fused into the third intracellular loop, and the linker was marked with a wavy line. The V139 (Ballesteros-Weinstein numbering system 34.51) in the second intracellular loop was mutated to cysteine (marked with a border). The amino acid sequence of the modified fusion protein is SEQ ID NO.8:

DYTLDLSVTTVTDYYYPDIFSSPCDAELIQTNGKLLLAVFYCLLFVFSLLGNSL
VILVLVVCKKLRSITDVYLLNLALSDLLFVFSFPFQTYYLLDQWVFGTVMCKVVSGF
YYIGFYSSMFFITLMSVDRYLAVVHACYALKVRTIRMGTTLCLAVWLTAIMATIPLL
VFYQVASEDGVLQCYSFYNQQTLKWKIFTNFKMNILGLLIPFTIFMFCYIKILHQLRRQ
LGHYLSTQTSLSKDLNEKRDHMVLLEFVTAAGLQKETKAIRLVLIVVIASLLFWVPFN
VVLFLTSLHSMHILDGCSISQQLTYATHVTEIISFTHCCVNPVIYAFVGEKFKKHLSEIF
QKSCSQIFNYLGRQMPRESCEKSSSCQQHSSRSSSVDYIL

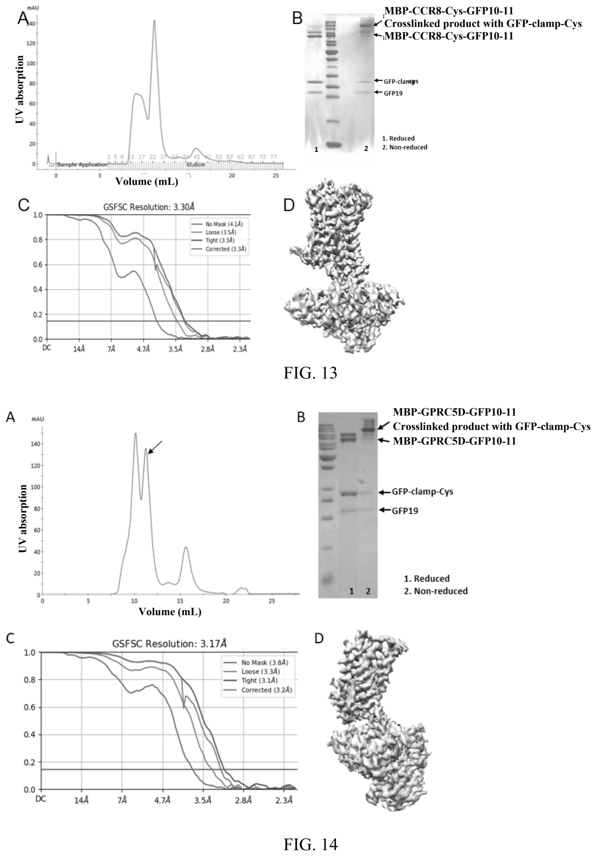

The purification and identification method of the modified fusion protein is seen in Example 4. The molecular sieve chromatography result graph of the modified fusion protein is seen in A of FIG. 13, and the SDS gel electrophoresis result graph of the modified fusion protein is seen in B of FIG. 13. The molecular sieve chromatography and SDS gel electrophoresis demonstrated that the protein possessed favorable properties and high purity, with each component contained consistent with expectations; the purified protein formed a non-aggregated UV absorption peak in molecular sieve chromatography, thereby proving good stability.

The CCR8 protein complex purified via molecular sieve chromatography was concentrated using a centrifugal filter (with a molecular cut off of 10 kDa) until the concentration was 6 mg/ml. The specific method for structural analysis refers to example 4. The high-quality 3D density (see D of FIG. 13) was obtained through homogenous refinement and non-uniform refinement (see C of FIG. 13). The 3D density was obtained from cryogenic electron microscopy data, the structural model of the protein complex was established using Coot software, and then the structure correction was performed using Phenix software, so as to obtain a high-quality structure model.

It can be seen that without the addition of ligands, the modified chemokine receptor CCR8 according to the present application has good stability. To further illustrate the superiority of the modified GPCR of the present application, as a contrast, unmodified wild type CCR8 proteins and modified CCR8 proteins fused with several other fragments commonly used in the field of GPCR in the third intracellular loop were constructed. The amino acid sequences of these proteins are as follows, wherein the sequence of the fusion fragment is marked with an underline:
**Wild type CCR8 (CCR8wt, SEQ ID NO. 75)** 
DYTLDLSVTTVTDYYYPDIFSSPCDAELIQTNGKLLLAVFYCLLFVFSLLGNSL
VILVLVVCKKLRSITDVYLLNLALSDLLFVFSFPFQTYYLLDQWVFGTVMCKVVSGF
YYIGFYSSMFFITLMSVDRYLAVVHAVYALKVRTIRMGTTLCLAVWLTAIMATIPLL
VFYQVASEDGVLQCYSFYNQQTLKWKIFTNFKMNILGLLIPFTIFMFCYIKILHQLKRC
QNHNKTKAIRLVLIVVIASLLFWVPFNVVLFLTSLHSMHILDGCSISQQLTYATHVTEII
 
SFTHCCVNPVIYAFVGEKFKKHLSEIFQKSCSQIFNYLGRQMPRESCEKSSSCQQHSSR

SSSVDYIL
**CCR8-T4L(T4 lysozyme, SEQ ID NO. 76)** 
DYTLDLSVTTVTDYYYPDIFSSPCDAELIQTNGKLLLAVFYCLLFVFSLLGNSL
VILVLVVCKKLRSITDVYLLNLALSDLLFVFSFPFQTYYLLDQWVFGTVMCKVVSGF
YYIGFYSSMFFITLMSVDRYLAVVHAVYALKVRTIRMGTTLCLAVWLTAIMATIPLL
VFYQVASEDGVLQCYSFYNQQTLKWKIFTNFKMNILGLLIPFTIFMFCYIKILHQLKRC
QGNIFEMLRIDEGLRLKIYKDTEGYYTIGIGHLLTKSPSLNAAKSELDKAIGRNTNGVI
TKDEAEKLFNQDVDAAVRGILRNAKLKPVYDSLDAVRRAALINMVFQMGETGVAG
FTNSLRMLQQKRWDEAAVNLAKSRWYNQTPNRAKRVITTFRTGTWDAYGNHNKTK
AIRLVLIVVIASLLFWVPFNVVLFLTSLHSMHILDGCSISQQLTYATHVTEIISFTHCCV
NPVIYAFVGEKFKKHLSEIFQKSCSQIFNYLGRQMPRESCEKSSSCQQHSSRSSSVDYI
L
**CCR8-Rubredoxin (SEQ ID NO. 77)** 
DYTLDLSVTTVTDYYYPDIFSSPCDAELIQTNGKLLLAVFYCLLFVFSLLGNSL
VILVLVVCKKLRSITDVYLLNLALSDLLFVFSFPFQTYYLLDQWVFGTVMCKVVSGF
YYIGFYSSMFFITLMSVDRYLAVVHAVYALKVRTIRMGTTLCLAVWLTAIMATIPLL
VFYQVASEDGVLQCYSFYNQQTLKWKIFTNFKMNILGLLIPFTIFMFCYIKILHQLKRC
QGMKKYTCTVCGYIYNPEDGDPDNGVNPGTDFKDIPDDWVCPLCGVGKDQFEEVEE
GNHNKTKAIRLVLIVVIASLLFWVPFNVVLFLTSLHSMHILDGCSISQQLTYATHVTEII
SFTHCCVNPVIYAFVGEKFKKHLSEIFQKSCSQIFNYLGRQMPRESCEKSSSCQQHSSR
SSSVDYIL
**CCR8-BRIL (Cyrochrom b562 RIL, SEQ ID NO. 78)** 
DYTLDLSVTTVTDYYYPDIFSSPCDAELIQTNGKLLLAVFYCLLFVFSLLGNSL
VILVLVVCKKLRSITDVYLLNLALSDLLFVFSFPFQTYYLLDQWVFGTVMCKVVSGF
YYIGFYSSMFFITLMSVDRYLAVVHAVYALKVRTIRMGTTLCLAVWLTAIMATIPLL
VFYQVASEDGVLQCYSFYNQQTLKWKIFTNFKMNILGLLIPFTIFMFCYIKILHQLKRC
QGADLEDNWETLNDNLKVIEKADNAAQVKDALTKMRAAALDAQKATPPKLEDKSP
DSPEMKDFRHGFDILVGQIDDALKLANEGKVKEAQAAAEQLKTTRNAYIQKYLGNH
NKTKAIRLVLIVVIASLLFWVPFNVVLFLTSLHSMHILDGCSISQQLTYATHVTEIISFT
HCCVNPVIYAFVGEKFKKHLSEIFQKSCSQIFNYLGRQMPRESCEKSSSCQQHSSRSSS
VDYIL
**CCR8-Flavodoxin (SEQ ID NO. 79)** 
DYTLDLSVTTVTDYYYPDIFSSPCDAELIQTNGKLLLAVFYCLLFVFSLLGNSL
VILVLVVCKKLRSITDVYLLNLALSDLLFVFSFPFQTYYLLDQWVFGTVMCKVVSGF
YYIGFYSSMFFITLMSVDRYLAVVHAVYALKVRTIRMGTTLCLAVWLTAIMATIPLL
VFYQVASEDGVLQCYSFYNQQTLKWKIFTNFKMNILGLLIPFTIFMFCYIKILHQLKRC
QGAKALIVYGSTTGNTEYTAETIARELADAGYEVDSRDAASVEAGGLFEGFDLVLLG
CSTWGDDSIELQDDFIPLFDSLEETGAQGRKVACFGCGDSSWEYFCGAVDAIEEKLK
NLGAEIVQDGLRIDGDPRAARDDIVGWAHDVRGAIGNHNKTKAIRLVLIVVIASLLF
WVPFNVVLFLTSLHSMHILDGCSISQQLTYATHVTEIISFTHCCVNPVIYAFVGEKFKK
HLSEIFQKSCSQIFNYLGRQMPRESCEKSSSCQQHSSRSSSVDYIL
**CCR8-PGS (Pyrococcus glycogen synthase, SEQ ID NO. 80)**

DYTLDLSVTTVTDYYYPDIFSSPCDAELIQTNGKLLLAVFYCLLFVFSLLGNSL
VILVLVVCKKLRSITDVYLLNLALSDLLFVFSFPFQTYYLLDQWVFGTVMCKVVSGF
YYIGFYSSMFFITLMSVDRYLAVVHAVYALKVRTIRMGTTLCLAVWLTAIMATIPLL
VFYQVASEDGVLQCYSFYNQQTLKWKIFTNFKMNILGLLIPFTIFMFCYIKILHQLKRC

QGGIDCSFWNESYLTGSRDERKKSLLSKFGMDEGVTFMFIGRFDRGQKGVDVLLKAI
EILSSKKEFQEMRFIIIGKGDPELEGWARSLEEKHGNVKVITEMLSREFVRELYGSVDF
VIIPSYFEPFGLVALEAMCLGAIPIASAVGGLRDIITNETGILVKAGDPGELANAILKAL
ELSRSDLSKFRENCKKRAMSFSGNHNKTKAIRLVLIVVIASLLFWVPFNVVLFLTSLHS
MHILDGCSISQQLTYATHVTEIISFTHCCVNPVIYAFVGEKFKKHLSEIFQKSCSQIFNY
LGRQMPRESCEKSSSCQQHSSRSSSVDYIL

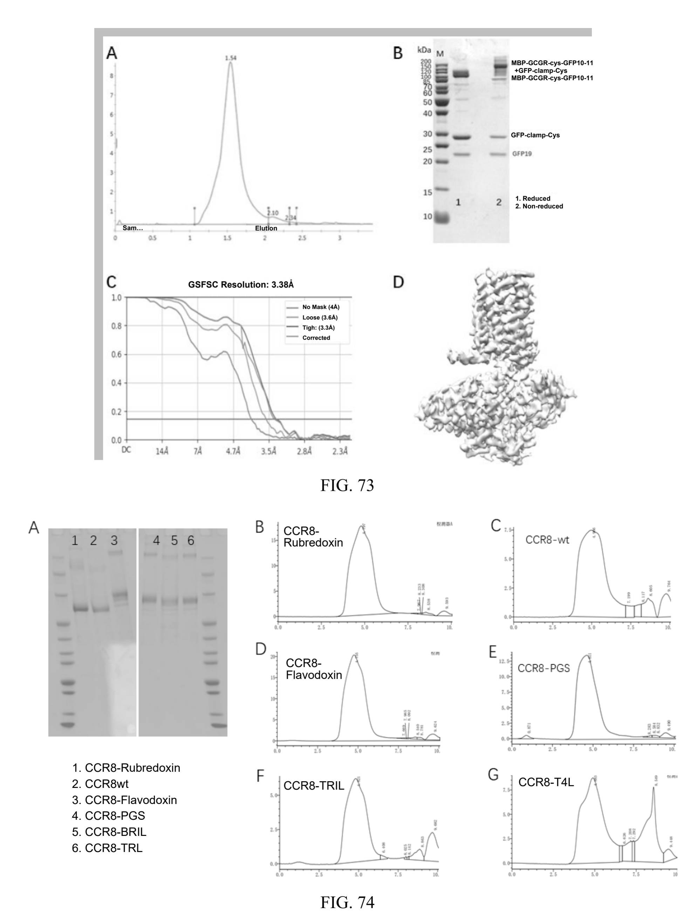

These proteins have MBP tags at the N-terminus and His tags at the C-terminus. The expression and purification methods of these proteins are the same as those in Example 4, except that no GFP-clamp is added during the purification. The results show (see FIG. 74) that the obtained protein exhibits a wide peak on a molecular sieve chromatography column, indicating that these natural or modified CCR8 proteins aggregate due to their failure to form native conformations, and therefore cannot be stably present in the ligand-free state.

### Example 8: Structural analysis of (GPCR C family) orphan receptor GPRC5D

The sequence of the orphan receptor GPRC5D was derived from an amino acid sequence with the GenBank accession number NM_018654.2. The first amino acid in the sequence was removed, a GFP10-11 fragment (marked with an underline) was fused into the third intracellular loop, and a linker was marked with a wavy line. The amino acid at the position 121 in the second intracellular loop was cysteine (marked with a border) and was not mutated. The amino acid sequence of the modified fusion protein is SEQ ID NO.9:

YKDCIESTGDYFLLCDAEGPWGIILESLAILGIVVTILLLLAFLFLMRKIQDCSQ
WNVLPTQLLFLLSVLGLFGLAFAFIIELNQQTAPVRYFLFGVLFALCFSCLLAHASNLV
KLVRGCVSFSWTTILCIAIGCSLLQIIIATEYVTLIMTRGMMFVNMTPCQLNVDFVVLL
VYVLFLMALTFFVSKATFCGPRRQLGHYLSTQTSLSKDLNEKRDHMVLLEFVTAAGL
QKEKQHGRLIFITVLFSIIIWVVWISMLLRGNPQFQRQPQWDDPVVCIALVTNAWVFL

LLYIVPELCILYRSCRQECPLQGNACPVTAYQHSFQVENQELSRARDSDGAEEDVALT

SYGTPIQPQTVDPTQECFIPQAKLSPQQDAGGV

The purification and identification method of the modified fusion protein is seen in example 4. The molecular sieve chromatography result graph of the modified fusion protein is seen in A of FIG. 14. The protein sample at the second ultraviolet absorption peak directed by an arrow was collected. The SDS gel electrophoresis identification result graph is seen in B of FIG. 14. The molecular sieve chromatography and SDS gel electrophoresis demonstrated that the protein possessed favorable properties and high purity, with each component contained consistent with expectations; and the purified protein formed a non-aggregated UV absorption peak in molecular sieve chromatography, thereby proving good stability.

The CCR8 protein complex purified via molecular sieve chromatography was concentrated using a centrifugal filter (with a molecular cut off of 10 kDa) until the concentration was 6 mg/ml. The specific method for structural analysis refers to Example 4. The high-quality 3D density (see D of FIG. 14) was obtained through homogenous refinement and non-uniform refinement (see C of FIG. 14). The 3D density was obtained from cryogenic electron microscopy data, the structural model of the protein complex was established using Coot software, and then the structure correction was performed using Phenix software, so as to obtain a high-quality structure model.

### Example 9: Structural analysis of (GPCR B1 family) glucagon receptor GCGR

The sequence of the glucagon receptor GCGR was derived from an amino acid sequence with the GenBank accession number NM_000160.5. A signal peptide at the N-terminus was removed, a GFP10-11 fragment (marked with an underline) was fused into the third intracellular loop, and a linker was marked with a wavy line. The T234 (Ballesteros-Weinstein numbering system 34.51) in the second intracellular loop was mutated to cysteine (marked with a border). The amino acid sequence of the modified fusion protein is SEQ ID NO.10:

AQVMDFLFEKWKLYGDQCHHNLSLLPPPTELVCNRTFDKYSCWPDTPANTTA
NISCPWYLPWHHKVQHRFVFKRCGPDGQWVRGPRGQPWRDASQCQMDGEEIEVQK
EVAKMYSSFQVMYTVGYSLSLGALLLALAILGGLSKLHCTRNAIHANLFASFVLKAS
SVLVIDGLLRTRYSQKIGDDLSVSTWLSDGAVAGCRVAAVFMQYGIVANYCWLLVE
GLYLHNLLGLACLPERSFFSLYLGIGWGAPMLFVVPWAVVKCLFENVQCWTSNDN
MGFWWILRFPVFLAILINFFIFVRIVQLLRRQLGHYLSTQTSLSKDLNEKRDHMVLLEF
VTAAGLQKEDYKFRLAKSTLTLIPLLGVHEVVFAFVTDEHAQGTLRSAKLFFDLFLSS
FQGLLVAVLYCFLNKEVQSELRRRWHRWRLGKVLWEERNTSNHRASSSPGHGPPSK
ELQFGRGGGSQDSSAETPLAGGLPRLAESPF

The purification and identification method of the modified fusion protein is seen in example 4. The molecular sieve chromatography result graph of the modified fusion protein is seen in A of FIG. 73, and the SDS gel electrophoresis result graph is seen in B of FIG. 73. The molecular sieve chromatography and SDS gel electrophoresis demonstrated that the protein possessed favorable properties and high purity, with each component contained consistent with expectations; the purified protein formed a non-aggregated UV absorption peak in molecular sieve chromatography, thereby proving good stability.

The GCGR protein complex purified via molecular sieve chromatography was concentrated using a centrifugal filter (with a molecular cut off of 10 kDa) to 8 mg/ml. The specific method for structural analysis refers to example 4. The high-quality 3D density (see D of FIG. 73) was obtained through homogenous refinement and non-uniform refinement (see C of FIG. 73).The 3D density was obtained from cryogenic electron microscopy data, the structural model of the protein complex was established using Coot software, and then the structure correction was performed using Phenix software, so as to obtain a high-quality structure model.

### Example 10: Other forms of modified GPCRs according to the present application

The sequence of CRFR was derived from an amino acid sequence with the GenBank accession number NP_001138618. A signal peptide sequence at the N-terminus was removed, and a GFP10-11 fragment (marked with an underline) was fused into the first intracellular loop. The amino acid sequence of the modified fusion protein is SEQ ID NO.11:

SLQDQHCESLSLASNISGLQCNASVDLIGTCWPRSPAGQLVVRPCPAFFYGVR
YNTTNNGYRECLANGSWAARVNYSECQEILNEEKKSKVHYHVAVIINYLGHCISLVA
LLVAFVLFLRLRGGGHYLSTQTSLSKDLNEKRDHMVLLEFVTAAGGGSLRNIIHWNL
ISAFILRNATWFVVQLTMSPEVHQSNVGWCRLVTAAYNYFHVTNFFWMFGEGCYLH
TAIVLTYSTDRLRKWMFICIGWGVPFPIIVAWAIGKLYYDNEKCWFGKRPGVYTDYI
YQGPMILVLLINFIFLFNIVRILMTKLRASTTSETIQYRKAVKATLVLLPLLGITYMLFF
VNPGEDEVSRVVFIYFNSFLESFQGFFVSVFYCFLNSEVRSAIRKRWHRWQDKHSIRA
RVARAMSIPTSPTRVSFHSIKQSTAV

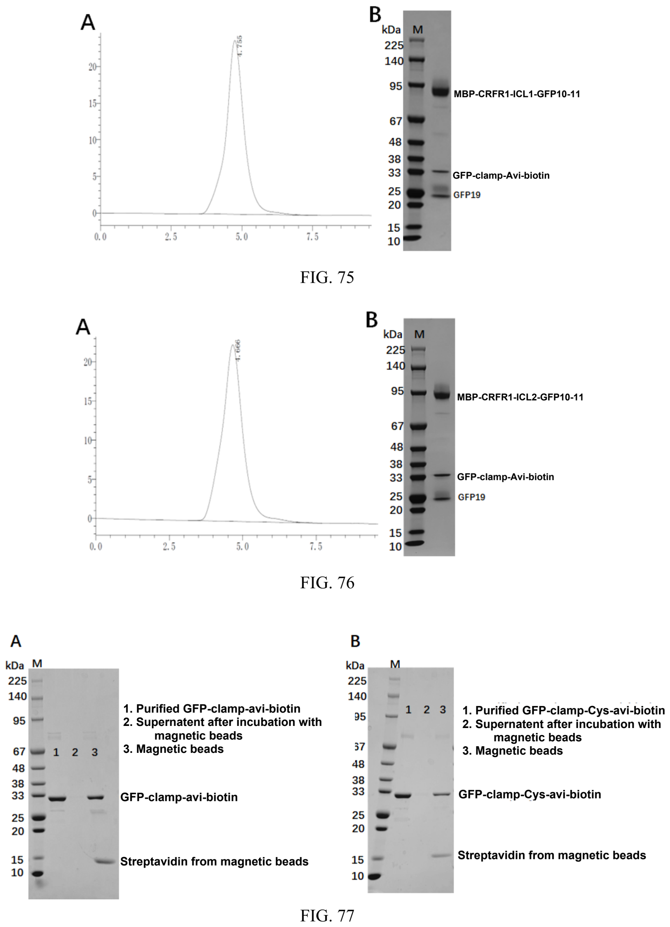

The purification and identification method of the modified fusion protein is seen in Example 4. The molecular sieve chromatography result graph of the modified fusion protein is seen in A of FIG. 75, and the SDS gel electrophoresis result graph of the modified fusion protein is seen in B of FIG. 75. The molecular sieve chromatography and SDS gel electrophoresis demonstrated that the protein possessed favorable properties and high purity, with each component contained consistent with expectations; and the purified protein formed a non-aggregated UV absorption peak in molecular sieve chromatography, thereby proving good stability.

A signal peptide sequence at the N-terminus of the CRFR receptor was removed, and a GFP10-11 fragment (marked with an underline) was fused into the second intracellular loop. The amino acid sequence of the modified fusion protein is SEQ ID NO.12:

SLQDQHCESLSLASNISGLQCNASVDLIGTCWPRSPAGQLVVRPCPAFFYGVR
YNTTNNGYRECLANGSWAARVNYSECQEILNEEKKSKVHYHVAVIINYLGHCISLVA
LLVAFVLFLRLRPGCTHWGDQADGALEVGAPWSGAPFQVRRSIRCLRNIIHWNLISAF
ILRNATWFVVQLTMSPEVHQSNVGWCRLVTAAYNYFHVTNFFWMFGEGCYLHTAI
VLTGGGHYLSTQTSLSKDLNEKRDHMVLLEFVTAAGGGDRLRKWMFICIGWGVPFPI
IVAWAIGKLYYDNEKCWFGKRPGVYTDYIYQGPMILVLLINFIFLFNIVRILMTKLRAS
TTSETIQYRKAVKATLVLLPLLGITYMLFFVNPGEDEVSRVVFIYFNSFLESFQGFFVS
VFYCFLNSEVRSAIRKRWHRWQDKHSIRARVARAMSIPTSPTRVSFHSIKQSTAV

The purification and identification method of the modified fusion protein is seen in Example 4. The molecular sieve chromatography result graph of the modified fusion protein is seen in A of FIG. 76, and the SDS gel electrophoresis result graph of the modified fusion protein is seen in B of FIG. 76. The molecular sieve chromatography and SDS gel electrophoresis demonstrated that the protein possessed favorable properties and high purity, with each component contained consistent with expectations; and the purified protein formed a non-aggregated UV absorption peak in molecular sieve chromatography, thereby proving good stability.

The results showed that the fusion of the GFP10-11 fragment was performed in the first intracellular loop or second intracellular loop of GPCR, thereby forming a stable complex with GFP1-9 and GFP-clamp proteins.

### Example 11: Preparation of biotinylated GFP-clamp

By taking advantage of the property that the modified GPCR-GFP10-11 fusion protein forms a stable complex with GFP1-9 and GFP-clamp, an Avi tag was added to the GFP-clamp, which was then co-expressed with biotin ligase BirA to complete the biotinylation of GFP-clamp-Avi in the cells. The purified GFP-clamp-avi-biotin was used for purification of GPCR-GFP10-11, the obtained complex had a biotin tag which was used for screening of a compound drug and an antibody drug, or affinity determination was performed by utilizing a biological and physical technology.

The amino acid sequence of GFP-clamp-avi is as follows (SEQ ID NO.74), and the Avi tag (marked with a wavy line) is at the C-terminus:

DLGKKLLEAARAGQDDEVRILMANGADVNAADDVGVTPLHLAAQRGHLEIV
EVLLKYGADVNAADLWGQTPLHLAATAGHLEIVEVLLKNGADVNARDNIGHTPLHL
AAWAGHLEIVEVLLKYGADVNAQDKFGKTPFDLAIDNGNEDIAEVLQKAAGGGSGG
GDVNAYDEVGWTPLHKAAWGHLEKVEDLLKNGADVNAADIDGYTPLHLAAFSGHL
EIVEVLLKYGADVNADDQAGFTPLHLAAIFGHLEIVEVLLKNGADVNAQDKFGKTPF
DLAIDNGNEDIAEVLQKAASHHHHHHHHSGGGLNDIFEAQKIEWHE

GFP-clamp-avi and a biotin ligase BirA were co-expressed in Rosetta^{™} 2 (DE3) competent cells and purified using the same method as that in Example 1 (1) to obtain biotinylated GFP-clamp-avi-biotin. By the experiment for binding to streptavidin magnetic beads, it is confirmed that the protein has an extremely high degree of biotinylation, and almost all the proteins are bound to the streptavidin magnetic beads. The SDS gel electrophoresis result of the GFP-clamp-avi-biotin protein is seen in FIG. 77A. The SDS gel electrophoresis shows that the protein has good property and high purity, and each component contained is consistent with expected component; the purified protein forms a non-aggregation state in molecular sieve chromatography, thereby proving good stability.

The glutamic acid at the position 186 of GFP-clamp-Avi was mutated to cysteine (marked with a border), other parts comprise a maltose binding protein and a TEV protease digestion site at the N-terminus, and the histidine tag at the C-terminus was kept unchanged. The amino acid sequence of GFP-clamp-Cys-Avi is SEQ ID NO.82:

DLGKKLLEAARAGQDDEVRILMANGADVNAADDVGVTPLHLAAQRGHLEIV
EVLLKYGADVNAADLWGQTPLHLAATAGHLEIVEVLLKNGADVNARDNIGHTPLHL
AAWAGHLEIVEVLLKYGADVNAQDKFGKTPFDLAIDNGNEDIAEVLQKAAGGGSGG
GDVNAYDEVGWTPLHKAAWGHLCKVEDLLKNGADVNAADIDGYTPLHLAAFSGH
LEIVEVLLKYGADVNADDQAGFTPLHLAAIFGHLEIVEVLLKNGADVNAQDKFGKTP
FDLAIDNGNEDIAEVLQKAASHHHHHHHHSGGGLNDIFEAQKIEWHE

GFP-clamp-avi and a biotin ligase BirA were co-expressed in Rosetta^{™} 2 (DE3) competent cells and purified using the same method as that in Example 3 to obtain biotinylated GFP-clamp-avi-biotin. By the experiment for binding to streptavidin magnetic beads, it is confirmed that the protein has an extremely high degree of biotinylation, and almost all the proteins are bound to the streptavidin magnetic beads. The SDS gel electrophoresis result of the GFP-clamp-Cys-avi-biotin protein is seen in FIG. 77B. The SDS gel electrophoresis shows that the protein has good property and high purity, and each component contained is consistent with expected component; the purified protein forms a non-aggregation state in molecular sieve chromatography, thereby proving good stability.

### Example 12: Binding feature of modified MC4R according to the present application and its ligand

The GFP10-11 fragment (marked with an underline) was fused into the third intracellular loop of the melanocortin receptor 4 (MC4R), and the linker was marked with a wavy line. The modified sequence is SEQ ID NO.13:

GRAWNRSSYRLHSNASESLGKGYSDGGCYEQLFVSPVVFVTLGVISLLENILVI
VAIAKNKNLHSPMYFFICSLAVADMLVSVSLGFETIVITLLNSTDTDAQSFTVNIDNVI
DSVICASLLASICSLLSIAVDRYFTIFYALQYHNIMTVKRVGIIISCIWAACTVSGILFIIY
SDSSAVIICLITMFFTMLALMASLYVHMFLMARRQLGHYLSTQTSLSKDLNEKRDHM
VLLEFVTAAGLQKEMKGAITLTILIGVFVVCWAPFFLHLIFYISCPQNPYCVCFMSHFN
LYLILIMCNSIINPLIYALRSQELRKTFKEIICCY

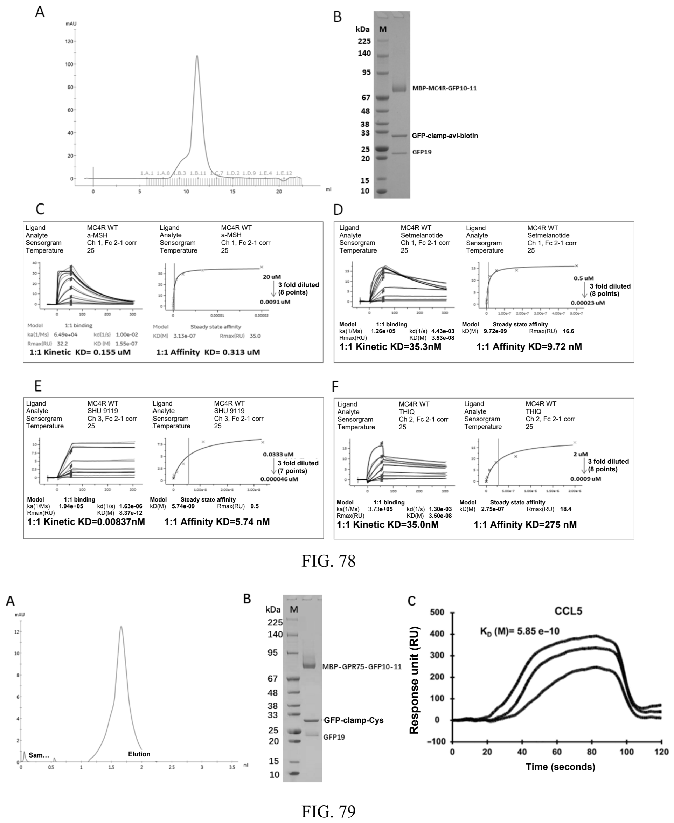

The purification and identification method of the modified MC4R fusion protein is seen in Example 4. The difference is that during the purification, GFP-clamp-avi-biotin is added, rather than GFP-clamp. The molecular sieve chromatography result graph of the modified fusion protein is seen in A of FIG. 78, and the SDS gel electrophoresis result graph of the modified fusion protein is seen in B of FIG. 78. The molecular sieve chromatography and SDS gel electrophoresis demonstrated that the protein possessed favorable properties and high purity, with each component contained consistent with expectations; and the purified protein formed a non-aggregated UV absorption peak in molecular sieve chromatography, thereby proving good stability.

The purified MC4R was used for determination of affinity to 4 known ligands via surface plasmon resonance (SPR). The used instrument was Biacore 8K, and the chip was an SPR sensor chip SAD200M. Both a protein immobilization buffer and a running buffer contained 20 mM HEPES, 150 mM NaCl, 2 mM CaCl₂, 1 mM MgCl₂, 1 mM EDTA, 0.005% LMNG and 0.0005% CHS, with a pH of 7.4. The affinity determination results are respectively seen in C-F of FIG. 78.

The results show that the modified MC4R protein according to the present application has stable property, the determination results of affinity to several known compounds are close to reported results, indicating that the ligand binding site of the modified GPCR has the same structure as that of a natural protein, and therefore the modified GPCR according to the present application can be used for screening of a compound library.

### Example 13: Binding feature of modified GPR75 and its ligand

The GFP10-11 fragment (marked with an underline) was fused into the third intracellular loop of the obesity-related receptor GPR75 (1-395). The modified sequence is SEQ ID NO.14:

MNSTGHLQDAPNATSLHVPHSQEGNSTSLQEGLQDLIHTATLVTCTFLLAVIF
CLGSYGNFIVFLSFFDPAFRKFRTNFDFMILNLSFCDLFICGVTAPMFTFVLFFSSASSIP
DAFCFTFHLTSSGFIIMSLKTVAVIALHRLRMVLGKQPNRTASFPCTVLLTLLLWATSF
TLATLATLKTSKSHLCLPMSSLIAGKGKAILSLYVVDFTFCVAVVSVSYIMIAQTLRK
NAGHYLSTQTSLSKDLNEKRDHMVLLEFVTAAGTAKDSKAVVTCVIIVLSVLVCCLP
LGISLVQVVLSSNGSFILYQFELFGFTLIFFKSGLNPFIYSRNSAGLRRKVLWCLQYIG

The purification and identification method of the modified GPR75 fusion protein refers to Example 4. The difference is that during the purification, GFP-clamp-avi-biotin was added rather than GFP-clamp. The molecular sieve chromatography result graph of the modified fusion protein is seen in A of FIG. 79, and the SDS gel electrophoresis result graph of the modified fusion protein is seen in B of FIG. 79. The molecular sieve chromatography and SDS gel electrophoresis demonstrated that the protein possessed favorable properties and high purity, with each component contained consistent with expectations; and the purified protein formed a non-aggregated UV absorption peak in molecular sieve chromatography, thereby proving good stability.

The purified GPR75 was used for determination of affinity to CCL5 ligand via surface plasmon resonance (SPR). The used instrument was Biacore 8K, and the chip was Series S Sensor SA Chip:SA-2429. Both a protein immobilization buffer and a running buffer contained 20 mM HEPES, 150 mM NaCl, 0.005% LMNG and 0.0005% CHS, pH 7.4. The affinity determination results are seen in C of FIG. 79.

The results showed that the modified GPR75 protein according to the present application has stable property, and the determination results of affinity to CCL5 are close to reported results, indicating that the ligand binding site of the modified GPCR has the same structure as that of the natural protein, and therefore the modified GPR75 protein according to the present application can be used for screening of a compound library.

### Example 14: Use of modified A2A adenosine receptor and cannabinoid type I receptor (CNR1) according to the present application for antibody discovery

The sequences of the A2A receptor and the CNR1 receptor after modification are the same as those in Example 1 and Example 2, respectively. The two modified receptors were co-expressed with GFP1-9 respectively and purified using the same method as that in Example 1 after GFP-clamp-avi-biotin was added to obtain a complex with a biotin tag.

The antibody titer ELISA detection of the sera of BALB/C mice immunized by the virus like particles (VLP) of the A2A receptor and the CNR1 receptor: streptavidin (0.1 M carbonate buffer, pH 9.6, 2.5 µg/well) was added in a 96-well microplate to be coated at 4°C overnight, 350 µL of 40 mg/ml bovine serum albumin BSA was added in each well for blockage, and the wells were washed once using a PBSL solution (10 mM PBS, pH7.4, 0.01% LMNG, 0.001% CHS); 1 µg of purified GPCR protein complex with biotin was added in each well and incubated for 1 h at room temperature, and then the wells were washed once using the PBSL solution; each well was blocked with PBSL containing 40 mg/ml bovine serum albumin BSA; the serums of mice were serially diluted using PBS, and then added to each well for 1 h of incubation, the wells were washed four times using the PBSL solution; a horseradish peroxidase-conjugated goat anti-mouse antibody was added and the above mixture was incubated for 1 h at room temperature, the wells were washed four times using the PBSL solution; each well was added with 100 µL of TMB substrate and then the above mixture was incubated for 10-20 min and then developed, 50 µL of terminating solution was added to each well after the color development was stabilized and basically unchanged, and then the absorbance of each well at 450 nm was read on the microplate. The detection results of A2A and CNR1 immune mice serum titer are respectively seen in A and B of FIG. 80.

The BALB/C mice immunized using virus like particles (VLP) of target protein A2A and CNR1 were executed, soaked in a 75% ethanol solution for 5 min and then taken out, the abdomens of the mice were wiped, limbs were fixed, the skins, muscles and peritoneums were scissored to take out spleens, and then the spleens were stored in PBS containing penicillin-streptomycin. The spleens of mice were washed in a serum-free RPMI-1640 culture medium, then placed in a 70 µm cell sieve mesh and ground using a grinding rod, wherein during the grinding, the RPMI-1640 culture medium was continuously added. The splenic cells passing through the sieve mesh were collected using a centrifugal tube, the collected splenic cells were centrifuged for 10 min at 300 g, supernatant was discarded, the cells were resuspended using the RPMI-1640 culture medium and washed twice, and finally the cells were counted. Meanwhile, mouse myeloma SP2/0 cells being in good growth state were resuspended and collected from the culture dish, the collected cells were centrifuged at 300 g for 5 min, and then the centrifuged cells were resuspended using a serum-free RPMI-1640 culture medium and counted.

The resuspended 1-2x10^8 mouse splenic cells were mixed with SP2/0 cells in a ratio of 10:1, then the mixed cells were centrifuged at 300 g for 10 min, and supernatant was discarded. The centrifugal tube was placed in a water bath at 37°C, 1 ml of 50% PEG 1450 solution preheated to 37°C was sucked using a pipette, the suction tip of the pipette was stretched into cell pellets to slowly inject the PEG solution, and meanwhile the cells were sufficiently mixed with the PEG solution under the gentle stirring, wherein the addition of the PEG solution needed to be completed within 1 min, and the gentle stirring was continued for 1 min. Subsequently, 1 ml of serum-free RPMI-1640 culture medium was sucked and slowly added to the cell and PEG mixed solution in the same way and gently stirred. 10 ml of serum-free RPMI-1640 culture medium continued to be slowly added while stirring. The cell pellets were collected after centrifuging for 10 min at 300 g, the cells were resuspended with 1 ml of 10% FBS-containing RPMI-1640 culture medium, and then supernatant was discarded. The cells were resuspended in a 120 ml HAT semi-solid selective culture medium (RPMI-1640 culture medium containing 10% FBS, 1xHAT selective reagent, 1x hybridoma feeder addition factors, 1× penicillin/streptomycin, 1.68% methylcellulose) and evenly mixed, then the mixture was poured into 8 100 mm cell culture dishes, underwent standing incubation in an incubator containing 5% carbon dioxide at 37°C for 10-14 days, and subsequently the successfully fused hybridoma cells grew into visible cell colonies to the naked eye.

200 µl of RPMI-1640 culture medium (containing 10% FBS, 0.5x hybridoma feeder addition factor and 1x penicillin/streptomycin) was added to each well of a 96-well cell culture medium. Single cell colonies in the semi-solid culture medium were sequentially transferred into each well of the 96-well plate. After the colonies grew to cover most of the well bottom, the hybridoma cells secreted antibodies into the supernatant of the culture medium during the growth.

ELISA detection was performed on target-specific antibodies in each well according to the same steps as those in the serum titer detection as described above.

Streptavidin (0.1M carbonate buffer, pH 9.6, 2.5 µg/well) was added to the 96-well microplate for coating overnight4°C, 350 µL of 40 mg/ml bovine serum albumin BSA was added to each well for blockage, and the wells were washed once with a PBSL solution (10 mM PBS, pH7.4, 0.01% LMNG, 0.001% CHS); each well was added with 1 µg of purified GPCR protein complex with a biotin tag for 1 h of incubation at room temperature, and then the wells were washed once with the PBSL solution; each well was blocked using PBSL containing 40 mg/ml bovine serum albumin BSA; hydridoma cell supernatant incubated in each well of the 96-well cell culture plate was transferred to a corresponding well of the 96-well microplate to be incubated for 1 h at room temperature, a horseradish peroxidase-conjugated goat anti-mouse antibody was added for 1 h of incubation at room temperature, and the wells were washed four times with the PBSL solution; each well was added with 100 µ L of TMB substrate and then the above mixture was incubated for 10-20 min in the dark at room temperature for developing, 50 µ L of terminating solution was added to each well after the developing was stable and basically unchanged, and then the absorbance of each well at 450 nm. The ELISA detection results of the supernatant in one anti-A2A hydridoma 96-well plate and the supernatant in one anti-CNR1 hydridoma 96-well plate are seen in C and D of FIG. 80, respectively.

The results show that the modified GPCR of the present application can be effectively used for antibody discovery. The difficulty for therapeutic GPCR antibody discovery through a mouse immunization and hybridoma technology is that the generated and identified antibody can recognize GPCR with native conformation on the surfaces of human cells. First, there is a need to use GPCR with native conformation to immunize the mouse, the immune system of the mouse is stimulated to generate the antibody capable of recognizing GPCR with native conformation, and then hybridoma cells are prepared and identified using GPCR with native conformation. Given that a large proportion of antibodies can bind to the target protein but are not suitable for use as therapeutic antibodies due to affinity, stability and other factors, it is essential to obtain as many hybridoma cell strains secreting specific antibodies as possible in the step of hybridoma preparation and screening. For multi-transmembrane proteins such as GPCR, the extracellular domain is relatively small, and the proportion of B cells producing GPCR protein antibodies in splenocytes is usually very low (as indicated by the proportion of positive cells in FIG. 80). Therefore, it is often necessary to generate a large number (at least several thousand) of hybridoma cells for subsequent screening therefrom. ELISA has the characteristics of high sensitivity and high throughout, and therefore is suitable for rapidly identifying cells secreting antibodies recognizing GPCR with native conformation, however, high-purity proteins are needed to identify specific antibodies to avoid the screening of other hybridoma cells secreting non-specific antibodies. A relatively large amount of purified proteins are also required for the identification of a large number of hybridoma cells. According to the present disclosure, the high-purity GPCR protein can be conveniently obtained, which is used for excluding the interference of other background proteins via ELISA, rapidly screening hybridoma cells secreting a specific antibody against target GPCR (as shown in FIG. 80). Especially, the GPCR protein with a natural structure at the extracellular side is obtained in the present disclosure, and the antibody recognizing the GPCR natural structure is screened, which is crucial for therapeutic antibody.

### Example 15: Other modified G protein coupled receptors according to the present application

The amino acid sequence of the following GPCR was modified using the same method as that of CNR1 and NK1R as described above: 1, at least a part of the third intracellular loops of these GPCRs were replaced with the GFP10-11 fragment (SEQ ID NO. 1) with linkers at two sides; and 2, the amino acid of the Ballesteros-Weinstein numbering system 34.51 in the second intracellular loop was mutated to cysteine. The amino acid sequence of the modified GPCR is as follows:
GPR52-Cys-GFP10-11 (SEQ ID NO.15): 
GIVNVSERHSCPLGFGHYSVVDVCIFETVVIVLLTFLIIAGNLTVIFVFHCAPLLHH
YTTSYFIQTMAYADLFVGVSCLVPTLSLLHYSTGVHESLTCQVFGYIISVLKSVSMWC
LACISVDRYLAITKPCSYNQLVTPCRLRICIILIWIYSCLIFLPSFFGWGKPGYHGDIFEW
CATSWLTSAYFTGFIVCLLYAPAAFVVCFTYFHIFKICRRQLGHYLSTQTSLSKDLNEK
RDHMVLLEFVTAAGLQKERRYLMVLFRITSVFYMLWLPYIIYFLLESSRVLDNPTLSF
LTTWLAISNSFCNPVIYALSDSTFRLGLRRLSETMCTSCMC
GNRHR-Cys-GFP10-11 (SEQ ID NO.16): 
MANSASPEQNQNHCSAINNSIPLMQGNLPTLTLSGKIRVTVTFFLFLLSATFNASFLLK

LQKWTQKKEKGKKLSRMKLLLKHLTLANLLETLIVMPLDGMWNITVQWYAGELLC

KVLSYLKLFSMYAKAFMMVVISLDRSLAITRPCALKSNSKVGQSMVGLAWILSSVFA

GPQLYIFRMIHLADSSGQTKVFSQCVTHCSFSQWWHQAFYNFFTFSCLFIIPLFIMLICN

AKIIFTLRRQLGHYLSTQTSLSKDLNEKRDHMVLLEFVTAAGLQKERLKTLKMTVAF

ATSFTVCWTPYYVLGIWYWFDPEMLNRLSDPVNHFFFLFAFLNPCFDPLIYGYFSL
PTGDR2-Cys-GFP10-11 (SEQ ID NO.17): 
MSANATLKPLCPILEQMSRLQSHSNTSIRYIDHAAVLLHGLASLLGLVENGVILF
VVGCRMRQTVVTTWVLHLALSDLLASASLPFFTYFLAVGHSWELGTTFCKLHSSIFF
LNMFASGFLLSAISLDRCLQVVRPCWAQNHRTVAAAHKVCLVLWALAVLNTVPYF
VFRDTISRLDGRIMCYYNVLLLNPGPDRDATCNSRQVALAVSKFLLAFLVPLAIIASSH
AAVSLRLQHRGGHYLSTQTSLSKDLNEKRDHMVLLEFVTAAGRRRPGRFVRLVAAV
VAAFALCWGPYHVFSLLEARAHANPGLRPLVWRGLPFVTSLAFFNSVANPVLYVLT
CPDMLRKLRRSLRTVLESVLVDDSELGGAGSS
HTR2C-Cys-GFP10-11 (SEQ ID NO.18): GAPTSDGGRFKFPDGVQNWPALSIVIIIIMTIGGNILVIMAVSMEKKLHNATNYFL 
MSLAIADMLVGLLVMPLSLLAILYDYVWPLPRYLCPVWISLDVLFSTASIMHLCAISL
DRYVAIRNPCEHSRFNSRTKAIMKIAIVWAISIGVSVPIPVIGLRDEEKVFVNNTTCVL
NDPNFVLIGSFVAFFIPLTIMVITYCLTIYVLRRQLGHYLSTQTSLSKDLNEKRDHMVL
LEFVTAAGLQKERKASKVLGIVFFVFLIMWCPFFITNILSVLCEKSCNQKLMEKLLNV
FVWIGYVNSGINPLVYTLFNKIYRRAFSNYLRCNYKVEKKP
ADRA2B-Cys-GFP10-11 (SEQ ID NO.19): 
SVQATAAIAAAITFLILFTIFGNALVILAVLTSRSLRAPQNLFLVSLAAADILVATLI
IPFSLANELLGYWYFRRTWCEVYLALDVLFCTSSIVHLCAISLDRYWAVSRACEYNS
KRTPRRIKCIILTVWLIAAVISLPPLIYKGDQGPQPRGRPQCKLNQEAWYILASSIGSFF
APCLIMILVYLRIYLIARRQLGHYLSTQTSLSKDLNEKRDHMVLLEFVTAAGLQKEKR
FTFVLAVVIGVFVLCWFPFFFSYSLGAICPKHCKVPHGLFQFFFWIGYCNSSLNPVIYTI
FNQDFRRAFRRILCRPWTQTAW
ADRB1-Cys-GFP10-11 (SEQ ID NO.20): 
ASESPEPLSQQWTAGMGLLMALIVLLIVAGNVLVIVAIAKTPRLQTLTNLFIMSL
ASADLVMGLLVVPFGATIVVWGRWEYGSFFCELWTSVDVLCVTASIETLCVIALDRY
LAITSPCRYQSLLTRARARGLVCTVWAISALVSFLPILMHWWRAESDEARRCYNDPK
CCDFVTNRAYAIASSVVSFYVPLCIMAFVYLRVFREARRQLGHYLSTQTSLSKDLNEK
RDHMVLLEFVTAAGLQKEQKALKTLGIIMGVFTLCWLPFFLANVVKAFHRELVPDRL
FVFFNWLGYANSAFNPIIYCRSPDFRKAFQGLLCCARRAAR
ADRB2-Cys-GFP10-11 (SEQ ID NO.21): 
AADEVWVVGMGIVMSLIVLAIVFGNVLVITAIAKFERLQTVTNYFITSLACADLV
MGLAVVPFGAAHILTKTWTFGNFWCEFWTSIDVLCVTASIETLCVIAVDRYFAITSPC
KYQSLLTKNKARVIILMVWIVSGLTSFLPIQMHWYRATHQEAINCYAEETCCDFFTN
QAYAIASSIVSFYVPLVIMVFVYSRVFQEARRQLGHYLSTQTSLSKDLNEKRDHMVLL
 
EFVTAAGLQKEHKALKTLGIIMGTFTLCWLPFFIVNIVHVIQDNLIRKEVYILLNWIGY

VNSGFNPLIYCRSPDFRIAFQELLCLRRSSLKAYGNGYSSNGNTGEQSG
C5AR1 -Cys-GFP10-11 (SEQ ID NO.22): 
SNTLRVPDILALVIFAVVFLVGVLGNALVVWVTAFEAKRTINAIWFLNLAVADF
LSCLALPILFTSIVQHHHWPFGGAACSILPSLILLNMYASILLLATISADRFLLVFKPCW
CQNFRGAGLAWIACAVAWGLALLLTIPSFLYRVVREEYFPPKVLCGVDYSHDKRRER
AVAIVRLVLGFLWPLLTLTICYTFILLRTRRQLGHYLSTQTSLSKDLNEKRDHMVLLE
FVTAAGLQKETKTLKVVVAVVASFFIFWLPYQVTGIMMSFLEPSSPTFLLLKKLDSLC
VSFAYINCCINPIIYVVAGQGFQGRLRKSLPSLLRNVLTEESVVR
CCR2-Cys-GFP10-11 (SEQ ID NO.23): 
GTLSTSRSRFIRNTNESGEEVTTFFDYDYGAPCHKFDVKQIGAQLLPPLYSLVFIF
GFVGNMLVVLILINCKKLKCLTDIYLLNLAISDLLFLITLPLWAHSAANEWVFGNAMC
KLFTGLYHIGYFGGIFFIILLTIDRYLAIVHACFALKARTVTFGVVTSVITWLVAVFAS
VPGIIFTKCQKEDSVYVCGPYFPRGWNNFHTIMRNILGLVLPLLIMVICYSGISRASRR
QLGHYLSTQTSLSKDLNEKRDHMVLLEFVTAAGLQKEKKAVRVIFTIMIVYFLFWTP
YNIVILLNTFQEFFGLSNCESTSQLDQATQVTETLGMTHCCINPIIYAFVGEKFRRYLS
VFFRKHITKRFGR
CCR5-Cys-GFP10-11 (SEQ ID NO.24): 
GAPDYQVSSPIYDINYYTSEPCQKINVKQIAARLLPPLYSLVFIFGFVGNMLVILILINY

KRLKSMTDIYLLNLAISDLFFLLTVPFWAHYAAAQWDFGNTMCQLLTGLYFIGFFSGI

FFIILLTIDRYLAVVHACFALKARTVTFGVVTSVITWVVAVFASLPNIIFTRSQKEGLH

YTCSSHFPYSQYQFWKNFQTLKIVILGLVLPLLVMVICYSGILKTLRRQLGHYLSTQTS

LSKDLNEKRDHMVLLEFVTAAGLQKEHRDVRLIFTIMIVYFLFWAPYNIVLLLNTFQE

FFGLNNCSSSNRLDQAMQVTETLGMTHCCINPIIYAFVGEEFRNYLLVFFQKHIAKRF
 CKCCSIFQQEAPERASSVYTRSTGEQEISVGLGRPLEVLFQ
CCR6-Cys-GFP10-11 (SEQ ID NO.25): 
SGESMNFSDVFDSSEDYFVSVNTSYYSVDSEMLLCSLQEVRQFSRLFVPIAYSLIC
VFGLLGNILVVITFAFYKKARSMTDVYLLNMAIADILFVLTLPFWAVSHATGAWVFS
NATCKLLKGIYAINFNCGMLLLTCISMDRYIAIVQACKSFRLRSRTLPRSKIICLVVWG
LSVIISSSTFVFNQKYNTQGSDVCEPKYQTVSEPIRWKLLMLGLELLFGFFIPLMFMIFC
YTFIVKTLRRQLGHYLSTQTSLSKDLNEKRDHMVLLEFVTAAGLQKEHKAIRVIIAVV
LVFLACQIPHNMVLLVTAANLGKMNRSCQSEKLIGYTKTVTEVLAFLHCCLNPVLYA
FIGQKFRNYFLKILKDLWCVRRKYKSSGFSCAGRYSENISRQTSETADNDNASSFTM
CCR7-Cys-GFP10-11 (SEQ ID NO.26): 
GPGSSLCSKKDVRNFKAWFLPIMYSIICFVGLLGNGLVVLTYIYFKRLKTMTDTY
LLNLAVADILFLLTLPFWAYSAAKSWVFGVHFCKLIFAIYKMSFFSGMWLLLCISIDR
YVAIVQACSAHRHRARVLLISKLSCVGIWILATVLSIPELLYSDLQRSSSEQAMRCSLI
TEHVEAFITIQVAQMVIGFLVPLLAMSFCYLVIISKLRRQLGHYLSTQTSLSKDLNEKR
DHMVLLEFVTAAGLQKENKAIKVIIAVVVVFIVFQLPYNGVVLAQTVANFNITSSTCE
LSKQLNIAYDVTYSLACVRCCVNPFLYAFIGVKFRNDLFKLFKDLGCLSGGRLEVLFQ
CHRM2-Cys-GFP10-11 (SEQ ID NO.27): 
GPMDDSTDSSDNSLALTSPYKTFEVVFIVLVAGSLSLVTIIGNILVMVSIKVNRHL
QTVNNYFLFSLACADLIIGVFSMNLYTLYTVIGYWPLGPVVCDLWLALDYVVSNARV
MNLLIISFDRYFCVTKPCTYPVKRTTKMAGMMIAAAWVLSFILWAPAILFWQFIVGV
RTVEDGECYIQFFSNAAVTFGTAIAAFYLPVIIMTVLYWHISRASRRQLGHYLSTQTSL
SKDLNEKRDHMVLLEFVTAAGLQKEKKVTRTILAILLAFIITWAPYNVMVLINTFCAP
CIPNTVWTIGYWLCYINSTINPACYALCNATFKKTFKHLLMCHYKNIGATR
CLTR2-Cys-GFP10-11 (SEQ ID NO.28): 
MEPNGTFSNNNSRNCTIENFKREFFPIVYLIIFFVGVLGNGLSIYVFLQPYKKSTSV
NVFMLNLAISNLLFISTLPFRADYYLRGSNWIFGDLACRIMSYSLYVNMYSSIYFLTVL
SVVRYLAMVHPCRLLHVTSIRSAWILCGIIWILIMASSIMLLDSGSEQNGSVTSCLELN
LYKIAKLQTMNYIALVVGCLLPFFTLSICYLLIIRVLRRQLGHYLSTQTSLSKDLNEKR
DHMVLLEFVTAAGLQKERKALTTIIITLIIFFLCFLPYHTLRTVHLTTWKVGLCKDRLH
KALVITLALAAANACFNPLLYYFAGENFKDRLKSALRK
CXCR2-Cys-GFP10-11 (SEQ ID NO.29): 
APCEPESLEINKYFVVIIYALVFLLSLLGNSLVMLVILYSRVGRSVTDVYLLNLAL
ADLLFALTLPIWAASKVNGWIFGTFLCKVVSLLKEVNFYSGIWLLACISVDRYLAIVH
ACRTLTQKRYLVKFICLSIWGLSLLLALPVLLFRRTVYSSNVSPACYEDMGNNTANW
RMLLRILPQSFGFIVPLLIMLFCYGFTLRTLRRQLGHYLSTQTSLSKDLNEKRDHMVLL
EFVTAAGLQKEHREMRVIFAVVLIFLLCWLPYNLVLLADTLMRTQVIQETCERRNHI
DRALDATEILAILHSCLNPLIYAFIGQKFRHGLLKILAIHGLISKDSLPKDS
CXCR4-Cys-GFP10-11 (SEQ ID NO.30): 
AGAPEGISIYTSDNYTEEMGSGDYDSMKEPCFREENANFNKIFLPTIYSIIFLTGIV
GNGLVILVMGYQKKLRSMTDKYRLHLSVADLLFVITLPFWAVDAVANWYFGNFLC
KAVHVIYTVNLYSSVWILAFISLDRYLAIVHACNSQRPRKLLAEKVVYVGVWIPALL
LTIPDFIFANVSEADDRYICDRFYPNDLWVVVFQFQHIMVGLILPGIVILSCYCIIISKLR
RQLGHYLSTQTSLSKDLNEKRDHMVLLEFVTAAGLQKERKALKTTVILILAFFACWL
PYYIGISIDSFILLEIIKQGCEFENTVHKWISITEALAFFHCCLNPILYAFLGAKFKTSAQ
HALTSVSRGSS
DRD2-Cys-GFP10-11 (SEQ ID NO.31): 
YNYYATLLTLLIAVIVFGNVLVCMAVSREKALQTTTNYLIVSLAVADLLVATLV
MPWVVYLEVVGEWKFSRIHCDIFVTLDVMMCTASALNLCAISIDRYTAVAMPCLYN
 
TRYSSKRRVTVMISIVWVLSFTISCPLLFGLNNADQNECIIANPAFVVYSSIVSFYVPFI
VTLLVYIKIYIVLRRQLGHYLSTQTSLSKDLNEKRDHMVLLEFVTAAGLQKEKKATQ
MAAIVAGVFIICWLPFFITHILNIHCDCNIPPVLYSAFTWLGYVNSAVNPIIYTTFNIEFR
KAFLKILHC
DRD3-Cys-GFP10-11 (SEQ ID NO.32): 
APASLSQLSSHLNYTCGAENSTGASQARPHAYYALSYCALILAIVFGNGLVCMA
VLKERALQTTTNYLVVSLAVADLLVATLVMPWVVYLEVTGGVWNFSRICCDVFVTL
DVMMCTASIWNLCAISIDRYTAVVMPCHYQHGTGQSSCRRVALMITAVWVLAFAVS
CPLLFGFNTTGDPTVCSISNPDFVIYSSVVSFYLPFGVTVLVYARIYVVLRRQLGHYLS
TQTSLSKDLNEKRDHMVLLEFVTAAGLQKEKKATQMVAIVLGAFIVCWLPFFLTHVL
NTHCQTCHVSPELYSATTWLGYVNSALNPVIYTTFNIEFRKAFLKILSC
GPBAR-Cys-GFP10-11 (SEQ ID NO.33): 
MTPNSTGEVPSPIPKGALGLSLALASLIITANLLLALGIAWDRRLRSPPAGCFFLSL
LLAGLLTGLALPTLPGLWNQSRRGYWSCLLVYLAPNFSFLSLLANLLLVHGERYMA
VLRPCQPPGSIRLALLLTWAGPLLFASLPALGWNHWTPGANCSSQAIFPAPYLYLEVY
GLLLPAVGAAAFLSVRVLATARRQLGHYLSTQTSLSKDLNEKRDHMVLLEFVTAAG
LQKEWRQARAQAGAMLLFGLCWGPYVATLLLSVLAYEQRPPLGPGTLLSLLSLGSA
SAAAVPVAMGLGDQRYTAPWRAAAQRCLQGLWGRASRDSPGPSIAYHPSSQSSVDL
DLN
HRH1-Cys-GFP10-11 (SEQ ID NO.34): 
TTMASPQLMPLVVVLSTICLVTVGLNLLVLYAVRSERKLHTVGNLYIVSLSVAD
LIVGAVVMPMNILYLLMSKWSLGRPLCLFWLSMDYVASTASIFSVFILCIDRYRSVQQ
PCRYLKYRTKTRASATILGAWFLSFLWVIPILGWNHFMQQTSVRREDKCETDFYDVT
WFKVMTAIINFYLPTLLMLWFYAKIYKAVRRQLGHYLSTQTSLSKDLNEKRDHMVL
 
LEFVTAAGLQKERKAAKQLGFIMAAFILCWIPYFIFFMVIAFCKNCCNEHLHMFTIWL

GYINSTLNPLIYPLCNENFKKTFKRILHIRS
HTR1A-Cys-GFP10-11 (SEQ ID NO.35): 
GTTTGISDVTVSYQVITSLLLGTLIFCAVLGNACVVAAIALERSLQNVANYLIGSL
AVTDLMVSVLVLPMAALYQVLNKWTLGQVTCDLFIALDVLCCTSSIWHLCAIALDR
YWAITDPCDYVNKRTPRRAAALISLTWLIGFLISIPPMLGWRTPEDRSDPDACTISKDH
GYTIYSTFGAFYIPLLLMLVLYGRIFRAARRQLGHYLSTQTSLSKDLNEKRDHMVLLE
FVTAAGLQKERKTVKTLGIIMGTFILCWLPFFIVALVLPFCESSCHMPTLLGAIINWLG
YSNSLLNPVIYAYFNKDFQNAFKKIIKCKFCRQ
HTR1B-Cys-GFP10-11 (SEQ ID NO.36): 
GTCSAKDYIYQDSISLPWKVLLVMLLALITLATTLSNAFVIATVYRTRKLHTPAN
YLIASLAVTDLLVSILVMPISTMYTVTGRWTLGQVVCDFWLSSDITCCTASIWHLCVI
ALDRYWAITDACEYSAKRTPKRAAVMIALVWVFSISISLPPFFWRQAKAEEEVSECV
VNTDHILYTVYSTVGAFYFPTLLLIALYGRIYVEARRQLGHYLSTQTSLSKDLNEKRD
HMVLLEFVTAAGLQKERKATKTLGIILGAFIVCWLPFFIISLVMPICKDACWFHLAIFD
FFTWLGYLNSLINPIIYTMSNEDFKQAFHKLIRFKCTS
HTR2B-Cys-GFP10-11 (SEQ ID NO.37): 
GAPTESIPEEMKQIVEEQGNKLHWAALLILMVIIPTIGGNTLVILAVSLEKKLQYA
TNYFLMSLAVADLLVGLFVMPIALLTIMFEAMWPLPLVLCPAWLFLDVLFSTASIWH
LCAISVDRYIAIKKPCQANQYNSRATAFIKITVVWLISIGIAIPVPIKGIETDVDNPNNIT
CVLTKERFGDFMLFGSLAAFFTPLAIMIVTYFLTIHALRRQLGHYLSTQTSLSKDLNEK
RDHMVLLEFVTAAGLQKEQRASKVLGIVFFLFLLMWCPFFITNITLVLCDSCNQTTLQ
MLLEIFVWIGYVSSGVNPLVYTLFNKTFRDAFGRYITCNYRATKSV
LPAR1-Cys-GFP10-11 (SEQ ID NO.38): 
STSIPVISQPQFTAMNEPQCFYNESIAFFYNRSGKHLATEWNTVSKLVMGLGITV
CIFIMLANLLVMVAIYVNRRFHFPIYYLMANLAAADFFAGLAYFYLMFNTGPNTRRL
TVSTWLLRQGLIDTSLTASVANLLAIAIERHITVFRMCLHTRMSNRRVVVVIVVIWTM
AIVMGAIPSVGWNCICDIENCSNMAPLYSDSYLVFWAIFNLVTFVVMVVLYAHIFGY
VRRQLGHYLSTQTSLSKDLNEKRDHMVLLEFVTAAGLQKEMSLLKTVVIVLGAFIIC
WTPGLVLLLLDVCCPQCDVLAYEKFFLLLAEFNSAMNPIIYSYRDKEMSATFRQILGR
MTNR1B-Cys-GFP10-11 (SEQ ID NO.39): 
GDGARPSWVAPALSAVLIVTTAVDVVGNLLVILSVLRNRKLRNAGNLFLVSLAL
ANLVVAFYPYPLILVAIFYDGWAFGEEHCKASAFVMGLSVIGSVWNITAIAIDRYLYI
CHSCAYHRIYRRWHTPLHICLIWLLTVVALLPNFFVGSLEYDPRIYSCTFIQTASTQYT
AAVVVIAFLLPIAVVSFCYLRIWVLVRRQLGHYLSTQTSLSKDLNEKRDHMVLLEFV
TAAGLQKELRSFLTMFVVFVIFAICFAPLNCIGLAVAINPQEMAPQIPEGLFVTSYLLA
YFNSCLNPIVYGLLDQNFRREYKRILLALWNPRHCIQDASKGS
NPY1R-Cys-GFP10-11 (SEQ ID NO.40): 
APNSTLFSQVENHSVHSNFSEKNAQLLAFENDDCHLPLAMIFTLALAYGAVIILG
VSGNLALIIIILKQKEMRNVTNILIVNLSFSDLLVAIMCLPFTFVYTLMDHWVFGEAMC
KLNPFVQCVSITVSIWSLVLIAVERHQLIINPCGWRPNNRHAYVGIAVIWVLAVASSL
PFLIYQVMTDEPFQNVTLDAYKDKYVCFDQFPSDSHRLSYTTLLLVLQYFGPLCFIFIC
YFKIYIRLRRQLGHYLSTQTSLSKDLNEKRDHMVLLEFVTAAGLQKEKRINIMLLSIV
VAFAVCWLPLTIFNTVFDWNHQIIATCNHNLLFLLCHLTAMISTCVNPIFYGFLNKNF
QRDLQFFFNFCDFRSRDDDYETIAMSTMHTD
OPRD-Cys-GFP10-11 (SEQ ID NO.41): 
PGARSASSLALAIAITALYSAVCAVGLLGNVLVMFGIVRYTKMKTATNIYIFNLA
LADALATSTLPFQSAKYLMETWPFGELLCKAVLSIDYYNMFTSIFTLTMMSVDRYIA
 
VCHPCKALDFRTPAKAKLINICIWVLASGVGVPIMVMAVTRPRDGAVVCMLQFPSPS
WYWDTVTKICVFLFAFVVPILIITVCYGLMLLRLRRQLGHYLSTQTSLSKDLNEKRDH
MVLLEFVTAAGLQKERRITRMVLVVVGAFVVCWAPIHIFVIVWTLVDIDRRDPLVVA
ALHLCIALGYANSSLNPVLYAFLDENFKRCFRQLCRKPCG
OX2R-Cys-GFP10-11 (SEQ ID NO.42): 
MGTKLEDSPPCRNWSSASELNETQEPFLNPTDYDDEEFLRYLWREYLHPKEYEW
VLIAGYIIVFVVALIGNVLVCVAVWKNHHMRTVTNYFIVNLSLADVLVTITCLPATLV
VDITETWFFGQSLCKVIPYLQTVSVSVSVLTLSCIALDRWYAICHPCMFKSTAKRARN
SIVIIWIVSCIIMIPQAIVMECSTVFPGLANKTTLFTVCDERWGGEIYPKMYHICFFLVT
YMAPLCLMVLAYLQIFRKLRRQLGHYLSTQTSLSKDLNEKRDHMVLLEFVTAAGLQ
KERKTARMLMVVLLVFAICYLPISILNVLKRVFGMFAHTEDRETVYAWFTFSHWLVY
ANSAANPIIYNFLSGKFREEFKAAFSCCCLGV
PTGDR-Cys-GFP10-11 (SEQ ID NO.43): 
MKSPFYRCQNTTSVEKGNSAVMGGVLFSTGLLGNLLALGLLARSGLGWCSRRP
LRPLPSVFYMLVCGLTVTDLLGKCLLSPVVLAAYAQNRSLRVLAPALDNSLCQAFAF
FMSFFGLSSTLQLLAMALECWLSLGHPCFYRRHITLRLGALVAPVVSAFSLAFCALPF
MGFGKFVQYCPGTWCFIQMVHEEGSLSVLGYSVLYSSLMALLVLATVLCNLGAMRN
LYAMHRRLQGHYLSTQTSLSKDLNEKRDHMVLLEFVTAAGPLEELDHLLLLALMTV
LFTMCSLPVIYRAYYGAFKDVKEKNRTSEEAEDLRALRFLSVISIVDPWIFIIFRSPVFRI
FFHKIFIRPLRYRSRCSNSTNMESSL
GPR146 (SEQ ID NO.44): 
MWSCSWFNGTGLVEELPACQDLQLGLSLLSLLGLVVGVPVGLCYNALLVLANL
HSKASMTMPDVYFVNMAVAGLVLSALAPVHLLGPPSSRWALWSVGGEVHVALQIPF
NVSSLVAMYSTALLSLDHYIERALPCTYMASVYNTRHVCGFVWGGALLTSFSSLLFY
 
ICSHVSTRALECAKMQNAEAADATLVFIGYVVPALATLYALVLLSRVRRRQLGHYLS
TQTSLSKDLNEKRDHMVLLEFVTAAGLQKEPSAHRLLVATVCTQFGLWTPHYLILLG
HTVIISRGKPVDAHYLGLLHFVKDFSKLLAFSSSFVTPLLYRYMNQSFPSKLQRLMKK
LPCGDRHCSPDHMGVQQVLA
MCHR1 (SEQ ID NO.45): 
MDLEASLLPTGPNASNTSDGPDNLTSAGSPPRTGSISYINIIMPSVFGTICLLGIIGN
STVIFAVVKKSKLHWCNNVPDIFIINLSVVDLLFLLGMPFMIHQLMGNGVWHFGETM
CTLITAMDANSQFTSTYILTAMAIDRYLATVHPCSSTKFRKPSVATLVICLLWALSFISI
TPVWLYARLIPFPGGAVGCGIRLPNPDTDLYWFTLYQFFLAFALPFVVITAAYVRILQ
RMTSSVGHYLSTQTSLSKDLNEKRDHMVLLEFVTAAGRLRTKRVTRTAIAICLVFFV
CWAPYYVLQLTQLSISRPTLTFVYLYNAAISLGYANSCLNPFVYIVLCETFRKRLVLS
VKPAAQGQLRAVSNAQTADEERTESKGT
TAAR1 (SEQ ID NO.46): 
MMPFCHNIINISCVKNNWSNDVRASLYSLMVLIILTTLVGNLIVIVSISHFKQLHT
PTNWLIHSMATVDFLLGCLVMPYSMVRSAEHCWYFGEVFCKIHTSTDIMLSSASIFHL
SFISIDRYYAVCDPCRYKAKMNILVICVMIFISWSVPAVFAFGMIFLELNFKGAEEIYY
KHVHCRGGCSVFFSKISGVLTFMTSFYIPGSIMLCVYYRIYLIAKEQARGHYLSTQTSL
SKDLNEKRDHMVLLEFVTAAGQSKERKAVKTLGIVMGVFLICWCPFFICTVMDPFLH
YIIPPTLNDVLIWFGYLNSTFNPMVYAFFYPWFRKALKMMLFGKIFQKDSSRCKLFLE
LSS
AGTR1 (SEQ ID NO.47): 
MILNSSTEDGIKRIQDDCPKAGRHNYIFVMIPTLYSIIFVVGIFGNSLVVIVIYFYM
KLKTVASVFLLNLALADLCFLLTLPLWAVYTAMEYRWPFGNYLCKIASASVSFNLYA
SVFLLTCLSIDRYLAIVHPCKSRLRRTMLVAKVTCIIIWLLAGLASLPAIIHRNVFFIEN
 
TNITVCAFHYESQNSTLPIGLGLTKNILGFLFPFLIILTSYTLIWKALKKAYGHYLSTQT
SLSKDLNEKRDHMVLLEFVTAAGKPRNDDIFKIIMAIVLFFFFSWIPHQIFTFLDVLIQL
GIIRDCRIADIVDTAMPITICIAYFNNCLNPLFYGFLGKKFKRYFLQLLKYIPPKAKSHS
NLSTKMSTLSYRPSDNVSSSTKKPAPCFEVE
FPR1 (SEQ ID NO.48): 
METNSSLPTNISGGTPAVSAGYLFLDIITYLVFAVTFVLGVLGNGLVIWVAGFRM
THTVTTISYLNLAVADFCFTSTLPFFMVRKAMGGHWPFGWFLCKFVFTIVDINLFGSV
FLIALIALDRCVCVLHPCWTQNHRTVSLAKKVIIGPWVMALLLTLPVIIRVTTVPGKT
GTVACTFNFSPWTNDPKERINVAVAMLTVRGIIRFIIGFSAPMSIVAVSYGLIATKIHK
QGGHYLSTQTSLSKDLNEKRDHMVLLEFVTAAGLIKSSRPLRVLSFVAAAFFLCWSP
YQVVALIATVRIRELLQGMYKEIGIAVDVTSALAFFNSCLNPMLYVFMGQDFRERLIH
ALPASLERALTEDSTQTSDTATNSTLPSAEVELQAK
GALR1 (SEQ ID NO.49): 
MELAVGNLSEGNASWPEPPAPEPGPLFGIGVENFVTLVVFGLIFALGVLGNSLVI
TVLARSKPGKPRSTTNLFILNLSIADLAYLLFCIPFQATVYALPTWVLGAFICKFIHYFF
TVSMLVSIFTLAAMSVDRYVAIVHSCRSSSLRVSRNALLGVGCIWALSIAMASPVAY
HQGLFHPRASNQTFCWEQWPDPRHKKAYVVCTFVFGYLLPLLLICFCYAKVLNHLH
KKLGHYLSTQTSLSKDLNEKRDHMVLLEFVTAAGEASKKKTAQTVLVVVVVFGISW
LPHHIIHLWAEFGVFPLTPASFLFRITAHCLAYSNSSVNPIIYAFLSENFRKAYKQVFKC
HIRKDSHLSDTKESKSRIDTPPSTNCTHV
GHSR (SEQ ID NO.50) 
MWNATPSEEPGFNLTLADLDWDASPGNDSLGDELLQLFPAPLLAGVTATCVAL
FVVGIAGNLLTMLVVSRFRELRTTTNLYLSSMAFSDLLIFLCMPLDLVRLWQYRPWN
FGDLLCKLFQFVSESCTYATVLTITALSVERYFAICFPCRAKVVVTKGRVKLVIFVIW
 
AVAFCSAGPIFVLVGVEHENGTDPWDTNECRPTEFAVRSGLLTVMVWVSSIFFFLPVF
CLTVLYSLIGRKLRRQLGHYLSTQTSLSKDLNEKRDHMVLLEFVTAAGLQKEKQTVK
MLAVVVFAFILCWLPFHVGRYLFSKSFEPGSLEIAQISQYCNLVSFVLFYLSAAINPILY
NIMSKKYRVAVFRLLGFEPFSQRKLSTLKDESSRAWTESSINT
CCKAR (SEQ ID NO.51) 
MDVVDSLLVNGSNITPPCELGLENETLFCLDQPRPSKEWQPAVQILLYSLIFLLSV
LGNTLVITVLIRNKRMRTVTNIFLLSLAVSDLMLCLFCMPFNLIPNLLKDFIFGSAVCK
TTTYFMGTSVSVSTFNLVAISLERYGAICKPCQSRVWQTKSHALKVIAATWCLSFTIM
TPYPIYSNLVPFTKNNNQTANMCRFLLPNDVMQQSWHTFLLLILFLIPGIVMMVAYG
LISLELYQGIGHYLSTQTSLSKDLNEKRDHMVLLEFVTAAGLMAKKRVIRMLIVIVVL
FFLCWMPIFSANAWRAYDTASAERRLSGTPISFILLLSYTSSCVNPIIYCFMNKRFRLGF
MATFPCCPNPGPPGARGEVGEEEEGGTTGASLSRFSYSHMSASVPPQ
MTLR (SEQ ID NO.52) 
MGSPWNGSDGPEGAREPPWPALPPCDERRCSPFPLGALVPVTAVCLCLFVVGVS
GNVVTVMLIGRYRDMRTTTNLYLGSMAVSDLLILLGLPFDLYRLWRSRPWVFGPLL
CRLSLYVGEGCTYATLLHMTALSVERYLAICRPCRARVLVTRRRVRALIAVLWAVA
LLSAGPFLFLVGVEQDPGISVVPGLNGTARIASSPLASSPPLWLSRAPPPSPPSGPETAE
AAALFSRECRPSPAQLGALRVMLWVTTAYFFLPFLCLSILYGLIGRELRRQLGHYLST
QTSLSKDLNEKRDHMVLLEFVTAAGLQKERQTVRVLLVVVLAFIICWLPFHVGRIIYI
NTEDSRMMYFSQYFNIVALQLFYLSASINPILYNLISKKYRAAAFKLLLARKSRPRGF
HRSRDTAGEVAGDTGGDTVGYTETSANVKTMG
EDNRA (SEQ ID NO.53) 
DNPERYSTNLSNHVDDFTTFRGTELSFLVTTHQPTNLVLPSNGSMHNYCPQQTKI
TSAFKYINTVISCTIFIVGMVGNATLLRIIYQNKCMRNGPNALIASLALGDLIYVVIDLP
 
INVFKLLAGRWPFDHNDFGVFLCKLFPFLQKSSVGITVLNLCALSVDRYRAVASWCR
VQGIGIPLVTAIEIVSIWILSFILAIPEAIGFVMVPFEYRGEQHKTCMLNATSKFMEFYQ
DVKDWWLFGFYFCMPLVCTAIFYTLMTCEMLNRRGHYLSTQTSLSKDLNEKRDHM
VLLEFVTAAGLKQRREVAKTVFCLVVIFALCWFPLHLSRILKKTVYNEMDKNRCELL
SFLLLMDYIGINLATMNSCINPIALYFVSKKFKNCFQSCLCCCCYQSKSLMTSVPMNG
TSIQWKNHDQNNHNTDRSSHKDSMN
PRLHR (SEQ ID NO.54) 
MASSTTRGPRVSDLFSGLPPAVTTPANQSAEASAGNGSVAGADAPAVTPFQSLQ
LVHQLKGLIVLLYSVVVVVGLVGNCLLVLVIARVRRLHNVTNFLIGNLALSDVLMCT
ACVPLTLAYAFEPRGWVFGGGLCHLVFFLQPVTVYVSVFTLTTIAVDRYVVLVHPCR
RRISLRLSAYAVLAIWALSAVLALPAAVHTYHVELKPHDVRLCEEFWGSQERQRQLY
AWGLLLVTYLLPLLVILLSYVRVSVKLRNRVGHYLSTQTSLSKDLNEKRDHMVLLEF
VTAAGGGRARRRRTFCLLVVIVVVFAVCWLPLHVFNLLRDLDPHAIDPYAFGLVQLL
CHWLAMSSACYNPFIYAWLHDSFREELRKLLVAWPRKIAPHGQNMTVSVVI
NPFF1 (SEQ ID NO.55): 
MEGEPSQPPNSSWPLSQNGTNTEATPATNLTFSSYYQHTSPVAAMFIVAYALIFL
LCMVGNTLVCFIVLKNRHMHTVTNMFILNLAVSDLLVGIFCMPTTLVDNLITGWPFD
NATCKMSGLVQGMSVSASVFTLVAIAVERFRCIVHPCREKLTLRKALVTIAVIWALA
LLIMCPSAVTLTVTREEHHFMVDARNRSYPLYSCWEAWPEKGMRRVYTTVLFSHIYL
APLALIVVMYARIARKLCQAPGHYLSTQTSLSKDLNEKRDHMVLLEFVTAAGGGSRR
RARVVHMLVMVALFFTLSWLPLWALLLLIDYGQLSAPQLHLVTVYAFPFAHWLAFF
NSSANPIIYGYFNENFRRGFQAAFRARLCPRPSGSHKE
CXCR1 (SEQ ID NO.56): MSNITDPQMWDFDDLNFTGMPPADEDYSPCMLETETLNKYVVIIAYALVFLLSL 
LGNSLVMLVILYSRVGRSVTDVYLLNLALADLLFALTLPIWAASKVNGWIFGTFLCK
VVSLLKEVNFYSGILLLACISVDRYLAIVHACRTLTQKRHLVKFVCLGCWGLSMNLS
LPFFLFRQAYHPNNSSPVCYEVLGNDTAKWRMVLRILPHTFGFIVPLFVMLFCYGFTL
RTLFKAHGHYLSTQTSLSKDLNEKRDHMVLLEFVTAAGGGMGQKHRAMRVIFAVV
LIFLLCWLPYNLVLLADTLMRTQVIQESCERRNNIGRALDATEILGFLHSCLNPIIYAFI
GQNFRHGFLKILAMHGLVSKEFLARHRVTSYTSSSVNVSSNL
TRFR (SEQ ID NO.57) 
MENETVSELNQTQLQPRAVVALEYQVVTILLVLIICGLGIVGNIMVVLVVMRTK
HMRTPTNCYLVSLAVADLMVLVAAGLPNITDSIYGSWVYGYVGCLCITYLQYLGINA
SSCSITAFTIERYIAICHPIKAQFLCTFSRAKKIIIFVWAFTSLYCMLWFFLLDLNISTYK
DAIVISCGYKISRNYYSPIYLMDFGVFYVVPMILATVLYGFIARILFLNPGHYLSTQTSL
SKDLNEKRDHMVLLEFVTAAGGGVSSRKQVTKMLAVVVILFALLWMPYRTLVVVN
SFLSSPFQENWFLLFCRICIYLNSAINPVIYNLMSQKFRAAFRKLCNCKQKPTEKPANY
SVALNYSVIKESDHFSTELDDITVTDTYLSATKVSFDDTCLASEVSFSQS
CML1 (SEQ ID NO.58): 
MRMEDEDYNTSISYGDEYPDYLDSIVVLEDLSPLEARVTRIFLVVVYSIVCFLGIL
GNGLVIIIATFKMKKTVNMVWFLNLAVADFLFNVFLPIHITYAAMDYHWVFGTAMC
KISNFLLIHNMFTSVFLLTIISSDRCISVLLPVWSQNHRSVRLAYMACMVIWVLAFFLS
SPSLVFRDTANLHGKISCFNNFSLSTPGSSSWPTHSQMDPVGYSRHMVVTVTRFLCGF
LVPVLIITACYLTIVCKLQRNRGHYLSTQTSLSKDLNEKRDHMVLLEFVTAAGGGLA
KTKKPFKIIVTIIITFFLCWCPYHTLNLLELHHTAMPGSVFSLGLPLATALAIANSCMNP
ILYVFMGQDFKKFKVALFSRLVNALSEDTGHSSYPSHRSFTKMSSMNERTSMNERET
GML
QRFPR (SEQ ID NO.59) 
MQALNITPEQFSRLLRDHNLTREQFIALYRLRPLVYTPELPGRAKLALVLTGVLIF
ALALFGNALVFYVVTRSKAMRTVTNIFICSLALSDLLITFFCIPVTMLQNISDNWLGGA
FICKMVPFVQSTAVVTEILTMTCIAVERHQGLVHPCKMKWQYTNRRAFTMLGVVW
LVAVIVGSPMWHVQQLEIKYDFLYEKEHICCLEEWTSPVHQKIYTTFILVILFLLPLMV
MLILYSKIGYELWIKKGHYLSTQTSLSKDLNEKRDHMVLLEFVTAAGGGARKKKRA
VIMMVTVVALFAVCWAPFHVVHMMIEYSNFEKEYDDVTIKMIFAIVQIIGFSNSICNPI
VYAFMNENFKKNVLSAVCYCIVNKTFSPAQRHGNSGITMMRKKAKFSLRENPVEET
KGEAFSDGNIEVKLCEQTEEKKKLKRHLALFRSELAENSPLDSGH
SSTR2 (SEQ ID NO.60): 
MDMADEPLNGSHTWLSIPFDLNGSVVSTNTSNQTEPYYDLTSNAVLTFIYFVVCI
IGLCGNTLVIYVILRYAKMKTITNIYILNLAIADELFMLGLPFLAMQVALVHWPFGKAI
CRVVMTVDGINQFTSIFCLTVMSIDRYLAVVHPCKSAKWRRPRTAKMITMAVWGVS
LLVILPIMIYAGLRSNQWGRSSCTINWPGESGAWYTGFIIYTFILGFLVPLTIICLCYLFII
IKVKSSGGHYLSTQTSLSKDLNEKRDHMVLLEFVTAAGGGKKSEKKVTRMVSIVVA
VFIFCWLPFYIFNVSSVSMAISPTPALKGMFDFVVVLTYANSCANPILYAFLSDNFKKS
FQNVLCLVKVSGTDDGERSDSKQDKSRLNETTETQRTLLNGDLQTSI
FFAR1 (SEQ ID NO.61): 
MDLPPQLSFGLYVAAFALGFPLNVLAIRGATAHARLRLTPSLVYALNLGCSDLL
LTVSLPLKAVEALASGAWPLPASLCPVFAVAHFFPLYAGGGFLAALSAGRYLGAAFP
CGYQAFRRPCYSWGVCAAIWALVLCHLGLVFGLEAPGGWLDHSNTSLGINTPVNGS
PVCLEAWDPASAGPARFSLSLLLFFLPLAITAFCYVGCLRALARSGGHYLSTQTSLSK
DLNEKRDHMVLLEFVTAAGGGHRRKLRAAWVAGGALLTLLLCVGPYNASNVASFL
YPNLGGSWRKLGLITGAWSVVLNPLVTGYLGRGPGLKTVCAARTQGGKSQK
PTAFR (SEQ ID NO.62) 
MEPHDSSHMDSEFRYTLFPIVYSIIFVLGVIANGYVLWVFARLYPCKKFNEIKIFM
VNLTMADMLFLITLPLWIVYYQNQGNWILPKFLCNVAGCLFFINTYCSVAFLGVITYN
RFQAVTRPCKTAQANTRKRGISLSLVIWVAIVGAASYFLILDSTNTVPDSAGSGNVTR
CFEHYEKGSVPVLIIHIFIVFSFFLVFLIILFCNLVIIRTLLMQPGHYLSTQTSLSKDLNEK
RDHMVLLEFVTAAGGGAEVKRRALWMVCTVLAVFIICFVPHHVVQLPWTLAELGFQ
DSKFHQAINDAHQVTLCLLSTNCVLDPVIYCFLTKKFRKHLTEKFYSMRSSRKCSRAT
TDTVTEVVVPFNQIPGNSLKN
P2RY1 (SEQ ID NO.63): 
MTEVLWPAVPNGTDAAFLAGPGSSWGNSTVASTAAVSSSFKCALTKTGFQFYY
LPAVYILVFIIGFLGNSVAIWMFVFHMKPWSGISVYMFNLALADFLYVLTLPALIFYYF
NKTDWIFGDAMCKLQRFIFHVNLYGSILFLTCISAHRYSGVVYPCKSLGRLKKKNAIC
ISVLVWLIVVVAISPILFYSGTGVRKNKTITCYDTTSDEYLRSYFIYSMCTTVAMFCVP
LVLILGCYGLIVRALIYKDGHYLSTQTSLSKDLNEKRDHMVLLEFVTAAGGGSPLRR
KSIYLVIIVLTVFAVSYIPFHVMKTMNLRARLDFQTPAMCAFNDRVYATYQVTRGLA
SLNSCVDPILYFLAGDTFRRRLSRATRKASRRSEANLQSKSEDMTLNILPEFKQNGDT
SL
HCAR2 (SEQ ID NO.64): 
MNRHHLQDHFLEIDKKNCCVFRDDFIVKVLPPVLGLEFIFGLLGNGLALWIFCFH
LKSWKSSRIFLFNLAVADFLLIICLPFLMDNYVRRWDWKFGDIPCRLMLFMLAMNRQ
GSIIFLTVVAVDRYFRVVHPCHALNKISNRTAAIISCLLWGITIGLTVHLLKKKMPIQN
GGANLCSSFSICHTFQWHEAMFLLEFFLPLGIILFCSARIIWSLRQRQGHYLSTQTSLSK
DLNEKRDHMVLLEFVTAAGGGHAKIKRAITFIMVVAIVFVICFLPSVVVRIRIFWLLHT
SGTQNCEVYRSVDLAFFITLSFTYMNSMLDPVVYYFSSPSFPNFFSTLINRCLQRKMT
GEPDNNRSTSVELTGDPNKTRGAPEALMANSGEPWSPSYLGPTSP
SUCR1 (SEQ ID NO.65): 
MLGIMAWNATCKNWLAAEAALEKYYLSIFYGIEFVVGVLGNTIVVYGYIFSLKN
WNSSNIYLFNLSVSDLAFLCTLPMLIRSYANGNWIYGDVLCISNRYVLHANLYTSILFL
TFISIDRYLIIKYPCREHLLQKKEFAILISLAIWVLVTLELLPILPLINPVITDNGTTCNDF
ASSGDPNYNLIYSMCLTLLGFLIPLFVMCFFYYKIALFLKQRNGHYLSTQTSLSKDLNE
KRDHMVLLEFVTAAGGGLQKEEKPLNLVIMAVVIFSVLFTPYHVMRNVRIASRLGS
WKQYQCTQVVINSFYIVTRPLAFLNSVINPVFYFLLGDHFRDMLMNQLRHNFKSLTSF
SRWAHELLLSFREK
APJ (SEQ ID NO.66) 
EEGGDFDNYYGADNQSECEYTDWKSSGALIPAIYMLVFLLGTTGNGLVLWTVF
RSSREKRRSADIFIASLAVADLTFVVTLPLWATYTYRDYDWPFGTFFCKLSSYLIFVN
MYASVFCLTGLSFDRYLAIVRPVANARLRLRVSGAVATAVLWVLAALLAMPVMVL
RTTGDLENTTKVQCYMDYSMVATVSSEWAWEVGLGVSSTTVGFVVPFTIMLTCYFF
IAQTIAGHSGGHYLSTQTSLSKDLNEKRDHMVLLEFVTAAGGGGLRKRRRLLSIIVVL
VVTFALCWMPYHLVKTLYMLGSLLHWPCDFDLFLMNIFPYCTCISYVNSCLNPFLYA
FFDPRFRQACTSMLCCGQSR
GPR39 (SEQ ID NO.67): 
MASPSLPGSDCSQIIDHSHVPEFEVATWIKITLILVYLIIFVMGLLGNSATIRVTQV
LQKKGYLQKEVTDHMVSLACSDILVFLIGMPMEFYSIIWNPLTTSSYTLSCKLHTFLFE
ACSYATLLHVLTLSFERYIAICHPCRYKAVSGPCQVKLLIGFVWVTSALVALPLLFAM
GTEYPLVNVPSHRGLTCNRSSTRHHEQPETSNMSICTNLSSRWTVFQSSIFGAFVVYL
VVLLSVAFMCWNMMQVLRRQLGHYLSTQTSLSKDLNEKRDHMVLLEFVTAAGLQK
ERQTIIFLRLIVVTLAVCWMPNQIRRIMAAAKPKHDWTRSYFRAYMILLPFSETFFYLS
SVINPLLYTVSSQQFRRVFVQVLCCRLSLQHANHEKRLRVHAHSTTDSARFVQRPLLF
 ASRRQSSARRTEKIFLSTFQSEAEPQSKSQSLSLESLEPNSGAKPANSAAENGFQEHEV
GPR75 (SEQ ID NO.68): 
MNSTGHLQDAPNATSLHVPHSQEGNSTSLQEGLQDLIHTATLVTCTFLLAVIFCL
GSYGNFIVFLSFFDPAFRKFRTNFDFMILNLSFCDLFICGVTAPMFTFVLFFSSASSIPDA
FCFTFHLTSSGFIIMSLKTVAVIALHRLRMVLGKCPNRTASFPCTVLLTLLLWATSFTL
ATLATLKTSKSHLCLPMSSLIAGKGKAILSLYVVDFTFCVAVVSVSYIMIAQTLRKNA
GHYLSTQTSLSKDLNEKRDHMVLLEFVTAAGTAKDSKAVVTCVIIVLSVLVCCLPLGI
SLVQVVLSSNGSFILYQFELFGFTLIFFKSGLNPFIYSRNSAGLRRKVLWCLQYIG
PTGER4 (SEQ ID NO.69): 
STPGVNSSASLSPDRLNSPVTIPAVMFIFGVVGNLVAIVVLCKSRKEQKETTFYTL
VCGLAVTDLLGTLLVSPVTIATYMKGQWPGGQPLCEYSTFILLFFSLSGLSIICAMSVE
RYLAINHACFYSHYVDKRLAGLTLFAVYASNVLFCALPNMGLGSSRLQYPDTWCFID
WTTNVTAHAAYSYMYAGFSSFLILATVLCNVLVCGALRRQLGHYLSTQTSLSKDLN
EKRDHMVLLEFVTAAGLQKEIQMVILLIATSLVVLICSIPLVVRVFVNQLYQPSLEREV
SKNPDLQAIRIASVNPILDPWIYILLRKTVLSKAIEKIKCLFCRIGGSRRERSGQHCSDS
Q
CCR1 (SEQ ID NO.70): 
ETPNTTEDYDTTTEFDYGDATPCQKVNERAFGAQLLPPLYSLVFVIGLVGNILVV
LVLVQYKRLKNMTSIYLLNLAISDLLFLFTLPFWIDYKLKDDWVFGDAMCKILSGFY
YTGLYSEIFFIILLTIDRYLAIVHACFALRARTVTFGVITSIIIWALAILASMPGLYFSKT
QWEFTHHTCSLHFPHESLREWKLFQALKLNLFGLVLPLLVMIICYTGIIKILRRQLGHY
LSTQTSLSKDLNEKRDHMVLLEFVTAAGLQKESKAVRLIFVIMIIFFLFWTPYNLTILIS
VFQDFLFTHECEQSRHLDLAVQVTEVIAYTHCCVNPVIYAFVGERFRKYLRQLFHRR
VAVHLVKWLPFLSVDRLERVSSTSPSTGEHELSAGF
CCR4 (SEQ ID NO.71):

NPTDIADTTLDESIYSNYYLYESIPKPCTKEGIKAFGELFLPPLYSLVFVFGLLGNS
VVVLVLFKYKRLRSMTDVYLLNLAISDLLFVFSLPFWGYYAADQWVFGLGLCKMIS
WMYLVGFYSGIFFVMLMSIDRYLAIVHACFSLRARTLTYGVITSLATWSVAVFASLP
GFLFSTCYTERNHTYCKTKYSLNSTTWKVLSSLEINILGLVIPLGIMLFCYSMIIRTLRR
QLGHYLSTQTSLSKDLNEKRDHMVLLEFVTAAGLQKENKAVKMIFAVVVLFLGFWT
PYNIVLFLETLVELEVLQDCTFERYLDYAIQATETLAFVHCCLNPIIYFFLGEKFRKYIL
QLFKTCRGLFVLCQYCGLLQIYSADTPSSSYTQSTMDHDLHDAL

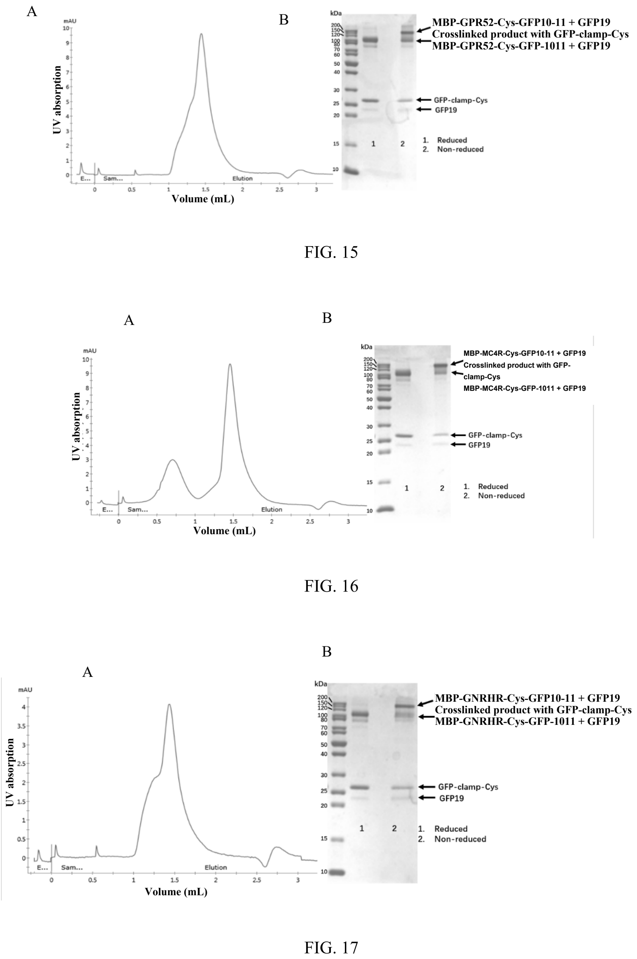

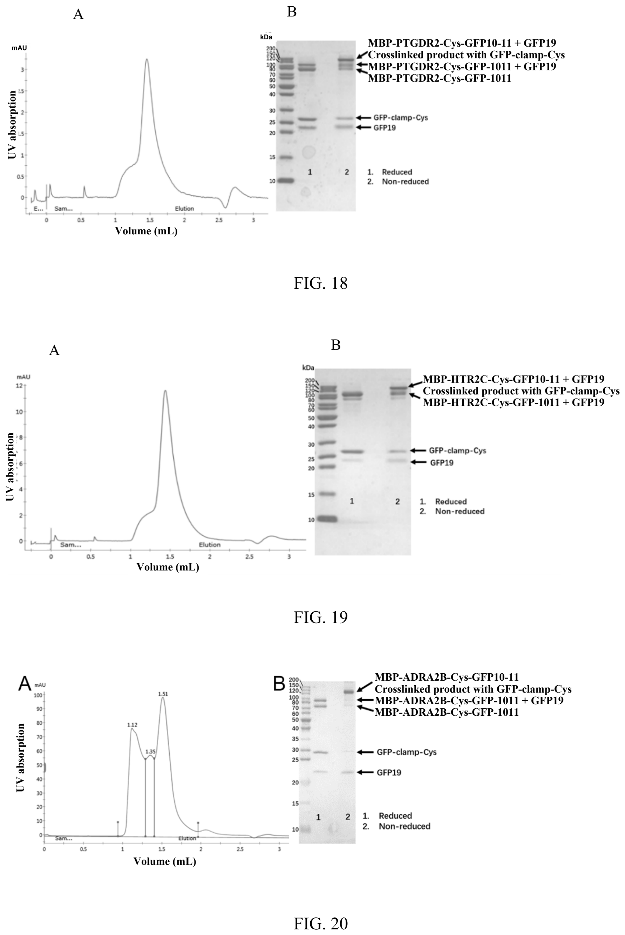

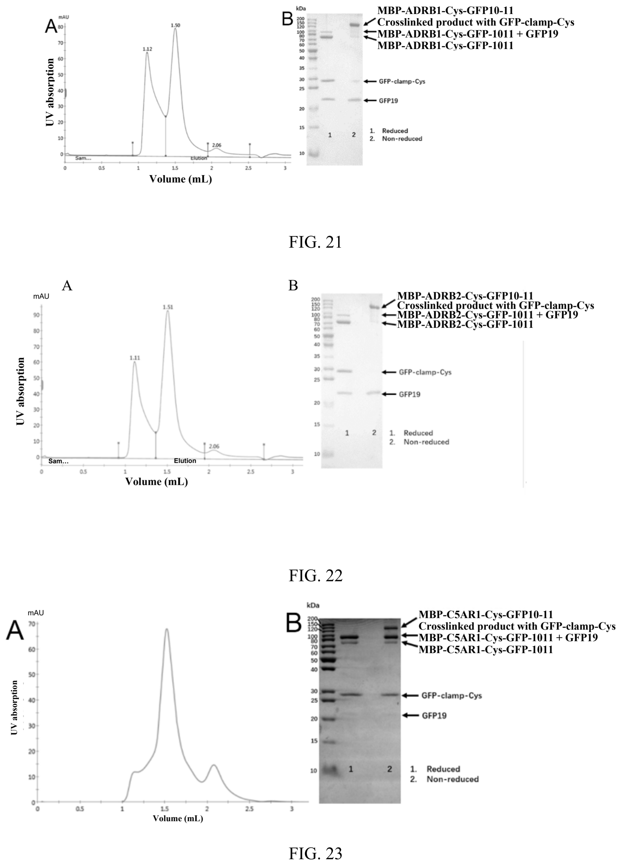

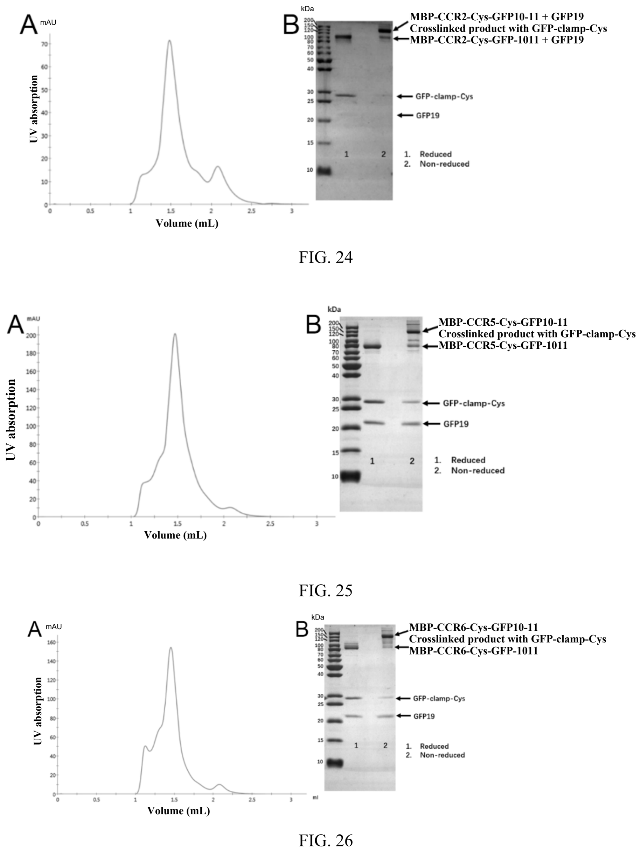

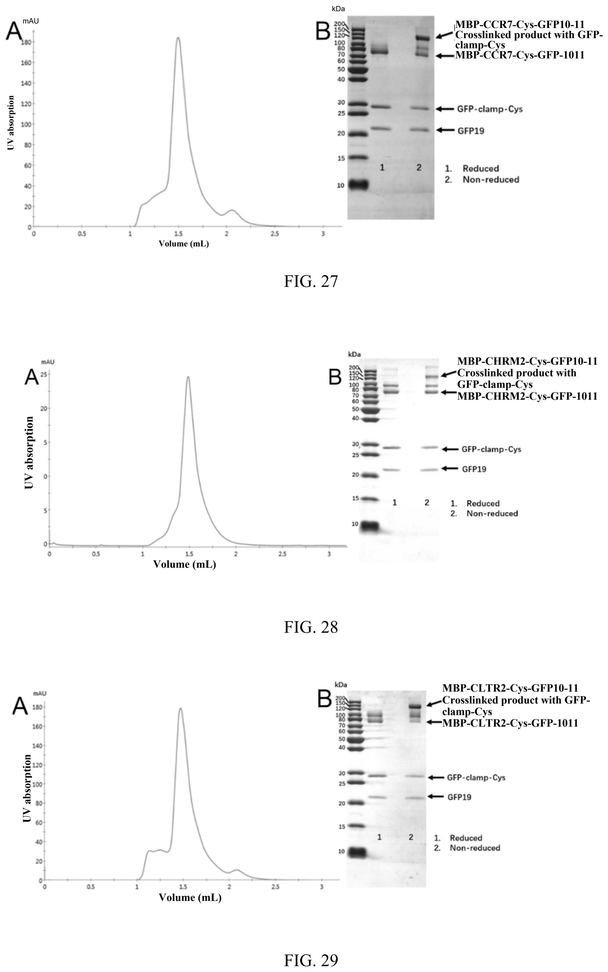

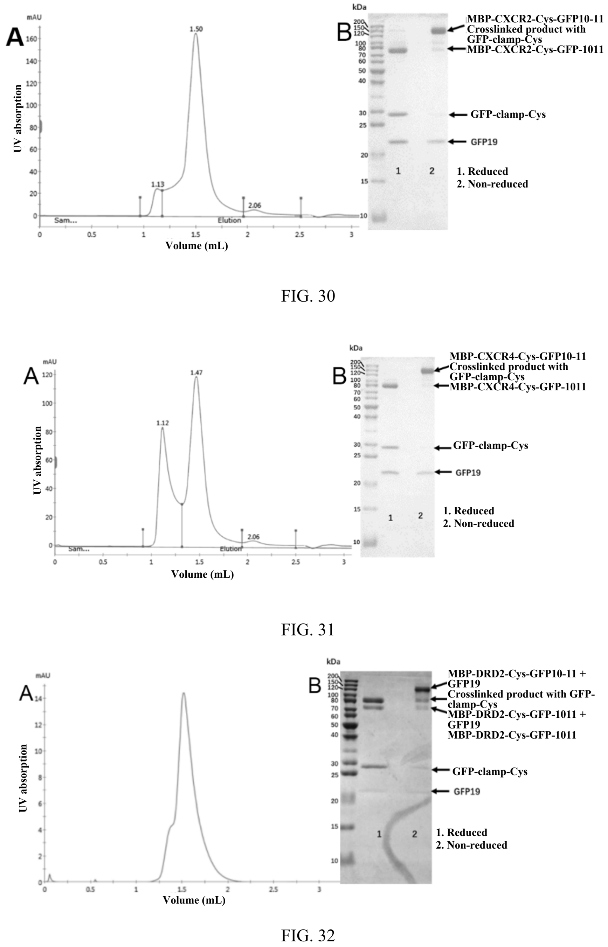

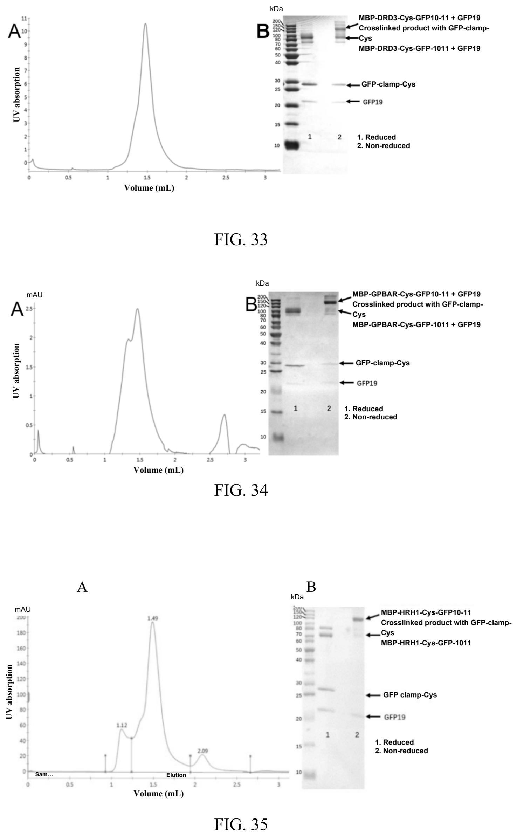

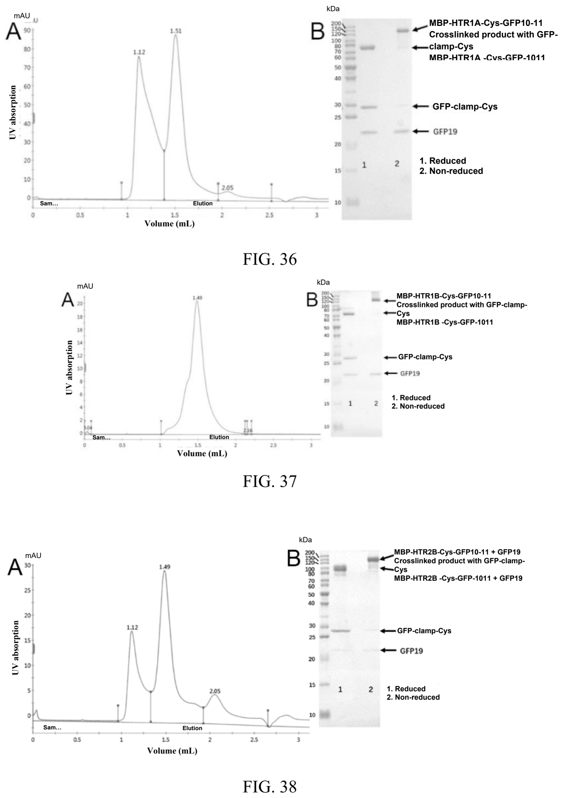

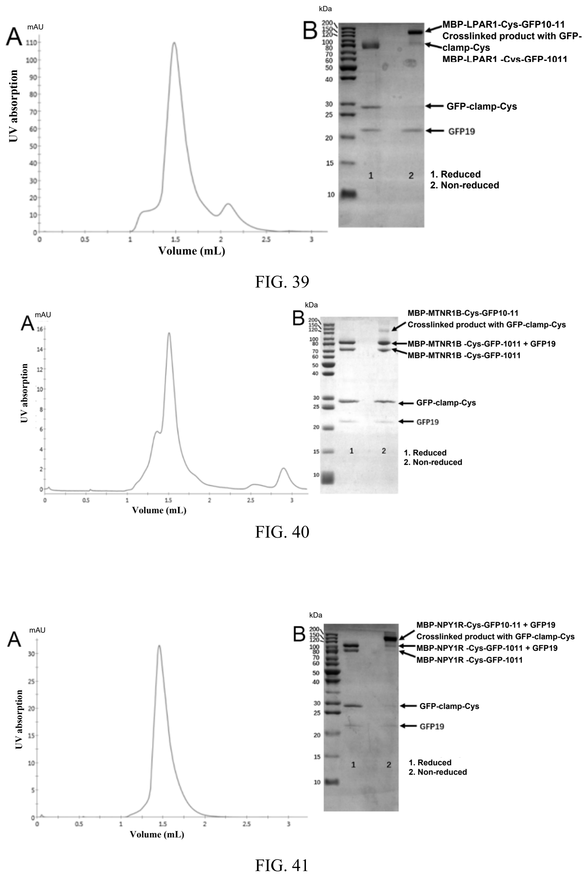

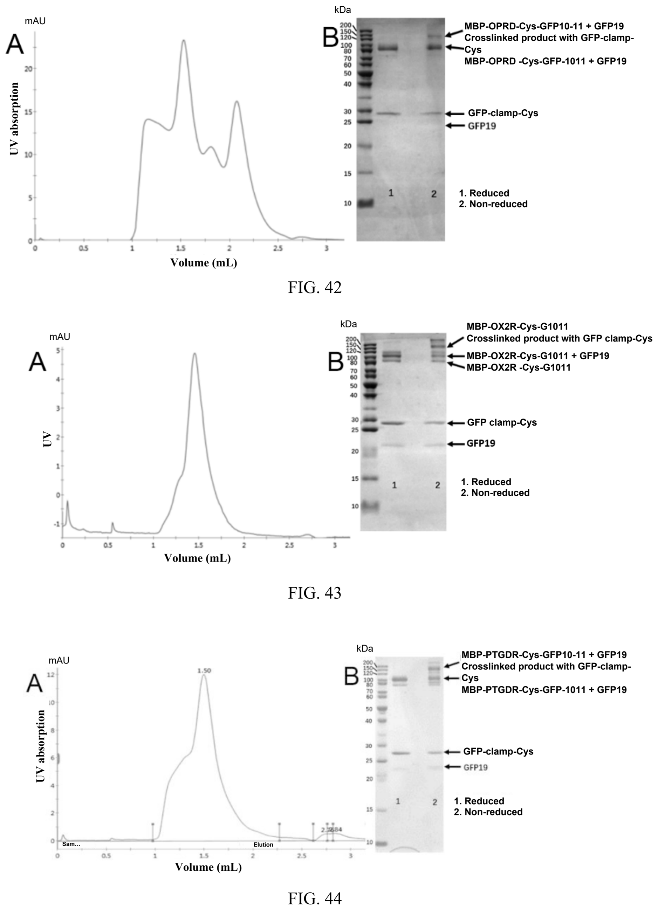

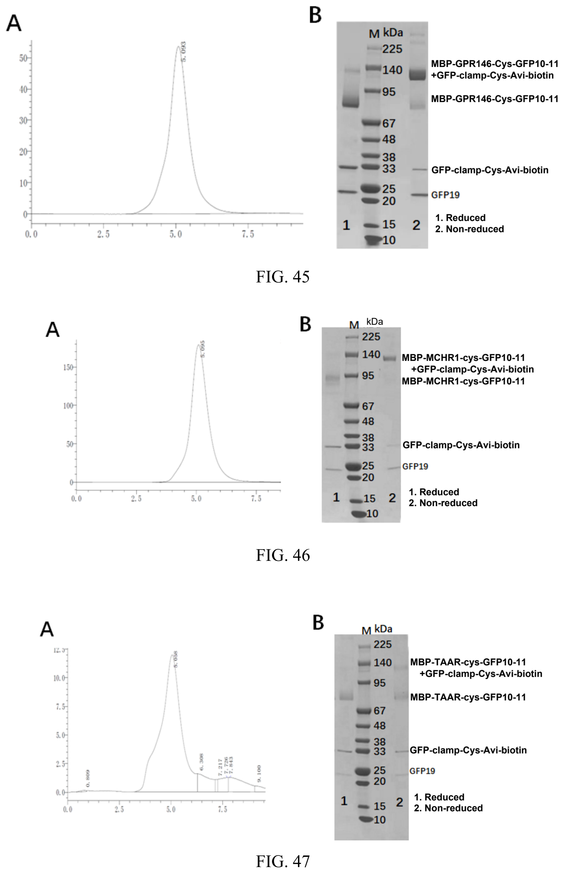

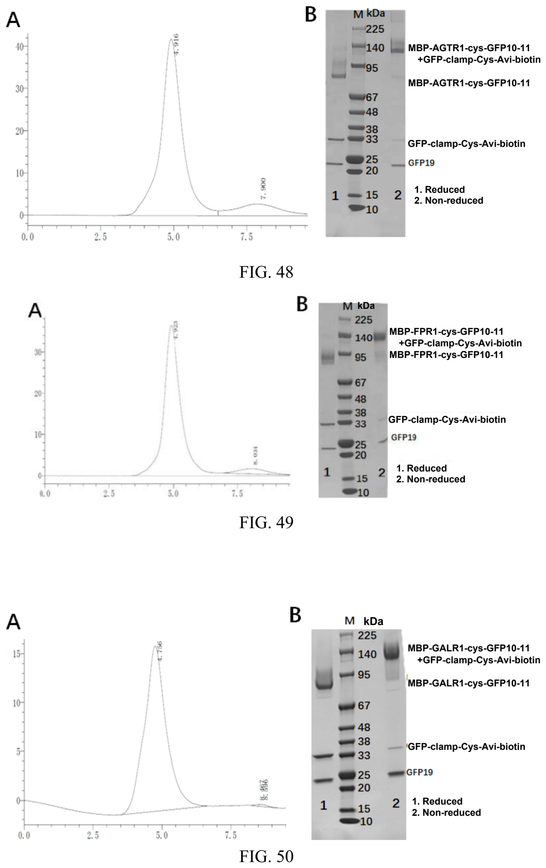

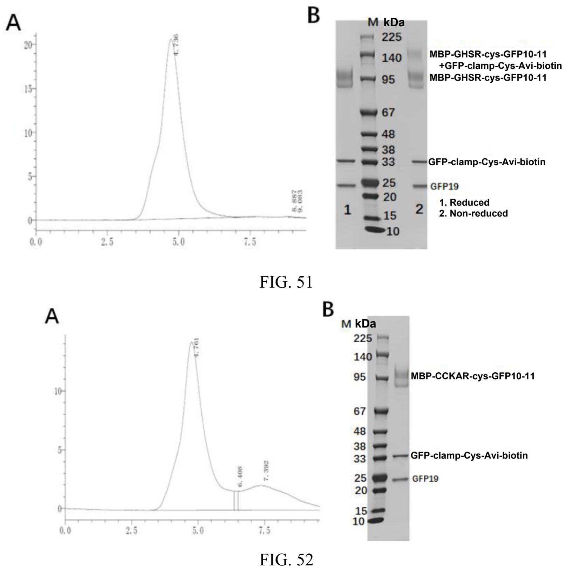

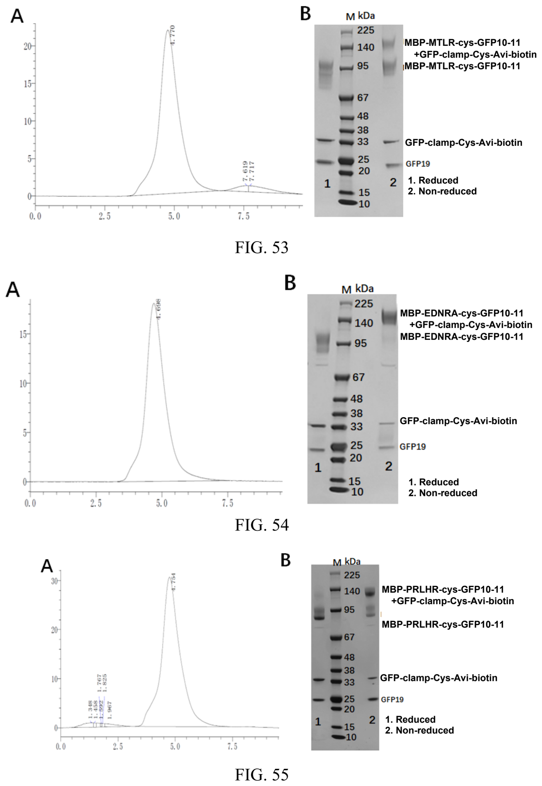

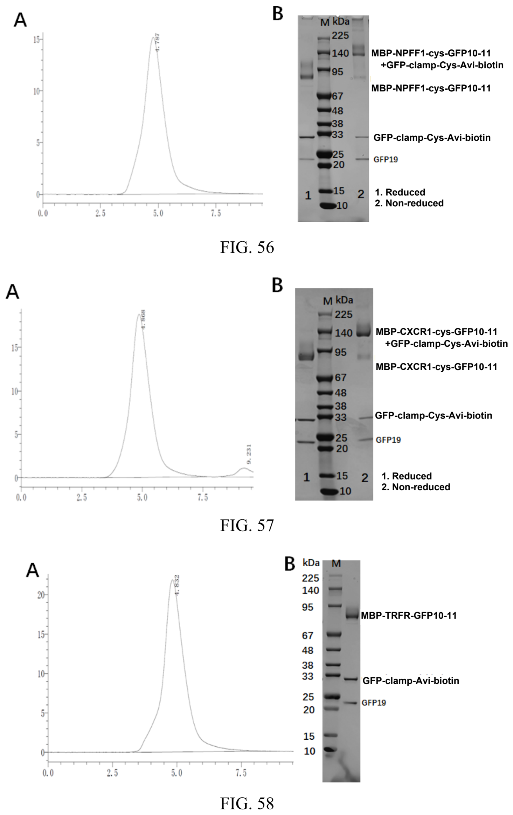

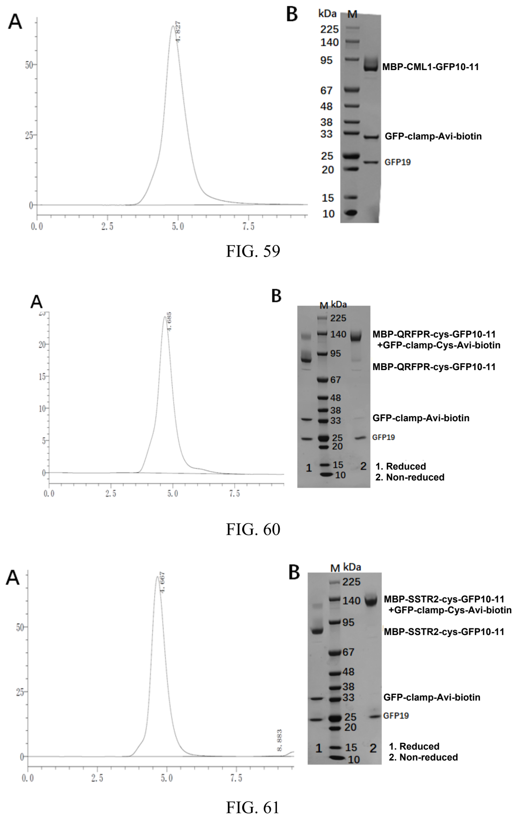

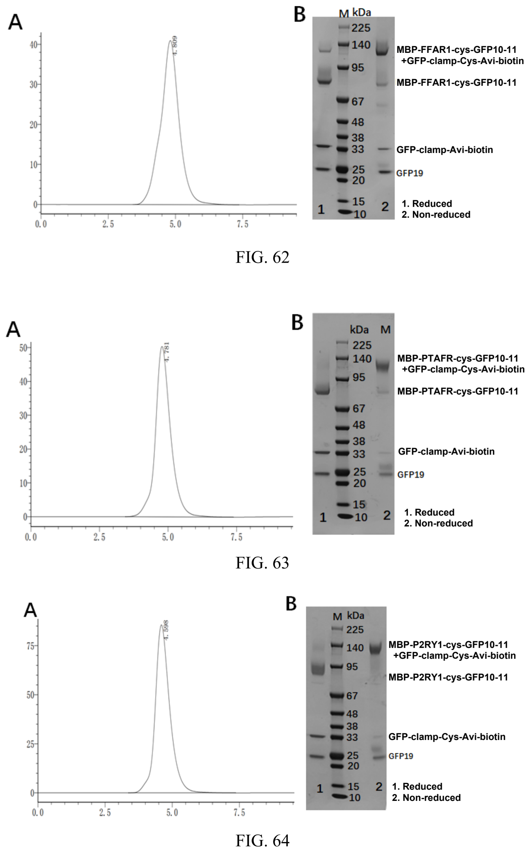

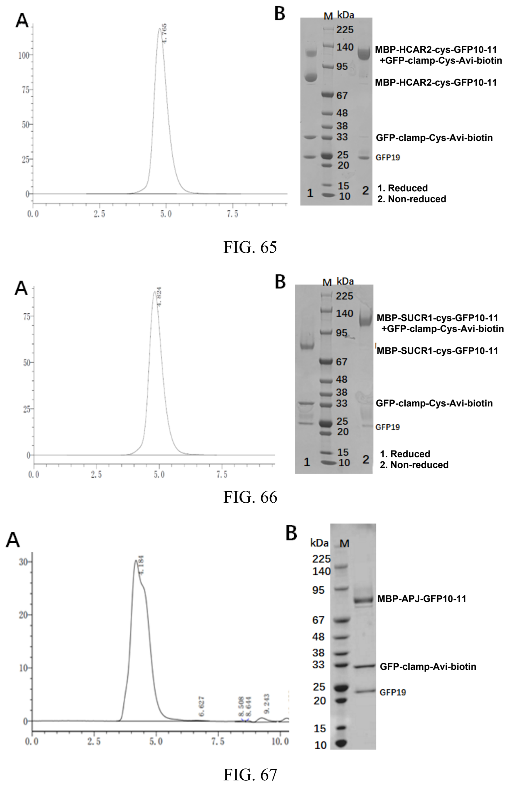

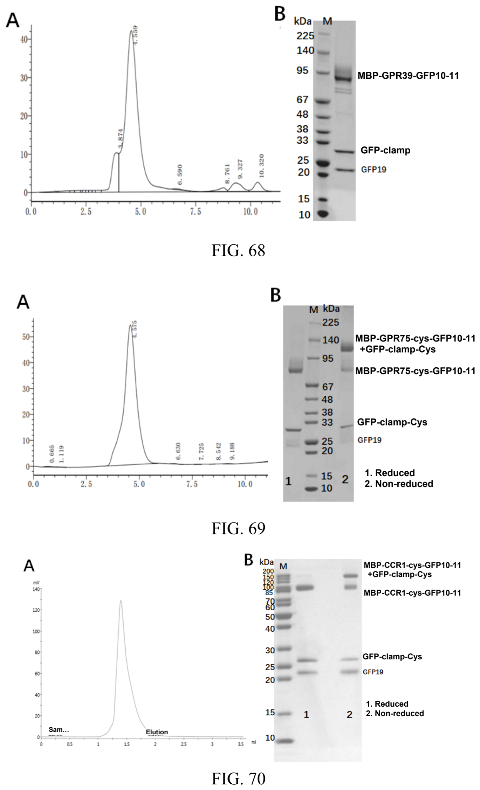

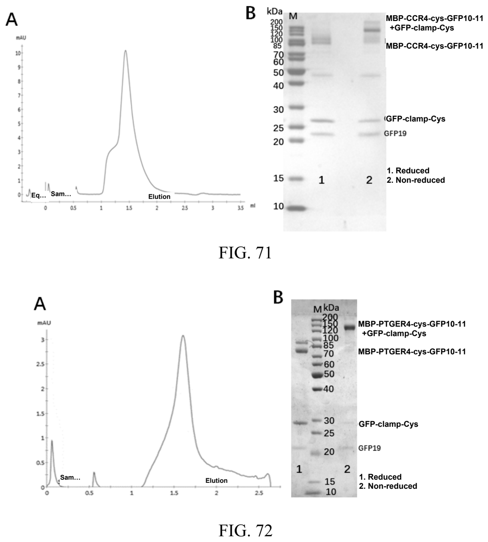

The nucleotide sequences encoding the above modified GPCR and GFP10-11 fusion proteins were cloned into the mammalian cell expression vector pBacMam4R, respectively. Then the vector was transfected to HEK293F cells for co-expressing the GPCR and GFP10-11 fusion protein and the GFP1-9 fragment. The cells were harvested and then added to the GFP-clamp-Cys protein obtained in example 3 and purified using the same steps as those in Example 7. The purified protein was analyzed using molecular sieve chromatography and SDS gel electrophoresis. The results are as shown in FIG. 15-FIG. 74. The results show that these modified GPCRs have good properties and high purity, each component contained is consistent with that expected; and the purified protein forms a UV absorption peak corresponding to a non-aggregated state in molecular sieve chromatography.

## Claims

1. A modified G protein coupled receptor, the modified G protein coupled receptor successively comprising, from the N terminus to the C terminus, the N terminus, a first transmembrane domain, a first intracellular loop, a second transmembrane domain, a first extracellular loop, a third transmembrane domain, a second intracellular loop, a fourth transmembrane domain, a second extracellular loop, a fifth transmembrane domain, a third intracellular loop, a sixth transmembrane domain, a third extracellular loop, a seventh transmembrane domain and the C terminus, wherein at least a part of the first intracellular loop, the second intracellular loop and/or the third intracellular loop is replaced with an optional first linker, a first part of a fluorescent protein and an optional second linker, wherein the first linker and the second linker each independently comprise one or more amino acids.

2. The modified G protein coupled receptor according to claim 1, wherein the fluorescent protein comprises one or more of a green fluorescent protein, a red fluorescent protein, a yellow fluorescent protein, a blue fluorescent protein, a cyan fluorescent protein and an enhanced green fluorescent protein.

3. The modified G protein coupled receptor according to claim 1 or 2, wherein the first part of the fluorescent protein comprises β-strands 10-11, β-strands 1-2, β-strands 8-11 or β-strands 1-4 of the fluorescent protein.

4. The modified G protein coupled receptor according to any one of claims 1-3, wherein the first part of the fluorescent protein comprises an amino acid sequence as shown in SEQ ID NO.1 or an amino acid sequence having at least 50%, at least 60%, at least 70%, at least 80%, at least 90%, at least 95%, at least 96%, at least 97%, at least 98%, at least 99% and at least 99.5% sequence identity to the amino acid sequence as shown in SEQ ID NO.1.

5. The modified G protein coupled receptor according to any one of claims 1-4, wherein an amino acid at the position 34.51 of a Ballesteros-Weinstein numbering system in the second intracellular loop is cysteine.

6. The modified G protein coupled receptor according to any one of claims 1-5, wherein the modified G protein coupled receptor comprises an amino acid sequence as shown in any one of SEQ ID NOs. 3-71 or an amino acid sequence having at least 50%, at least 60%, at least 70%, at least 80%, at least 90%, at least 95%, at least 96%, at least 97%, at least 98%, at least 99% and at least 99.5% sequence identity to the amino acid sequence as shown in any one of SEQ ID NOs. 3-71.

7. A complex, the complex comprising the modified G protein coupled receptor according to any one of claims 1-6 and a second part of a fluorescent protein, wherein the first part of the fluorescent protein binds to the second part of the fluorescent protein.

8. The complex according to claim 7, wherein the second part of the fluorescent protein comprises β-strands 1-9, β-strands 3-11, β-strands 1-7 or β-strands 5-11 of the fluorescent protein.

9. The complex according to claim 7 or 8, wherein the second part of the fluorescent protein comprises an amino acid sequence as shown in SEQ ID NO. 2 or an amino acid sequence having at least 50%, at least 60%, at least 70%, at least 80%, at least 90%, at least 95%, at least 96%, at least 97%, at least 98%, at least 99% and at least 99.5% sequence identity to the amino acid sequence as shown in SEQ ID NO. 2.

10. The complex according to any one of claims 7-9, wherein the complex further comprises a clamp protein which binds to the first part of the fluorescent protein and the second part of the fluorescent protein.

11. The complex according to claim 10, wherein the clamp protein comprises an amino acid sequence as shown in any one of SEQ ID NOs. 72-74 and SEQ ID NO. 82 or an amino acid sequence having at least 50%, at least 60%, at least 70%, at least 80%, at least 90%, at least 95%, at least 96%, at least 97%, at least 98%, at least 99% and at least 99.5% sequence identity to the amino acid sequence as shown in any one of SEQ ID NOs. 72-74 and SEQ ID NO. 82.

12. The complex according to claim 11, wherein cysteine is located at the position 186 of the clamp protein, and the position refers to SEQ ID NO. 72.

13. The complex according to claim 11 or 12, wherein a disulfide bond is formed between the amino acid at the position 186 of the clamp protein and the amino acid at the position 34.51 of the Ballesteros-Weinstein numbering system in the second intracellular loop.

14. Use of the modified G protein coupled receptor according to any one of claims 1-6 or the complex according to any one of claims 7-13 in ligand affinity determination and drug discovery or screening.

15. Use of the complex according to any one of claims 7-13 in analysis of a 3D structure via cryogenic electron microscopy.
